(19) Europäisches Patentamt
European Patent Office
Office européen des brevets

(11) Publication number: 0 016 277
B1

(12) # EUROPEAN PATENT SPECIFICATION

(45) Date of publication of patent specification: 02.12.81

(51) Int. Cl.[3]: C 07 C 121/76, C 07 C 153/11, C 07 D 333/24, C 07 D 333/28, A 61 K 31/275, A 61 K 31/38

(21) Application number: 79301687.4

(22) Date of filing: 17.08.79

(54) 2-Carbonyl-3-hydroxy-alkenonitriles, their use as anti-arthritic agents and processes for their preparation.

(30) Priority: 03.11.78 US 957595
13.12.78 US 968899
30.04.79 US 34315

(43) Date of publication of application:
01.10.80 Bulletin 80/20

(45) Publication of the grant of the European patent:
02.12.81 Bulletin 81/48

(84) Designated Contracting States:
AT BE CH DE FR GB IT LU NL SE

(56) References cited:
DE - A - 2 654 797
DE - A - 2 655 009
DE - A - 2 655 094
DE - B - 1 194 630
FR - A - 1 499 489
FR - A - 2 202 883
FR - A - 2 241 524
US - A - 2 540 982
US - A - 3 076 015

(73) Proprietor: AMERICAN CYANAMID COMPANY
Berdan Avenue
Wayne New Jersey 06904 (US)

(72) Inventor: Hanifin, John William, Jr.
2 Sunset Terrace
Suffern, New York (US)
Inventor: Ridge, David Nelson
Old Mountain Road
Upper Grandview, New York (US)

(74) Representative: Allam, Peter Clerk et al,
LLOYD WISE, TREGEAR & CO. Norman House
105-109 Strand
London WC2R 0AE (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 016 277 B1

Courier Press, Leamington Spa, England.

2-Carbonyl-3-hydroxy-2-alkenonitriles, their use as anti-arthritic agents and processes for their preparation

The present invention concerns compounds and compositions useful as anti-inflammatory agents and as inhibitors of the progressive joint deterioration characteristic of arthritic disease, as well as methods for preparation thereof, and also pharmaceutical compositions for use in meliorating inflammation and of inhibiting joint deterioration in mammals therewith. More particularly, the invention is concerned with 2-carbonyl-3-hydroxy-2-alkenonitriles.

According to the present invention there is provided 2-carbonyl-3-hydroxy-2-alkenonitriles of the formula:

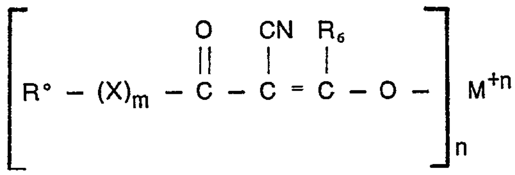

...I

wherein

n is an integer from 1 to 3 inclusive;

M is hydrogen or a pharmaceutically acceptable cation;

$R_6$ is an alkyl group of 1 to 4 carbons;

$R^o$ is one of the following aromatic groups:

(1) the group

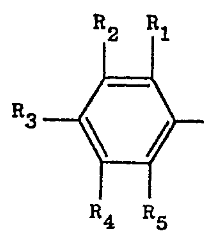

in which $R_1$, $R_2$, $R_3$, $R_4$ and $R_5$ are the same or different and are hydrogen, halogen, alkyl having up to 4 carbons, alkoxy having up to 4 carbons, trifluoromethyl or trichloromethyl, with the proviso that when m is the number 0 at least two of $R_1$, $R_2$, $R_3$, $R_4$ and $R_5$ must be hydrogen but when $R_6$ is methyl and m is the number 0 not all of $R_1$, $R_2$, $R_3$, $R_4$ and $R_5$ may be hydrogen; or

(2) the thienyl moiety

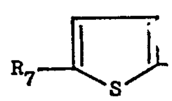

or

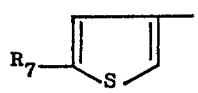

wherein $R_7$ is hydrogen, an alkyl group of up to 3 carbons, fluorine, chlorine or bromine;

X is $CH_2$, O or S and m is the number 0 or 1, provided that when $R^o$ is a thienyl moiety then m is 0;

and the tautomers of the above compounds.

Also provided according to the invention is an anti-arthritic composition for use in inhibiting the progression of arthritis meliorating the inflammation and/or the progressive joint deterioration characteristic of arthritic disease in mammals, comprising a pharmaceutically acceptable carrier or diluent and a 2-carbonyl-3-hydroxy-2-alkenonitrile of the formula:

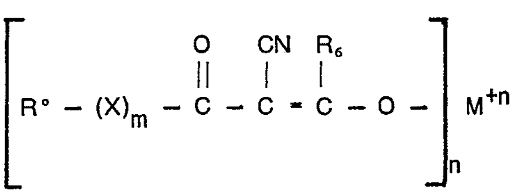

...II

wherein n is an integer from 1 to 3 inclusive;

M is hydrogen or a pharmaceutically acceptable cation;

$R_6$ is an alkyl group of 1 to 4 carbons;

$R^o$ is one of the following aromatic groups:

(1) the group

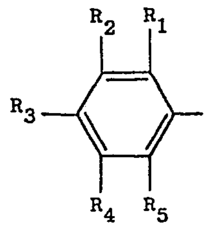

in which $R_1$, $R_2$, $R_3$, $R_4$ and $R_5$ are the same or different and are hydrogen, halogen, alkyl having up to 4 carbons, alkoxy having up to 4 carbons, trifluoromethyl or trichloromethyl, with the proviso that when m is the number 0 at least two of $R_1$, $R_2$, $R_3$, $R_4$ and $R_5$ must be hydrogen; or
(2) the thienyl moiety

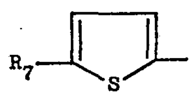 or 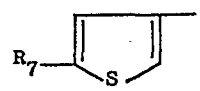

wherein $R_7$ is hydrogen, an alkyl group of up to 3 carbons, fluorine, chlorine or bromine;
X is $CH_2$, O or S and m is the number 0 to 1, provided that when R° is a thienyl moiety then m is 0; and the tautomers of the above compounds.

Further according to the present invention there is provided a method of producing compounds of Formula I, which comprises:

(a) reacting an acetonitrile of the formula:

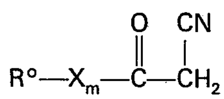

with a compound of the formula:

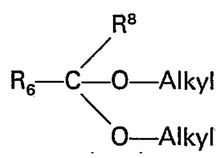

wherein R°, X, m and $R_6$ are as defined above and $R^8$ is O-Alkyl or $N(Alkyl)_2$ and then hydrolyzing the product produced, and wherein, when $R^8$ is O-Alkyl the resultant product may be treated with ammonia or a primary or secondary amine prior to said hydrolysis; or

(b) reacting the metal (Me) enolate salt of an acetonitrile of the formula:

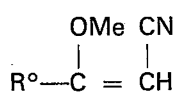

with an acyl halide of the formula:

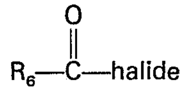

wherein R° and $R_6$ are as defined above; or

(c) reacting an acyl halide of the formula:

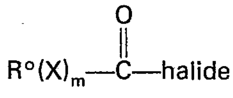

with a metal (Me) enolate salt of the formula

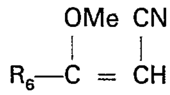

wherein X is $CH_2$, m is the number 0 or 1, R° and $R_6$ are as defined above; or

(d) reacting a compound of the formula:

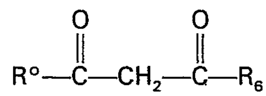

with N,N-dimethylformamide dimethylacetal to produce an intermediate compound of the formula:

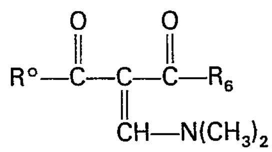

reacting said intermediates with hydroxylamine hydrochloride and treating the resulting reaction mixture with a strong base,

wherein R° and $R_6$ are as defined above; or

(e) reacting a compound of the formula:

R°—X—H

with a compound of the formula:

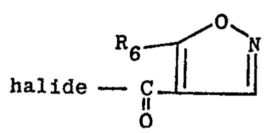

in the presence of a proton scavenger such as a tertiary amine, to form an intermediate of the formula:

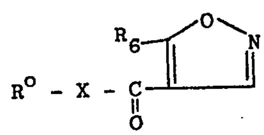

wherein X is O or S and R° and $R_6$ are as defined above, and further treating said intermediate with a proton scavenger such as a tertiary amine or alkoxide, thereby producing the corresponding final product; and

(f) if desired, forming pharmaceutically acceptable cation salts.

Also provided in accordance with the present invention is a method for preparing an anti-arthritic composition for use in inhibiting the progression of arthritis, meliorating inflammation and/or inhibiting progressive joint deterioration in mammals, comprising incorporating into a pharmaceutical carrier a 2-carbonyl-3-hydroxy-2-alkenonitrile of the Formula II, hereinabove.

The useful pharmaceutically acceptable salts of the compounds of the above structural formula wherein M is hydrogen are those with pharmacologically acceptable metal cations, ammonium, amine cations or quaternary ammonium cations. Preferred metal cations are those derived from the alkali metals, e.g., lithium, sodium and potassium, and from the alkaline earth metals, e.g., aluminum, zinc, iron and in particular copper are within the scope of the invention.

Pharmacologically acceptable amine cations and those derived from primary, secondary, or tertiary amines such as mono-, di- or trimethylamine, ethylamine, dibutylamine, triisopropylamine, N-methylhexylamine, decylamine, dodecylamine, allylamine, crotylamine, cyclopentylamine, dicyclohexylamine, mono- or dibenzylamine, $\alpha$- or $\beta$-phenylethylamine, ethylenediamine, diethylenetriamine, and aryliphatic amines containing up to and including 18 carbon atoms, as well as heterocyclic amines, e.g., piperidine, morpholine, pyrrolidine, piperazine and lower alkyl derivative thereof, e.g., 1-methylpiperidine, 4-ethylmorpholine, 1-isopropylpyrrolidine, 2-methylpyrrolidine, 1,4-dimethylpiperazine, 2-methylpiperidine, and the like, as well as amines containing water-solubilizing or hydrophilic groups, e.g., mono-, di-, or triethanolamine, ethyldiethanolamine, N-butylethanolamine, 2-amino-1-butanol, 2-amino-2-ethyl-1,3-propanediol, 2-amino-2-methyl-1-propanol, tris(hydroxymethyl)aminomethane, N-phenylethanolamine, N-(p-tert-amylphenyl)diethanolamine, galactamine, N-methylglucamine, N-methylglucosamine, ephedrine, phenylephrine, epinephrine, procaine and the like.

Examples of suitable pharmacologically acceptable quaternary ammonium cations are tetramethylammonium, tetraethylammonium, benzyltrimethylammonium, phenyltriethylammonium and the like.

Suitable alkyl an alkoxy groups contemplated by the present invention may be, for example, methyl, ethyl, *iso*propyl, *sec*-butyl, methoxy, ethoxy, *n*-propoxy, *iso*butoxy, etc., whereas halogen is exemplified by fluoro, chloro and bromo.

The compounds of the present invention may exist in other tautomeric forms as follows:

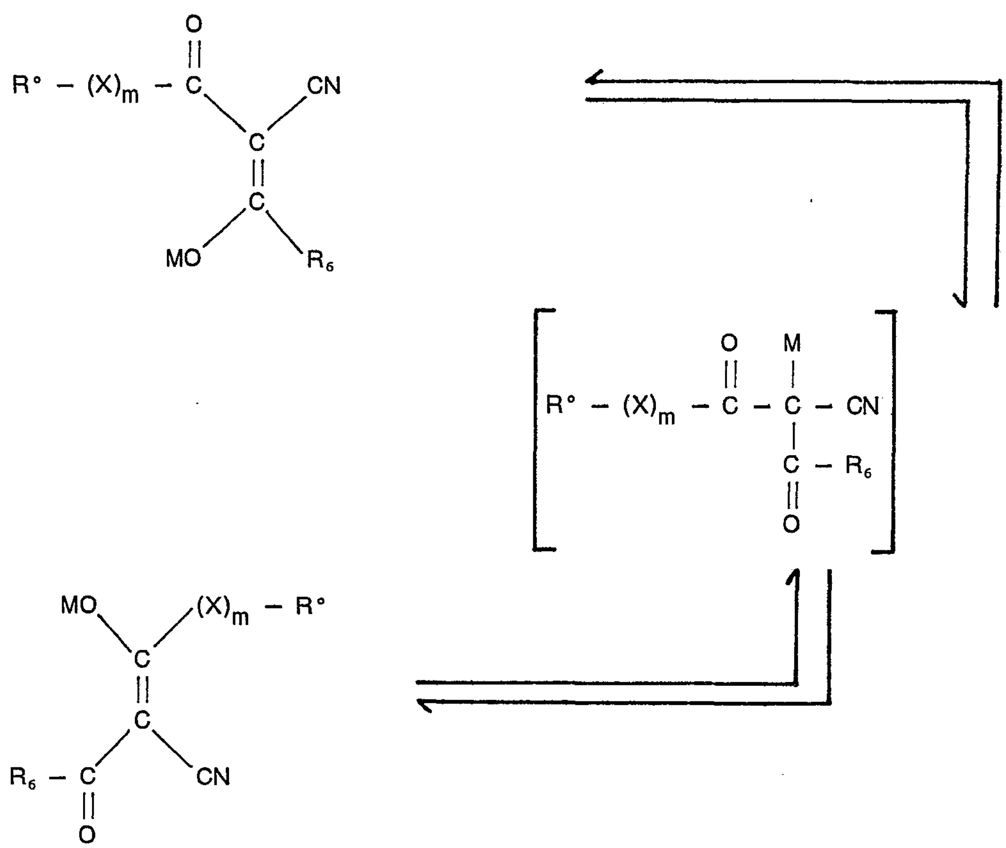

The compounds and compositions of the invention include *cis*-2-benzoyl-3-hydroxy-2-alkenonitriles and the pharmacologically acceptable cationic salts thereof which may be represented by the following structural formula:

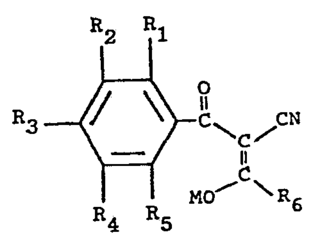

III

wherein the groups are as above defined.

Several procedures exist for the attachment of the acyl fragment to a benzoylacetonitrile side chain. The first involves direct acylation of the benzoylacetonitrile anion (1) with an acyl halide (2) in an appropriate solvent to provide the product (3) as set forth in the following reaction scheme wherein Me is a metal cation as hereinabove defined, X is chloro or fluoro, and $R_1$, $R_2$, $R_3$, $R_4$, $R_5$ and $R_6$ are as hereinabove defined. The enolate anions (1)

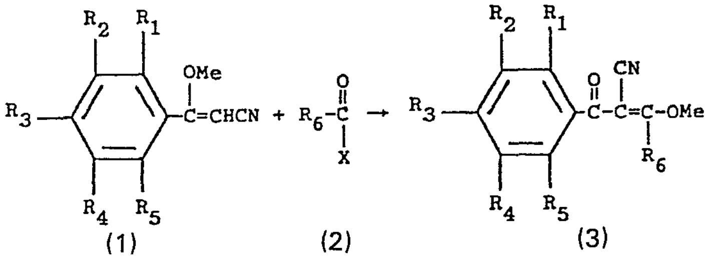

(1) (2) (3)

are prepared by the treatment of the benzoylacetonitrile with the appropriate base in an inert solvent. This enolate may be generated *in situ* and acylated with (2) in the same solvent or may be isolated and reacted in a different solvent system. When Me represents sodium where (1) has been generated by treatment of the benzoylacetonitrile with sodium hydride, sodium amide, sodium methoxide, etc. and X represents chlorine, yields of (3) are low with undesired side products sometimes predominating. Preferably, the benzoylacetonitrile is dissolved in diethyl ether and one equivalent of thallium (I) ethoxide is added. The stable enolate (1), where Me is thallium, precipitates and may be collected by filtration, dried and stored indefinitely. Suspension of (1) in an inert solvent such as ether, tetrahydrofuran, dioxane, etc. at room temperature and the treatment of same with an acyl fluoride (2) causes precipitation of thallium (I) fluoride. This is removed by filtration and the product is extracted from the filtrate.

Another approach involves addition of the acyl fragment as a hydrolyzable portion to yield (5) wherein $R_1$, $R_2$, $R_3$, $R_4$ and $R_5$ are as hereinabove defined. This may be

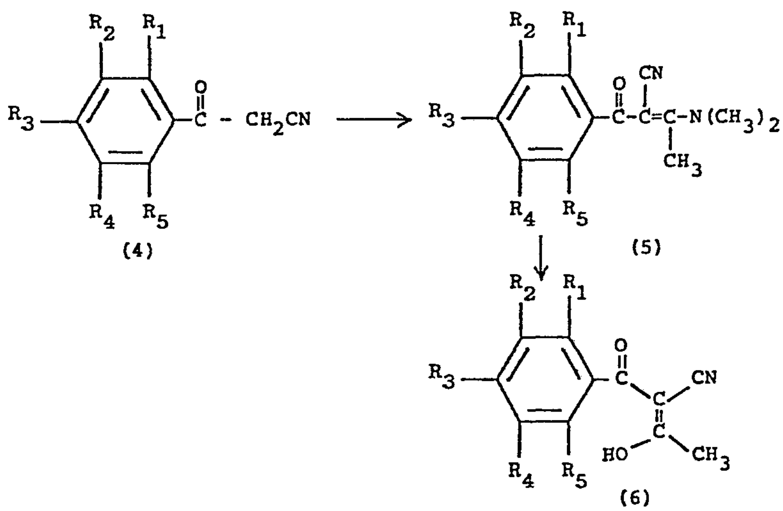

(4) (5) (6)

performed by treatment of the benzoylacetonitrile (4) with N,N-dimethylacetamide dimethylacetal at low temperature in chloroform, methylene chloride, or even as a neat mixture of reagents. Purification yields the condensed product (5). Alternatively, the benzoylacetonitrile (4) is condensed with a trialkyl orthoester (7) in refluxing acetic anhydride as set forth in the following reaction scheme:

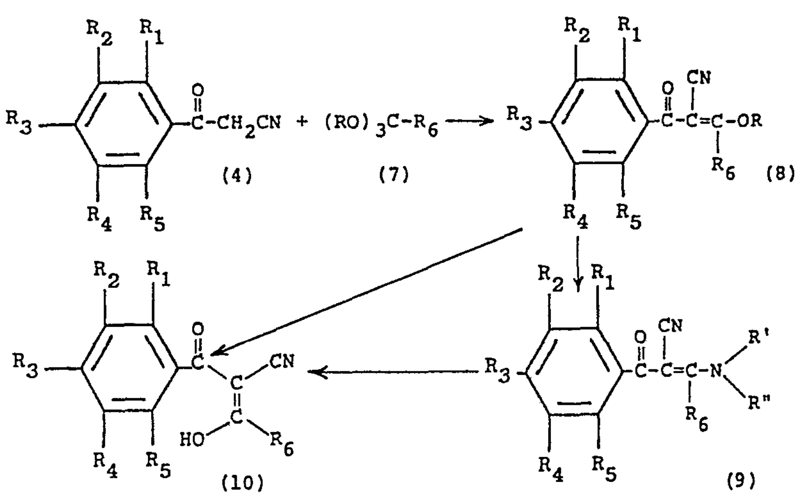

(4) (7) (8) (10) (9)

wherein $R_1$, $R_2$, $R_3$, $R_4$, $R_5$ and $R_6$ are as hereinbefore defined, R is alkyl having up to 4 carbon atoms, R' and R'' are each hydrogen or alkyl having up to 4 carbon atoms and R' and R'' taken together with the associated N(itrogen) is pyrrolidino, piperidino, morpholino, thiomorpholino or N-methylpiperazino. Evaporation of by-products and excess acetic anhydride *in vacuo* and purification of the product under anhydrous conditions provides (8). Treatment of (8) with ammonia or a primary or secondary amine at steam bath temperature under pressure in a sealed vessel for 8—12 hours then provides (9). Both of these intermediates (8) and (9) may be hydrolyzed under acidic conditions to provide the desired products (10).

A different approach to (10) particularly wherein $R_6$ is methyl or ethyl involves addition of the cyanoacyl fragment to a benzoyl chloride as set forth below wherein the groups are as hereinabove defined. This may be performed by generation of the enolate anion (12) *in situ* with the 5-alkylisoxazole (14) and a base such as sodium hydride or sodium amide and subsequent addition of the benzoyl chloride (11) to affect condensation to provide (13).

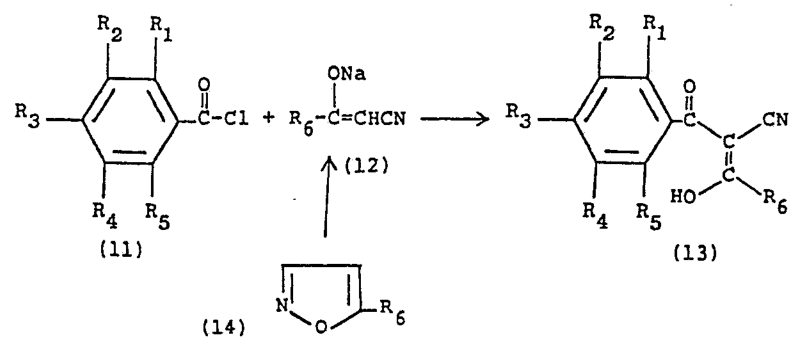

(11) (12) (13) (14)

Alternatively the $\alpha$-cyanoenolate (12) may be prepared separately in a similar manner as above and isolated. Addition of this enolate to the appropriate benzoyl chloride in ether, tetrahydrofuran, etc. at room temperature or at reflux provides (13).

A less desirable route to (10) involves that set forth below wherein $R_1$, $R_2$, $R_3$, $R_4$, $R_5$ and $R_6$ are as hereinabove defined. The acylacetophenone (15) is condensed with N,N-dimethylformamide dimethylacetal at reflux either neat or in solution with an inert solvent such as chloroform or carbon tetrachloride to provide the intermediate (16). Treatment of this with hydroxylamine hydrochloride in solvents such as alcohol, dioxane, dimethylformamide or aqueous solutions of the same provides a mixture of products (17) and (18). These are reacted as an unseparated mixture with a strong base such as sodium hydroxide or sodium alkoxide in alcohol and provides the desired product (10) upon acidification.

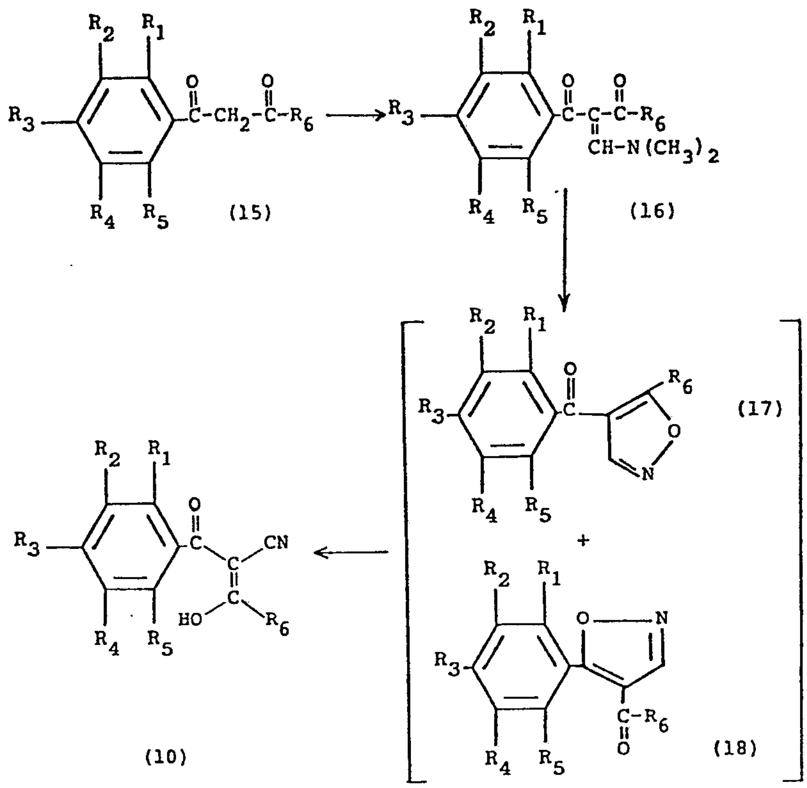

(15) (16) (17) (18) (10)

The compounds and compositions of the invention also include substituted *cis*-2-[2(or 3)-thenoyl]-3-hydroxy-2-alkenonitriles and the pharmacologically acceptable cationic salts thereof which may be represented by the following structural formula:

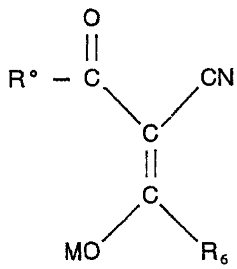

...IV

wherein R° is a 2-thienyl or 3-thienyl moiety of the formulae:

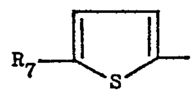

or

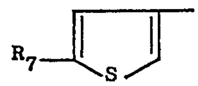

and the other groups are as defined above.

Several procedures exist for the attachment of the acyl fragment to a thenoylacetonitrile side chain. The first involves direct acylation of the thenoylacetonitrile anion (1) with an acyl halide (2) in an appropriate solvent to provide the product (3) as set forth in the following reaction scheme wherein Me is a metal cation as hereinabove defined and X is chloro or fluoro:

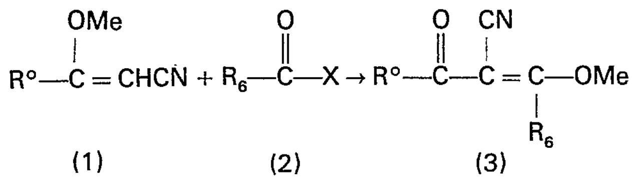

(1) (2) (3)

The enolate anions (1) are prepared by the treatment of the thenoylacetonitrile with the appropriate base in an inert solvent. This enolate may be generated *in situ* and aceylated with (2) in the same solvent or may be isolated and reacted in a different solvent system. When Me represents sodium where (1) has been generated by treatment of the thenoylacetonitrile with sodium hydride, sodium amide, sodium methoxide, etc. and X represents chlorine, yields of (3) are low with undesired side products sometimes predominating. Preferably, the thenoylacetonitrile is dissolved in diethyl ether and one equivalent of thallium (I) ethoxide is added. The stable enolate (1), where Me is thallium, precipitates and may be collected by filtration, dried and stored indefinitely. Suspension of (1) in an inert solvent such as diethyl ether, tetrahydrofuran, dioxane, etc. at room temperature and the treatment of same with an acyl fluoride (2) causes precipitation of thallium (I) fluoride. This is removed by filtration and the product is extracted from the filtrate.

Another approach involves addition of the acyl fragment as a hydrolyzable portion to yield (5) as set forth in the following reaction scheme wherein $R_1$ is as hereinabove defined:

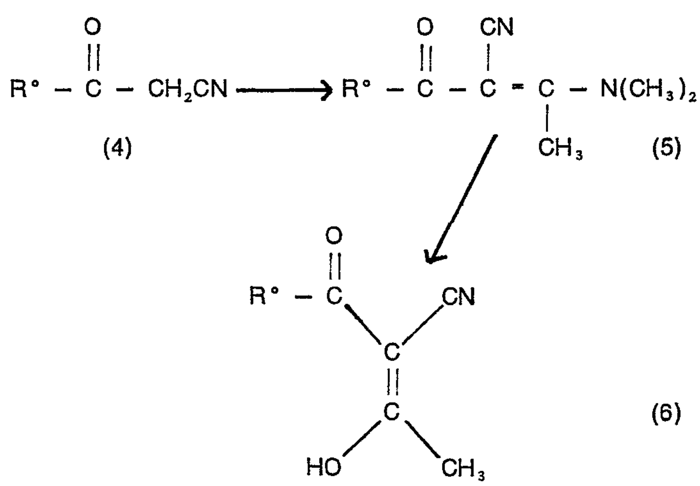

(4) (5) (6)

This may be performed by treatment of the thenoylacetonitrile (4) with N,N-dimethylacetamide, dimethylacetal at low temperature in chloroform, methylene chloride, or even as a neat mixture of reagents. Purification yields the condensed product (5). Hydrolysis of (5) with dilute aqueous mineral acid provides (6). Alternatively, the thenoylacetonitrile (4) is condensed with a trialkyl orthoester (7) in refluxing acetic anhydride as set forth in the following reaction scheme:

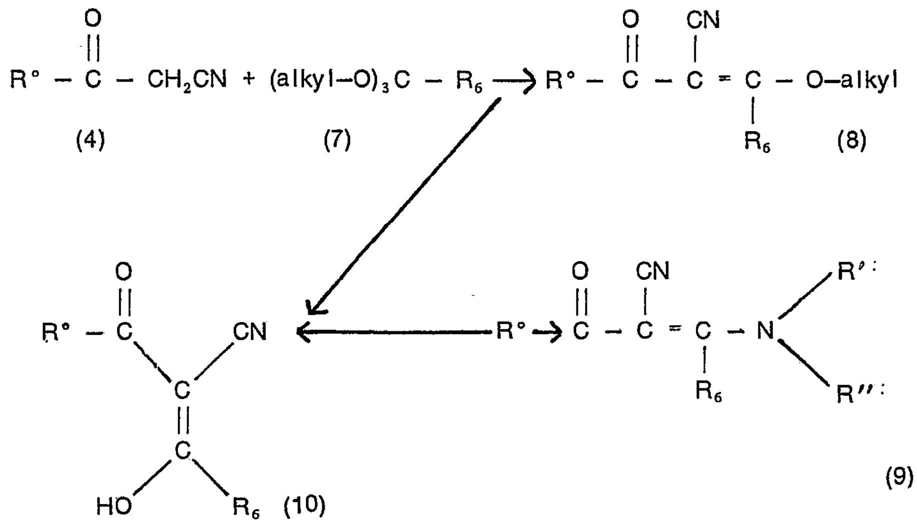

(4) (7) (8) (10) (9)

wherein alkyl is methyl or ethyl, R′ and R″ are each hydrogen or alkyl having up to 4 carbon atoms and R′ and R″ taken together with the associated N(itrogen) is pyrrolidino, piperidino, morpholino, thiomorpholino or N-methylpiperazino. Evaporation of by-products and excess acetic anhydride *in vacuo* and purification of the product under anhydrous conditions provides (8). Treatment of (8) with ammonia or a primary or secondary amine at steam bath temperature under pressure in a sealed vessel for 8—12 hours then provides (9). Both of these intermediates (8) and (9) may be hydrolyzed under acidic conditions to provide the desired products (10).

A different approach to (10) particularly wherein $R_6$ is methyl or ethyl involves addition of the cyanoacyl fragment to a 2(or 3)-thenoyl chloride (11) as set forth below. This may be performed by generation of the enolate

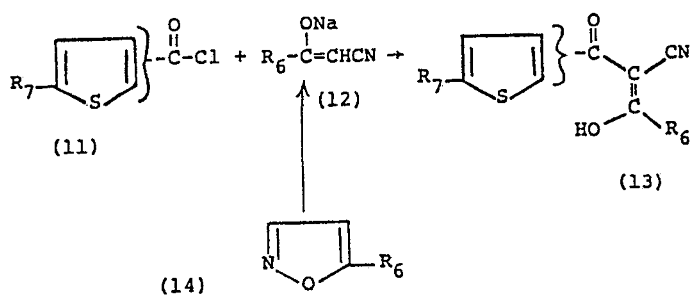

(11) (12) (13) (14)

anion (12) *in situ* with the 5-alkyl-isoxazole (14) and a base such as sodium hydride or sodium amide and subsequent addition of the 2(or 3)-thenoyl chloride (11) to affect condensation to provide (13). Alternatively, the $\alpha$-cyanoenolate (12) may be prepared separately in a similar manner as above and isolated. Addition of this enolate to the appropriate 2(or 3)-thenoyl chloride in ether, tetrahydrofuran, etc. at room temperature or at reflux provides (13).

The compounds and compositions of the invention also include substituted phenyl alkylidene acetoacetonitriles of the formula:

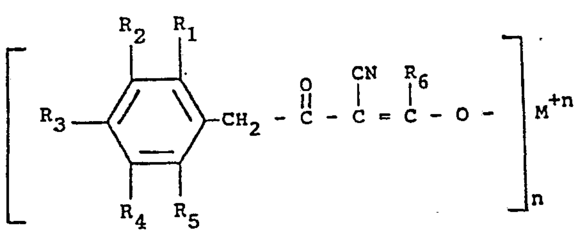

V

wherein the groups and symbols are as defined above.

The compounds of Formula V, for example, may be prepared according to the following Flowchart A:

FLOWCHART A

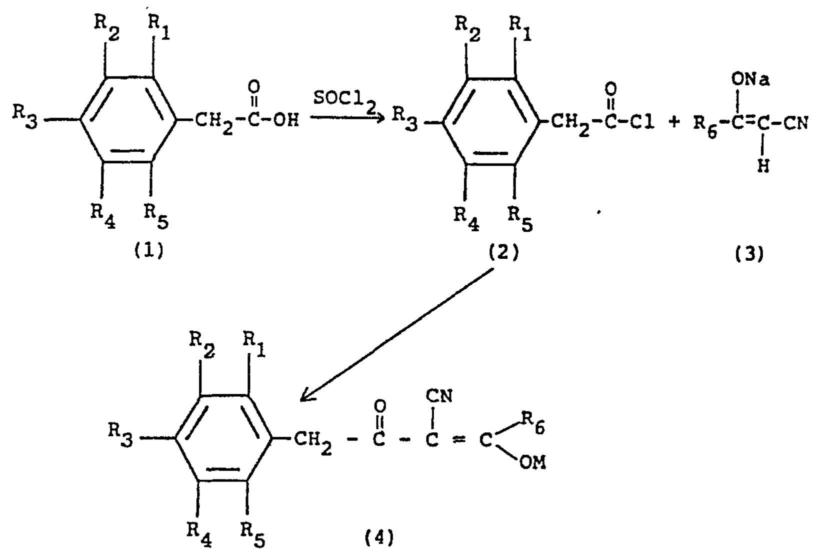


In accordance with Flowchart A an appropriate phenylacetic acid (1) is refluxed with thionyl chloride for several hours, giving the corresponding phenylacetyl chloride (2), which is reacted with cyanoacetone sodium enolate (3) in a solvent such as tetrahydrofuran for several hours, then after removal of the solvents, the residue in aqueous suspension is acidified and extracted into methylene chloride. Evaporation of this extract gives the products (4) where $R_1$, $R_2$, $R_3$, $R_4$, $R_5$, $R_6$ and M are as described above.

The compounds and compositions of the invention also include substituted phenyl-2-cyano-2-thioalkenoic acid esters of the formula:

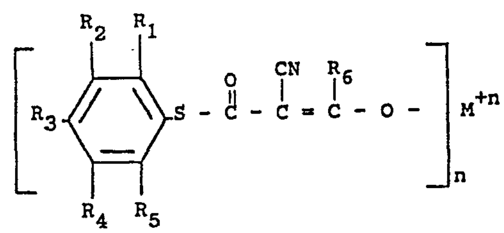 VI

wherein the groups and symbols are as defined above.

The compounds of Formula VI, for example, may be prepared according to the following Flowchart B:

FLOWCHART B

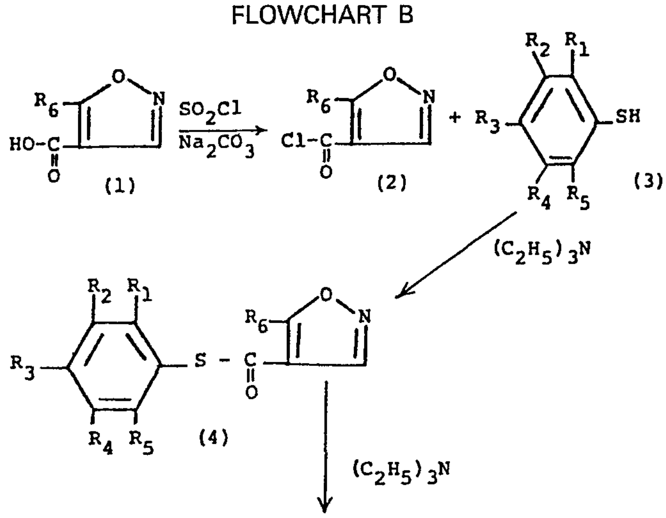

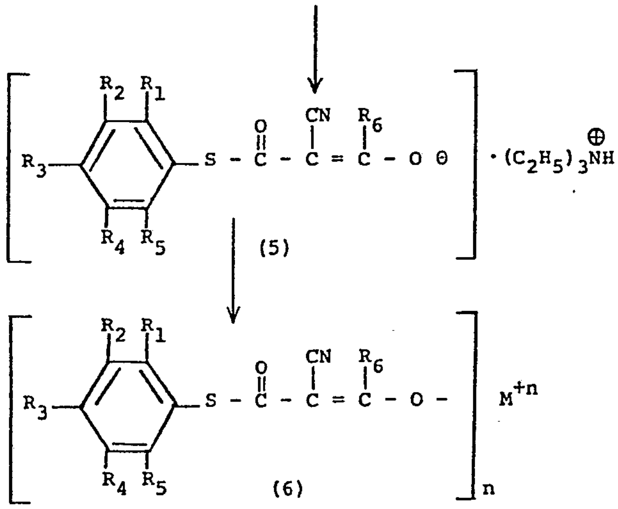

(5)

(6)

In accordance with Flowchart B, thionyl chloride and sodium carbonate in chloroform are used to convert a 5-alkylisoxazole 4-carboxylic acid (1) to the corresponding 5-alkylisoxazole carbonyl chloride (2) by heating for several hours. The compound (2) is then reacted with a substituted thiophenol (3) and one molar equivalent of triethylamine in ether at reduced temperature for several hours. The resulting alkylthio isoxazolecarboxylic acid-S-phenyl ester (4) is extracted in methylene chloride and crystallized with hexane at reduced temperature. The isoxazole (4) is treated with triethylamine in ether for several hours giving the 2-cyano-3-hydroxythio alkenoic acid-S-phenyl ester triethylamine salt (5) which is then acidified in aqueous solution with hydrochloric acid to provide (6) where $R_1$, $R_2$, $R_3$, $R_4$, $R_5$, $R_6$ are as described above and M is hydrogen.

The compounds and compositions of the invention also include substituted phenyl-2-cyano-2-alkenoic acid esters of the formula:

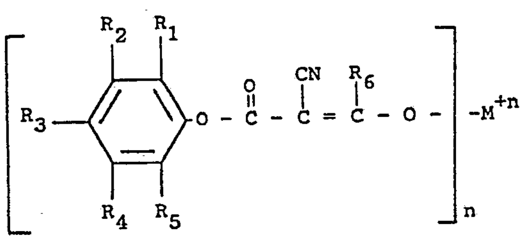

VII

wherein the groups and symbols are as defined above.

The compounds of Formula VII, for example, may be prepared according to the following Flowchart C:

FLOWCHART C

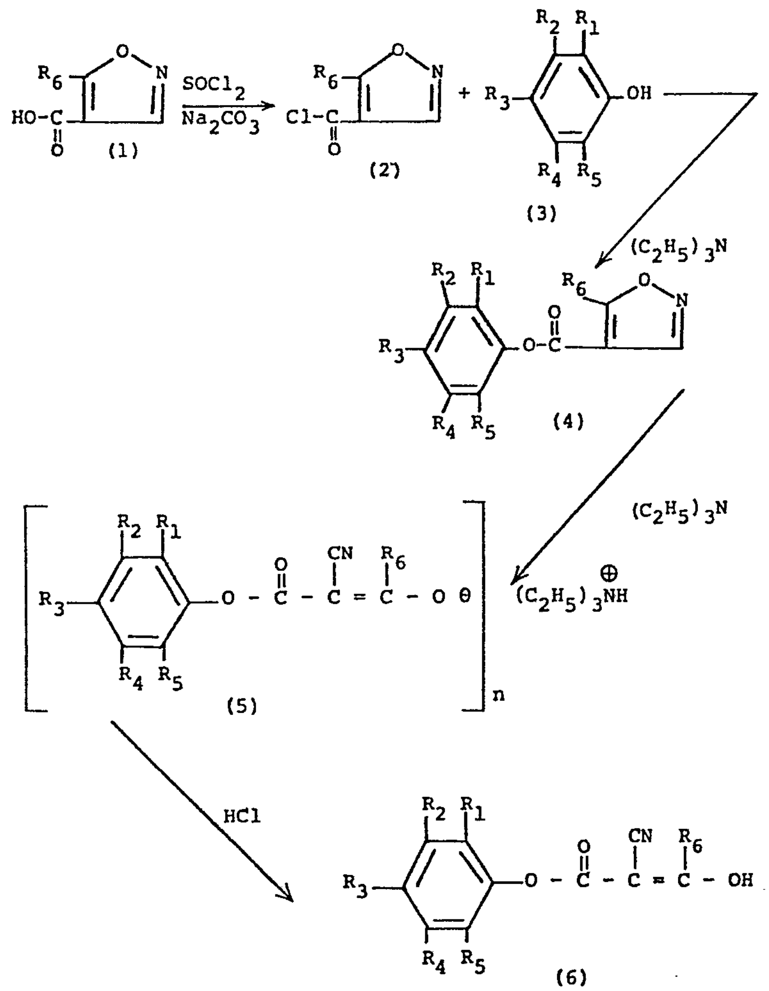


In accordance with Flowchart C, the 5-alkylisoxazole-4-carboxylic acid (1) [H. Yasuda, Yakugaku Zasshi, *79,* 836—838 (1959); C.A., *53,* 21885d] is reacted with thionyl chloride and sodium carbonate in chloroform with heat for several hours producing the corresponding 5-alkylisoxazole carbonyl chloride (2). The compound (2) is then reacted with the appropriate phenol (3) in ether at ice bath temperature with one molar equivalent of triethylamine for several hours giving the 5-alkyl-4-isoxazole-carboxylic acid phenyl ester (4). The ester (4) is treated with triethylamine at ice bath temperature for several hours giving the 2-cyano-3-substituted alkenoic acid phenyl ester triethylamine salt (5). The salt (5) is converted to the free ester (6) where $R_1$, $R_2$, $R_3$, $R_4$, $R_5$ and $R_6$ are as described above by treatment in aqueous solution with a mineral acid.

Certain of the compounds of this invention may be prepared from substituted 2-benzoyl-3-substituted-2-alkenonitriles of the formula:

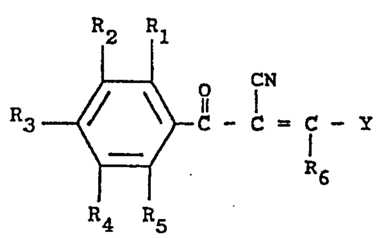

VIII

wherein Y represents the group OR or the group

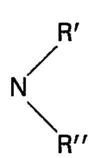

wherein $R_1$, $R_2$, $R_3$, $R_4$ and $R_5$ are each individually selected from the group consisting of hydrogen, halogen, alkyl having from 1 to 4 carbon atoms, alkoxy having from 1 to 4 carbon atoms, trifluoromethyl and trichloromethyl with the proviso that at least two of $R_1$, $R_2$, $R_3$, $R_4$ and $R_5$ must be hydrogen, $R_6$ is alkyl having from 1 to 4 carbon atoms; R is alkyl having from 1 to 4 carbon atoms; and R′ and R″ are each hydrogen or alkyl having from 1 to 4 carbon atoms or R′ and R″ taken together with the associated nitrogen is pyrrolidino, piperidino, morphilino, thiomorpholino or N-methylpiperazino. Halogen is exemplified by fluoro, chloro and bromo. The compounds of formula VIII form the subject of an application which has been divided herefrom.

The preparation of the intermediates of formula VIII and their conversion to useful final products of this invention is set forth in the following reaction scheme wherein $R_1$, $R_2$, $R_3$, $R_4$, $R_5$, $R_6$, R, R′ and R″ are as hereinbefore defined for Formula VIII.

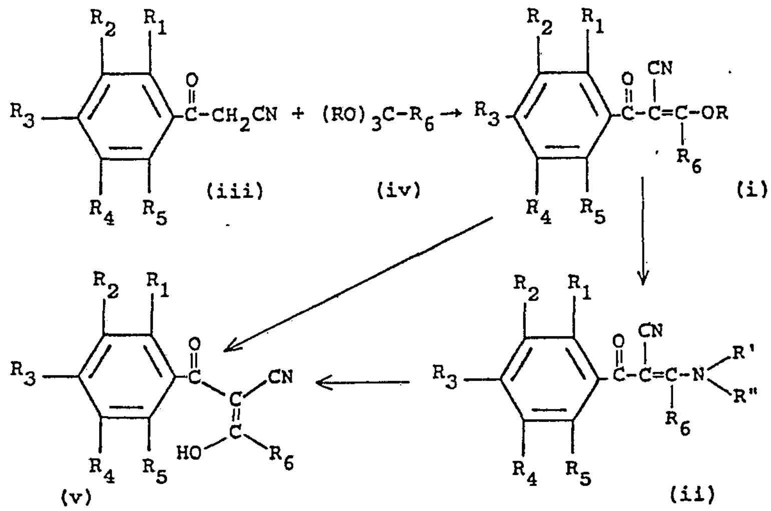

In accordance with this reaction scheme, an appropriately substituted benzoylacetonitrile (iii) is condensed with a trialkyl orthoester (iv) in refluxing acetic anhydride. Evaporation of by-products and excess acetic anhydride *in vacuo* and purification of the residue under anhydrous conditions provides the corresponding substituted 2-benzoyl-3-alkoxy-2-alkenonitrile (i). Treatment of (i) with ammonia or a primary or secondary amine of the formula:

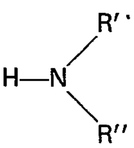

at steam bath temperature under pressure in a sealed vessel for 8—12 hours then provides the substituted 2-benzoyl-3-amino-2-alkenonitriles (ii). Both of these intermediates (i) and (ii) may be hydrolyzed under acidic conditions to provide the anti-inflammatory products (v). The compounds (ii) wherein R′ and R″ are both methyl may also be prepared by treatment of an appropriately substituted benzoylacetonitrile (iii) with N,N-dimethylacetamide dimethylacetal at low temperature in chloroform, methylene chloride, or even as a neat mixture of reagents.

Certain of the compounds of this invention may be prepared from isoxazolecarboxylic acid phenyl esters of the formula:

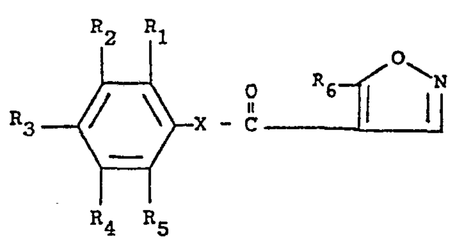

IX

wherein $R_1$, $R_2$, $R_3$, $R_4$ and $R_5$ are each selected from the group comprising hydrogen, halogen, lower alkyl ($C_1$—$C_4$), lower alkoxy ($C_1$—$C_4$), trifluoromethyl and trichloromethyl; $R_6$ is lower alkyl ($C_1$—$C_4$); and X is sulfur or oxygen. The compounds of formula IX form the subject of an application divided herefrom.

These compounds may be treated with triethylamine in ether solution followed by acidification to produce compounds of the present invention of the formula:

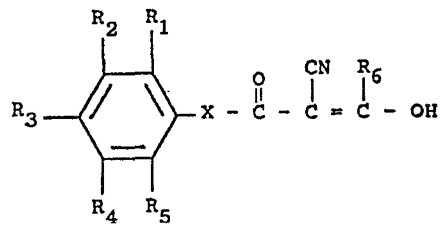

wherein $R_1$, $R_2$, $R_3$, $R_4$, $R_5$, $R_6$ and X are as described above for Formula IX, which compounds are active anti-inflammatory agents.

The novel compounds of the present invention have been found to be highly useful for meliorating inflammation and inhibiting joint deterioration in mammals when administered in amounts ranging from about one milligram to about 250 mg. per kilogram of body weight per day. A preferred dosage regimen for optimum results would be from about 5 mg. to about 100 mg. per kilogram of body weight per day, and such dosage units are employed that a total of from about 0.35 gm. to about 7.0 gm. of the active ingredient for a subject of about 70 kg. of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. A decided practical advantage of this invention is that the active ingredient may be administered in any convenient manner such as by the oral, intravenous, intramuscular, topical, intra-articular, or subcutaneous route. The anti-inflammatory activity of the novel compounds of the present invention was established by the following tests.

(A) Carrageenin-induced edema in the rat

In determining the acute anti-inflammatory activity of the active compounds of the present invention, Royal Hart, Wistar strain rats, ranging in weight from 80 to 90 grams were used. The rats were fasted overnight prior to dosing but had free access to water. The test compounds were administered in aqueous suspension, by gavage, in a volume of 1.7 ml. per 50 grams of rat [corresponds to hydration volume used by Winter, *et al.*, Proc. Soc. Exp. Biol. & Med., *111*, 544—547 (1962)]. The phologistic agent used was carrageenin prepared as a sterile 1% suspension in 0.9% aqueous sodium chloride for routine testing. A volume of 0.05 ml. was injected through a 26 gauge needle into the plantar tissue of the right hind paw. Measurements were made 5 hours after drug administration (4 hours after carrageenin challenge). Volumes of both the normal and carrageenin inflammed feet were determined. The difference between the two measurements is considered to be the increased edema due to the carrageenin administration. Results are expressed as a C/T efficacy ratio (edema of control animals/edema of treated animals). Table 1 records the results of this test at the indicated dose level with typical compounds of the present invention and demonstrates the anti-inflammatory effect of these compounds in comparison with known anti-inflammatory agents.

TABLE I

| Compound | Dose mg./kg. of Body Weight | No. of Rats | C/T Edema Ratio |
|---|---|---|---|
| Controls | — | 64 | — |
| Aspirin | 250 | 32 | 2.8* |
| Phenylbutazone | 250 | 32 | 2.3* |
| Indomethacin | 250 | 32 | 2.9* |
| *Cis*-2-benzoyl-3-hydroxy-crotononitrile | 250 | 12 | 2.3* |
| *Cis*-2-(*m*-fluorobenzoyl)-3-hydroxy-crotononitrile | 250 | 7 | 1.61* |
| *Cis*-3-hydroxy-2-*p*-toluoylcrotononitrile | 250 | 8 | 1.41* |
| *Cis*-3-hydroxy-2-(*p*-anisoyl)-crotononitrile | 250 | 5 | 1.59* |

TABLE I (continued)

cont'd.

| Compound | Dose mg./kg. of Body Weight | No. of Rats | C/T Edema Ratio |
|---|---|---|---|
| *Cis*-3-hydroxy-2-(*o*-anisoyl)-crotononitrile | 250 | 8 | 1.30* |
| *Cis*-2-(*o*-fluorobenzoyl)-3-hydroxy-crotononitrile | 250 | 8 | 2.18* |
| *Cis*-3-hydroxy-2-($\alpha,\alpha,\alpha$-trifluoro-*p*-toluoyl)crotononitrile | 250 | 3 | 1.97* |
| *Cis*-2-(2-thenoyl)-3-hydroxy-2-butenonitrile | 250 | 6 | 2.04* |
| *Cis*-2-(3-thenoyl)-3-hydroxy-2-butenonitrile | 250 | 7 | 2.42* |

* Statistically significant activity $p = <.05$ by t test.

(B) Adjuvant-induced arthritis in the rat

The following test shows the activity of the active compounds of this invention against chronic inflammation in adjuvant-induced arthritis which is accompanied by joint destruction. Groups of three Royal Hart, Wistar strain rats weighing 200 ± 10 g. each were injected intradermally in the right hind paw with Freund's adjuvant (dried human tubercle bacilli in a mineral oil vehicle) at a dose of 2 mg./kg. of body weight. The test compounds were administered orally in a 1.5% starch vehicle at various doses once daily on days 0 to 13 post challenge. Control rats were treated in a similar manner, but given only starch vehicle. On the 14th and 21st day post challenge the diameter of the injected paw (primary lesion) was measured by micrometer caliper. The volume of inflamed paws were estimated from these measurements and the effects of each compound are expressed as per cent inhibition of swelling as compared to controls. Table II records the results of these tests conducted with representative compounds of this invention and known anti-inflammatory agents. The active compounds of this invention suppress the progression of the arthritis and associated joint deterioration.

TABLE II

The Effect of Anti-Inflammatory Agents on Adjuvant-Induced Arthritis in Rats

| Compound | Oral Dose (mg./kg.) | Number of Rats | % Inhibition of Swelling Day 14 | Day 21 |
|---|---|---|---|---|
| Normal rats | — | | — | — |
| Adjuvant Controls | — | | 0 | 0 |
| Indomethacin | 2 | 57 | 51* | 24* |
| | 1 | 54 | 46* | 19* |
| | 0.5 | 54 | 40* | 20* |
| | 0.25 | 9 | 30* | 4* |
| Aspirin | 400 | 57 | 73* | 48* |
| | 200 | 66 | 48* | 27* |
| | 100 | 63 | 36* | 13 |
| | 50 | 21 | 23* | 3 |
| Phenylbutazone | 150 | 27 | 75* | 44* |
| | 75 | 39 | 62* | 28* |
| | 37.5 | 39 | 56* | 14 |
| | 18.8 | 21 | 31* | 7 |
| *Cis*-2-(*p*-fluorobenzoyl)-3-hydroxycrotononitrile | 25 | 18 | 65* | 30* |
| *Cis*-2-(*p*-chlorobenzoyl)-3-hydroxycrotononitrile | 25 | 21 | 58* | 15 |
| *Cis*-2-(*o*-chlorobenzoyl)-3-hydroxycrotononitrile | 50 | 9 | 39* | 29 |
| *Cis*-2-(*m*-fluorobenzoyl)-3-hydroxycrotononitrile | 50 | 9 | 48* | 37* |

TABLE II (continued)
The Effect of Anti-Inflammatory Agents on Adjuvant-Induced Arthritis in Rats

| Compound | Oral Dose (mg./kg.) | Number of Rats | % Inhibition of Swelling Day 14 | Day 21 |
|---|---|---|---|---|
| *Cis*-2-(3-chloro-*o*-toluoyl)-3-hydroxycrotononitrile | 50 | 9 | 61* | 17 |
| *Cis*-3-hydroxy-2-($\alpha$,$\alpha$,$\alpha$-trifluoro-*p*-toluoyl)-crotononitrile | 25 | 6 | 68* | 5 |
| *Cis*-2-(2-thenoyl)-3-hydroxy-2-butenonitrile | 50 | 18 | 42 | 6 |
| *Cis*-2-(3-thenoyl)-3-hydroxy-2-butenonitrile | 50 | 18 | 38* | 19 |
| *Cis*-2-(5-chloro-2-thenoyl)-3-hydroxy-2-butenonitrile | 25 | 9 | 49* | 31* |

* Statistically significant activity p = <.05 by t test.

Using the same procedure, additional active compounds of the present invention were tested against chronic inflammation in adjuvant-induced arthritis, with observation of the primary lesion. At the same time, the other inflamed sites, such as ears, paws and tail (secondary lesions) were observed and each rat was graded as to degree of inflammation and swelling present. The grading is based on a scale of 0 to 24, where 0 represents a complete absence of induced arthritic nodules and 24 represents the maximum degree of inflammation. The mean grade for each treated group is calculated and the effects of each compound are expressed as per cent inhibition of the control grade. Table III records the results of tests conducted with typical compounds of the present invention and known anti-inflammatory agents. The present active compounds appear to suppress the progression of the arthritis and associated joint deterioration.

TABLE III
The Effect of Anti-Inflammatory Agents on Adjuvant Arthritis in Rats

| Compound | Oral Dose mg./kg. of body weight | Dead/-Treated At 21 days | Mean Weight Gain (grams) Day 14 | Day 21 | % Inhibition Swelling (primary lesion) Day 14 | Day 21 | % Inhibition of Control Grade (secondary lesion) Day 14 | Day 21 |
|---|---|---|---|---|---|---|---|---|
| Normal Rats | — | 8/186 | 77 | 112 | — | — | — | — |
| Adjuvant Controls | — | 53/630 | 36 | 31 | 0 | 0 | 0 | 0 |
| Indomethacin | 2 | 8/57 | 68* | 68* | 51* | 24* | 38* | 25* |
| | 1 | 9/54 | 63* | 65* | 46* | 19* | 34* | 20* |
| | 0.5 | 5/54 | 53* | 51* | 40* | 20* | 25* | 17* |
| | 0.25 | 0/9 | 51 | 57* | 30* | 4 | 22* | 4 |
| Aspirin | 400 | 18/57 | 41 | 55* | 73* | 48* | 58* | 45* |
| | 200 | 10/66 | 40 | 44 | 48* | 27* | 26* | 17* |
| | 100 | 18/63 | 48 | 53* | 36* | 13 | 19* | 8 |
| | 50 | 2/21 | 56* | 44 | 23* | 3 | 12 | 9 |
| Phenylbutazone | 150 | 2/27 | 40 | 50* | 75* | 44* | 54* | 31* |
| | 75 | 2/39 | 51* | 50* | 62* | 28* | 27* | 15 |
| | 37.5 | 5/39 | 53* | 53* | 56* | 14 | 18 | 13 |
| | 18.8 | 2/21 | 50* | 45 | 31 | 7 | 4 | 8 |
| *Cis*-2-benzoyl-3-hydroxy-crotononitrile | 100 | 5/18 | 43 | 41 | 68* | 53* | 38* | 46* |
| | 50 | 3/36 | 51* | 45* | 64* | 54* | 14 | 17 |
| | 25 | 3/18 | 37 | 28 | 26 | 14 | 10 | 1 |

TABLE III (continued)
The Effect of Anti-Inflammatory Agents on Adjuvant Arthritis in Rats

| Compound | Oral Dose mg./kg. of body weight | Dead/-Treated At 21 days | Mean Weight Gain (grams) Day 14 | Day 21 | % Inhibition Swelling (primary) lesion) Day 14 | Day 21 | % Inhibition of Control Grade (secondary lesion) Day 14 | Day 21 |
|---|---|---|---|---|---|---|---|---|
| 4-(*p*-Chlorophenyl)-2-(1-hydroxy-ethylidine)acetoacetonitrile | 25 | 6/21 | 41 | 21 | 68* | 17 | — | — |
| 2-Cyano-3-hydroxycrotonic acid, *p*-chlorophenyl ester | 25 | 2/18 | 60 | 46 | 45* | 0 | — | — |
| 2-Cyano-3-hydroxycrotonic acid, 2,3-dichlorophenyl ester | 50 | 11/24 | 36 | 32 | 41* | 0 | — | — |
| 2-Cyano-3-hydroxycrotonic acid, *o*-chlorophenyl ester | 50 | 0/9 | 25 | 29 | 57* | 35 | — | — |

* Significantly different from adjuvant controls

Adjuvant-induced experimental polyarthritis is a specific systemic disease of the rat which shares interesting similarities with rheumatoid arthritis. Specifically the histology of the two diseases bears a remarkable resemblence as shown by C. M. Pearson *et al.*, Am. J. Path. *42,* 73 (1963). E. M. Glenn, Am. J. Vet. Res. *27,* (116), 339 (1966) has classified adjuvant-induced polyarthritis as a crippling and permanent deformity resulting from diffuse connective tissue involvement around certain susceptible joints in the rat. Zahiri *et al.,* Can. Med. Ass. J., *101,* 269 (1969) have shown that the fusiform swelling of the distal joints is associated with edema, congestion and synovitis including pannus formation, all of which precede the ultimate destruction of bone and cartilage. Furthermore, Zahiri *et al.*, indicate that the cartilage destruction in the joint is due to an invasive pannus which originates in the marginal synovium and extends across the articular surface to erode it. When non-steroidal, anti-inflammatory agents such as indomethacin inhibit arthritic paw swelling, which is composed of inflammatory cell infiltrates, they have also been shown to prevent joint and bone deterioration [see S. Wong *et al.*, J. Pharm. & Exp. Ther. *185,* 127 (1973) and G. R. Bobalick *et al.,* Agents and Actions *4,* 364 (1974)]. The most pointed reference showing the relationship between arthritis and joint deterioration is an X-Ray analysis of adjuvant arthritis in the rat by Blackham *et al.,* Agents and Actions *7,* 145 (1977). In a similar manner, inhibition of the progress of arthritis in paws of rats treated with the compounds of this invention also lessens associated joint deterioration.

(C) Migration inhibitory factor in the guinea pig

Rheumatoid arthritis is a chronic inflammatory disease that is characterized by the migration of lymphocytes, macrophages and polymorphonuclear leukocytes to the sites of inflammation. The migration of lymphocytes and macrophages and their multiplication *in situ* is one reason for the very large increase in size of the normally thin synovial membrane, the membrane that encloses the joint space. The synovial membrane that has been transformed in this manner, slowly grows over the articular surfaces and causes the destruction of the articular cartilage and other connective tissue structures of the joint. One of the mechanisms for the destruction of articular cartilage by the synovium is believed to be through the release of various hydrolytic enzymes by the resident macrophages which have been immobilized and activated by migration inhibitory factor (MIF). Macrophages stimulated by MIF are termed "activated macrophages" and undergo the following changes: (a) increased glucose oxidation, (b) increased ruffling of plasma membrane and increased spreading of cells, (c) synthesis and secretion of neutral proteases, (d) release of preformed lysosomal enzymes, (e) decreased migration from a capillary tube. All these effects are associated with an inflammatory situation and it has been demonstrated that MIF is present in the synovial fluid from patients with rheumatoid arthritis. The mechanisms of cartilage destruction can be pictured as follows:

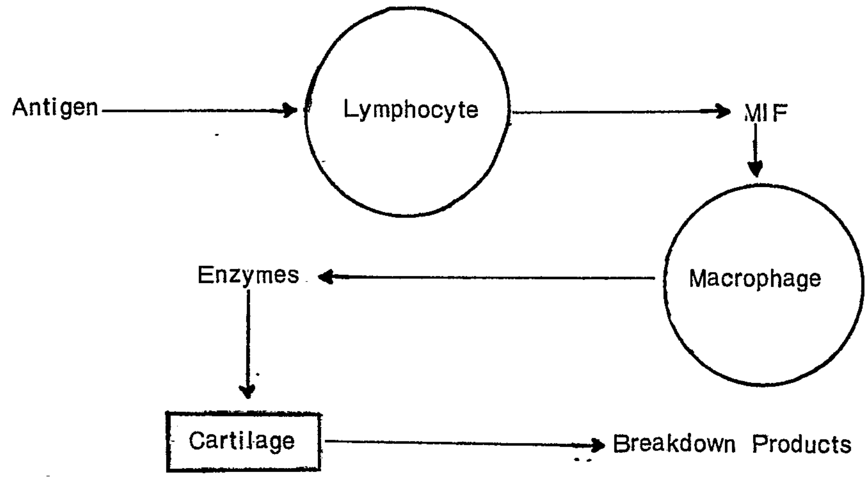


Although the postulated antigen initiating these destructive events in rheumatoid arthritis has not been discovered, the secretion of MIF by lymphocytes and the secretion of enzymes by macrophages as well as the breakdown of cartilage by macrophage enzymes has been demonstrated. Drugs that can therefore block the release of MIF by lymphocytes or the activation of macrophages by MIF should be clinically effective in retarding the destructive joint damage which occurs in rheumatoid arthritis. Drugs can be tested as MIF inhibitors by using the capillary tube migration assay as follows.

Male Hartley guinea pigs, weighing between 300 and 600 grams, are injected intraperitoneally with 25 ml. Marcal 52 Oil® (Humble Oil). Three to four days later, the guinea pigs are sacrificed by decapitation, the peritoneium opened, and the exudate aspirated into a separatory funnel after the addition of 50 ml. of cold Hank's solution. This is repeated two times. The oil phase is discarded and the cell suspension is spun down at 1200 r.p.m. for ten minutes. The cells are resuspended in Hank's solution and spun down at 900 r.p.m. for five minutes. This is repeated two times. Viability of the cells is then determined using trypan blue. In all experiments, viability is greater than 90%. The cell concentration is then adjusted to give a 10% packed cell volume by the addition of the appropriate amount of MEM® (Gibco 109) + 15% guinea pig serum. To the medium is added L-glutamine (2 nM), penicillin (100 U/ml.) and streptomycin (100 $\mu$g/ml). Capillary tubes (1.1—1.5 x 75 mm, Clay Adams) are filled with cells by capillary action and sealed at one end with a small plug of paraffin. Capillary tubes are centrifuged at 700—800 r.p.m. for five minutes to get approximately 4—5 mm of packed cells at the closed end. The tubes are cut at the cell-fluid interface. Two packed capillary tubes are then transferred to each chamber of the Lexy culture dish (Mini-Lab Co. LLC—4002, Quebec, Canada). The capillaries are held in place using a small amount of silicone grease (Dow Corning) and a cover glass then placed on top of the chamber, making a seal between the cover glass and the chamber using paraffin wax. Chambers are then filled with medium or in medium in which antigen (PPD) or antigen + drug are dissolved by filling the chamber through the passages. After the addition of medium, the passages are sealed off using silicone. The cells are then incubated at 37°C. for 24 hours. To determine the area of cell migration out of the capillary tube onto the chamber surface, the chambers are projected onto a microscope screen, the area traced onto paper, and then measured using a planimeter. The antigen, PPD, inhibits the migration of macrophages by approximately 50%. Drugs that reverse this inhibition by greater than 15% are considered active. Table IV lists the results of this direct MIF assay at the indicated dose level with typical compounds of the present invention.

TABLE IV
The Effect of Anti-Inflammatory Agents on Migration Inhibitory Factor

| Compound | Dose | % Reversal* | #Actives** Total Tests |
|---|---|---|---|
| *Cis*-2-(*p*-fluorobenzoyl)-3-hydroxycrotononitrile | $5 \times 10^{-5}$ M | 48 | 4/4 |
| *Cis*-2-(*p*-bromobenzoyl)-3-hydroxycrotononitrile | 10 $\mu$g./ml. | 25 | 2/3 |
| *Cis*-2-(*o*-anisoyl)-3-hydroxycrotononitrile | $5 \times 10^{-5}$ M | 18 | 4/5 |
| *Cis*-2-(3-chloro-*o*-toluoyl)-3-hydroxycrotononitrile | $5 \times 10^{-5}$ M | 16 | 3/5 |

Footnotes to TABLE IV

$$* \text{ Per Cent reversal of inhibition} = \frac{c-b}{a-b}$$

wherein a = cells with no addition, b = cells + antigen, and c = cells + antigen + drug.

** Ratio is the number of tests wherein the per cent reversal of inhibition $\geq$15%.

(D) Ultraviolet-induced erythema in guinea pigs

Another method of determining a drug effect on conditions which result in inflammation is by measuring the effect on ultraviolet induced erythema in guinea pigs. Albino guinea pigs were depilitated on their flanks, the evening before testing, with a standard mixture of barium sulfide and gum acacia. On the morning of the test, groups of four guinea pigs were dosed by gavage one hour prior (—1 hour) to ultraviolet exposure. At 0 hour they were restrained in a plastic container which allows exposure of 3 circular spots. They were then exposed to ultraviolet irradiation from a "Hanovia" Kromayer lamp, model 10, for 60 seconds. At one and four hours, the degree of erythema for each of the three sites was assessed according to the following scoring system: 0 = no erythema, 0.5 = incomplete circle or faint erythema, and 1.0 = complete circle of distinct erythema. Thus, the maximum score for each animal was 3.0. The following Table V summarizes the results of this test with a typical compound of the present invention and other drugs known to have a beneficial anti-inflammatory effect in suppressing ultra-violet induced erythema in warm blooded animals.

TABLE V

The Effect of Anti-Inflammatory Agents on Development of Erythema in Guinea Pigs (pooled data)

| Oral Treatment | Dose mg./kg. of body weight | Score (avg.) 1 hour | 4 hours | Number | Decision |
|---|---|---|---|---|---|
| Control | — | 2.1 | 2.8 | 384 | |
| Aspirin | 250 | 0.1 | 1.2 | 88 | A |
| | 125 | 0.1 | 2.0 | 16 | A |
| | 62.5 | 0.8 | 2.0 | 11 | A |
| | 31.3 | 1.2 | 2.3 | 12 | |
| Phenylbutazone | 250 | 0 | 0.5 | 60 | A |
| | 125 | 0.1 | 1.1 | 16 | A |
| | 62.5 | 0.3 | 0.9 | 12 | A |
| | 31.3 | 0.4 | 1.7 | 12 | A |
| | 15.6 | 0.4 | 2.3 | 8 | A |
| | 7.8 | 1.1 | 2.9 | 8 | |
| Indomethacin | 250 | 0 | 1.0 | 20 | A |
| | 125 | 0 | 1.3 | 12 | A |
| | 62.5 | 0 | 1.3 | 8 | A |
| | 31.3 | 0.1 | 1.9 | 8 | A |
| | 15.6 | 0 | 2.0 | 8 | A |
| | 7.8 | 0.6 | 2.3 | 8 | A |
| | 3.9 | 1.2 | 2.9 | 8 | |
| *Cis*-2-benzoyl-3-hydroxy-crotononitrile | 125 | 0 | 1.3 | 8 | A |
| | 62.5 | 0.8 | 2.4 | 4 | A |
| | 31.3 | 0.8 | 2.3 | 4 | A |
| | 15.6 | 2.3 | 2.5 | 4 | |

A — Statistically significant activity — $p = <.05$ by t test.

(E) Synovium-cartilage Test

Compounds of the invention have been tested by the synovium-cartilage test which is a modification of the method described by Dumonde, D.C. and Glynn. L.E., The Production of Arthritis in Rabbits by an Immunological Reaction to Fibrin, Brit. J. Exp. Pathol., *43*, 373 (1962).

Male, New Zealand rabbits, weighing 1.6—3.0 kg were used. The rabbits were housed in

individual cages and given food and water *at libitum.* The rabbits were sensitized to bovine serum albumin (BSA) by injection of 20 mg./kg. BSA in complete Freund's adjuvant. The emulsion was injected subcutaneously into multiple sites on the back. Total injection volume equaled one mg./kg. of body weight. Between 18 and 21 days following immunization, the rabbits were challenged with an intra-artricular injection of 5 mg. BSA into the right knee joint. Unanesthetized animals were restrained on their backs, and a 20-gauge, one-inch needle, with a 5 ml. syringe, was introduced into the joint and synovial fluid aspirated to determine that the synovium had been penetrated. The needle was then left in place for injection of the antigen. A 0.5 ml. portion of 10 mg./ml. BSA in sterile saline was injected into the synovial sac. The rabbits received an additional five intra-articular injections of 5 mg BSA into the right knee at intervals of 12—16 days. Following the sixth injection the rabbits were sacrificed by an intracardiac injection of 5 ml. of a saturated solution of potassium chloride. The right knee was shaved. A longitudinal incision was made over the patellar ligament and the skin reflected under sterile conditions. The patellar ligament was cut transversely and reflected. Fascia and capsular tissue were trimmed from the lateral and medial aspects of the knee exposing the synovial membrane. The membrane was grasped with forceps, stretched laterally and then excised in a single piece on each side of the knee. The infrapatellar fat pad was removed and the joint space widened by cutting the anterior attachments of the menisci and severing the collateral cruciate ligaments. The femur and tibia were then separated and the menisci excised along with sufficient amounts of synovial tissue from the popliteal area adhering to the menisci. The tissues were immediately placed in a sterile Petri dish containing tissue culture medium composed of MEM (Earle's salts) without sodium bicarbonate, with 25 mM Hepes buffer, pH adjusted to 7.34—7.37, and antibiotics (streptomycin and neomycin, 100 units/ml.). Ten per cent normal rabbit serum (NRS) was added. The tissues were rinsed three times in fresh medium plus 10% NRS, then cut into pieces of 20—30 mg.

Articular cartilage was obtained from the knees of normal, young rabbits, weighing 1.0 to 1.5 kg. The knees were shaved, the rabbits sacrificed and the joint exposed as described above. Synovial tissue and the infrapatellar fat pad were removed. Ligaments were severed and the menisci excised. Femur and tibia were then separated and articular cartilage was cut from supracondylar lines, patellar surface and femoral condyles. Due to curvature of the bone, these pieces were not more than 6—7 mg. each. No cartilage was taken from the tibia. The cartilage was placed in a sterile Petri dish containing tissue culture medium plus 10% NRS and rinsed three times with fresh medium + 10% NRS. The cartilage was then cut into 1—2 mg. pieces and stored at −70°C.

A 10 mg. portion of the test compound was dissolved or suspended in absolute ethanol. Ten $\mu$l. were then transferred to the complete tissue culture medium. The final concentration of test compound was then 10 mcg.-ml. and the vehicle 0.1%.

Tissue culture medium was added to 12 × 75 mm clear plastic culture tubes containing one piece of normal articular cartilage. A piece of arthritic synovial tissue was then added to all tubes except those tubes in which cartilage was incubated alone. All tubes were then incubated at 37°C. for 48 hours with constant rotation in a roller drum at 0.2 rpm. After 48 hours the cartilage was removed, hydrolyzed and assayed for hexoseamine and hydroxyproline. Hexoseamine to hydroxyproline ratios were calculated for the three groups:

1. Cartilage alone
2. Cartilage + synovium
3. Cartilage + synovium + test compound.

Cartilage hexoseamine decreases when it is cultured in the presence of synovium but remains constant when cultured alone. Hydroxyproline remains constant in all groups and the amount assayed is a measure of the size of the incubated cartilage. Therefore, the hexoseamine-hydroxyproline ratio decreases in the cartilage + synovium group relative to the cartilage alone group. The decrease is approximately 50%.

If a compound prevents the decrease in the hexoseamine/hydroxyproline ratio by greater than 50% it is retested. A compound is considered active if it averages greater than 50% suppression of breakdown in three separate tests.

The following Table VI gives the results of this test with compounds of the present invention. These compounds are indicated to be active when tested by the above procedure.

TABLE VI

| Compound | No. of Tests | Mean % Inhibition |
|---|---|---|
| 2-Cyano-3-hydroxycrotonic acid, *p*-methoxyphenyl ester | 4 | 58.38 |
| 2-Cyano-3-hydroxycrotonic acid, *p*-tolyl ester | 3 | 50.34 |
| 2-Cyano-3-hydroxycrotonic acid, *o*-methoxyphenyl ester | 3 | 49.30 |
| 2-Cyano-3-hydroxycrotonic acid, *o*-fluorophenyl ester | 3 | 52.34 |
| 2-Cyano-3-hydroxythiocrotonic acid-S-phenyl ester | 3 | 105.77 |

Compositions according to the present invention having the desired clarity, stability and adaptability for parenteral and intra-articular use are obtained by dissolving from 0.10% to 10.0% by weight of active compound in a vehicle consisting of a polyhydric aliphatic alcohol or mixtures thereof. Especially satisfactory are glycerin, propylene glycol, and polyethylene glycols. The polyethylene glycols consist of a mixture of non-volatile, normally liquid, polyethylene glycols which are soluble in both water and organic liquids and which have molecular weights of from about 200 to 1500. Although the amount of active compound dissolved in the above vehicle may vary from 0.10% to 10.0% by weight, it is preferred that the amount of active compound employed be from about 3.0% to about 9.0% by weight. Although various mixtures of the aforementioned non-volatile polyethylene glycols may be employed, it is preferred to use a mixture having an average molecular weight of from about 200 to about 400.

In addition to the active compound, the parenteral solutions may also contain various preservatives which may be used to prevent bacterial and fungal contamination. The preservatives which may be used for these purposes are, for example, myristyl-gamma-picolinium chloride, phenyl mercuric nitrate, benzalkonium chloride, phenethyl alcohol, *p*-chlorophenyl-$\alpha$-glycerol ether, methyl and propyl parabens, and thimerosal. As a practical matter it is also convenient to employ antioxidants. Suitable antioxidants include, for example, sodium bisulfite, sodium metabisulfite, and sodium formaldehyde sulfoxylate. Generally, from about 0.05 to about 0.2% concentrations of antioxidant are employed.

For intramuscular injection, the preferred concentration of active compound is 0.25 to 0.50 mg./ml. of the finished compositions. The active compounds of the invention are equally adapted to intravenous administration when diluted with water or diluents employed in intravenous therapy such as isotonic glucose in appropriate quantities. For intravenous use, initial concentrations down to about 0.05 to 0.25 mg./ml. of active compound are satisfactory.

The active compounds of the present invention may be orally administered, for example, with an inert diluent or with an assimilable edible carrier, or they may be enclosed in hard or soft shell gelatin capsules, or they may be compressed into tablets, or they may be incorporated directly with the food of the diet. For oral therapeutic administration, the active compounds may be incorporated with excipients and used in the form of tablets, troches, capsules, elixirs, suspensions, syrups, wafers, and the like. Such compositions and preparations should contain at least 0.1% of active compound. The percentage of the compositions and preparations may, of course, be varied and may conveniently be between about 2% to about 60% of the weight of the unit. The amount of active ingredient in such therapeutically useful compositions is such that a suitable dosage will be obtained. Preferred compositions or preparations according to the present invention are prepared so that an oral dosage unit form contains between about 50 and 250 milligrams of active compound.

The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier such as a fatty oil. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed.

The invention is illustrated by the following Examples.

Example 1

3-Dimethylamino-2-(*p*-fluorobenzoyl)crotononitrile

To a solution of 1.6 g. of *p*-fluorobenzoylacetonitrile [Pihl *et al.,* Reakts. Sposobnost Org. Soedin. Tartu. Gos. Univ., *5* (1), 27, (1968)] in 30 ml. of chloroform, cooled to —10°C., is added 1.4 g. of N,N-dimethylacetamide dimethylacetal. The reaction mixture is stirred at —10°C. for 2 hours and then evaporated *in vacuo* to an oil. This oil is dissolved in 150 ml. of benzene and filtered through Magnesol®. The filtrate is evaporated to 50 ml. and petroleum ether is added to effect crystallization. The product is collected by filtration and then recrystallized from benzene-petroleum ether with charcoal treatment giving the desired product.

Example 2

*Cis*-2-(*p*-fluorobenzoyl)-3-hydroxycrotononitrile

To a solution of 6.7 g. of *p*-fluorobenzoylacetonitrile in 100 ml. of chloroform, cooled to 0°C., is added 7 ml. of N,N-dimethylacetamide dimethylacetal. The reaction mixture is stirred in an ice bath for 2 hours and then at room temperature for 16 hours. The solution is evaporated *in vacuo* to an oil. This oil is dissolved in chloroform, filtered through Magnesol® and then evaporated to an oil. This oil is dissolved in 100 ml. of methanol and 20 ml. of 1N hydrochloric acid is added. The reaction mixture is heated on a steam bath for 25 minutes, cooled and the precipitate is collected. The solid is recrystallized from 50 ml. of cyclohexane, giving the desired product.

Example 3

3,4-Difluoroacetophenone

Dry aluminum chloride (26 g., 0.19 mole) is added to a 250 ml. three-neck flask under argon and the flask is cooled in ice. Acetyl chloride (14 ml., 0.19 mole) is then added dropwise, followed by 20 g. (0.18 mole) of o-difluorobenzene. The ice bath is removed and the reaction is slowly warmed and eventually held at 100° for 3.5 hours. The hot reaction solution is then poured onto ice and extracted with ether. The organic extracts are washed with aqueous sodium bicarbonate and evaporated. The dark residue (23 g.) is distilled at 39°/0.1 mm to provide 20 g. (73%) of colorless 3,4-difluoroacetophenone (mp. *ca.* 20°). Similarly prepared is 2,5-difluoroacetophenone.

Example 4

3,4-Difluorophenacyl bromide

To a solution of 10.1 g. (0.065 mole) of 3,4-difluoroacetophenone in 100 ml. of glacial acetic acid was added 3.4 ml. (0.065 mole) of bromine dropwise. When the addition was complete the solution was stirred for 0.5 hr. and then stripped to dryness under reduced pressure. The residue was dissolved in chloroform and washed with aqueous sodium bicarbonate. Evaporation of the organic phase provided 14.8 g. (97%) of colorless liquid product. Similarly prepared were 2,4-dichlorophenacyl bromide, *o*-ethylphenacyl bromide, *p-iso*propylphenacyl bromide, *m-iso*butylphenacyl bromide, 3,4-dimethylphenacyl bromide, *m*-ethoxyphenacyl bromide, *p-iso*propoxyphenacyl bromide, 3,4-diethoxyphenacyl bromide, and 2,5-difluorophenacyl bromide.

Example 5

3,4-Difluorobenzoylacetonitrile

A solution of 13.2 g. (0.056 mole) of 3,4-difluorophenacyl bromide was dissolved in 100 ml. of ethanol and cooled to 5°C. in ice. A solution of 7.6 g. (0.16 mole) of sodium cyanide in 40 ml. of water was added dropwise over 0.5 hr. and the reaction is stirred for an additional one hour. At that time, the mixture was diluted with 100 ml. of water and filtered through Celite®. Acidification of the filtrate gave a cloudy mixture which was extracted with methylene chloride. The organic phase was dried, filtered through Magnesol® and evaporated. Recrystallization of the residue from carbon tetrachloride provides 5.3 g. (52%) of colorless solid, m.p. 74°—75°C. Similarly prepared were 2,4-dichlorbenzoylacetonitrile, *o*-ethylbenzoylacetonitrile, *p-iso*propylbenzoylacetonitrile, *m-iso*butylbenzoylacetonitrile, 3,4-dimethylbenzoylacetonitrile, *m*-ethoxybenzoylacetonitrile, *p-iso*propoxybenzoylacetonitrile, 3,4-diethoxybenzoylacetonitrile, and 2,5-difluorobenzoylacetonitrile.

Example 6

p-Fluorobenzoylacetonitrile, thallium (I) salt

A suspension of 10 g. of p-fluorobenzoylacetonitrile in 200 ml. of dry diethyl ether is stirred at room temperature while 17.2 g of neat thallium (I) ethoxide is slowly added. The resulting reaction mixture is stirred for one hour at room temperature and then filtered. The collected precipitate is washed with diethyl ether and air dried whereby there is obtained 23.7 g. (99% yield) of the thallium (I) salt of p-fluorobenzoylacetonitrile which may be stored at room temperature.

In a similar manner, the thallium (I) salts tabulated below were prepared.

| Example No. | Thallium (I) salts |
|---|---|
| 7 | m-Chlorobenzoylacetonitrile, thallium (I) salt |
| 8 | p-Bromobenzoylacetonitrile, thallium (I) salt |
| 9 | o-Bromobenzoylacetonitrile, thallium (I) salt |
| 10 | 2,4-Dichlorobenzoylacetonitrile, thallium (I) salt |
| 11 | 3,4-Difluorobenzoylacetonitrile, thallium (I) salt |
| 12 | o-Ethylbenzoylacetonitrile, thallium (I) salt |
| 13 | p-*Iso*propylbenzoylacetonitrile, thallium (I) salt |
| 14 | m-*Iso*butylbenzoylacetonitrile, thallium (I) salt |
| 15 | 3,4-Dimethylbenzoylacetonitrile, thallium (I) salt |
| 16 | m-Ethoxybenzoylacetonitrile, thallium (I) salt |
| 17 | p-*Iso*propoxybenzoylacetonitrile, thallium (I) salt |
| 18 | 3,4-Diethoxybenzoylacetonitrile, thallium (I) salt |

Example 19

*Cis*-2-(p-fluorobenzoyl)-3-hydroxycrotononitrile

To a suspension of 2.2 g. of p-fluorobenzoylacetonitrile, thallium (I) salt in 20 ml. of tetrahydrofuran is added 2 ml. of acetyl fluoroide with stirring at room temperature. After one hour, another 2 ml. of acetyl fluoride is added, followed by 2 ml. more after twelve hours. Twelve hours later, the precipitated thallium (I) fluoride is removed by filtration and the filtrate is evaporated to dryness. The off-white residue is recrystallized from ethanol to provide the *cis*-2-(p-fluorobenzoyl)-3-hydroxycrotononitrile.

Example 20

Cis-2-(p-bromobenzoyl)-3-hydroxy-2-pentenonitrile

A suspension of 5.0 g. of p-bromobenzoylacetonitrile, thallium salt in 30 ml. of tetrahydrofuran is treated with an excess of propionyl fluoride. After 15 hours, the precipitate is filtered off and the filtrate is stripped to dryness. The residue is dissolved in chloroform and extracted with aqueous sodium bicarbonate. The aqueous phase is acidified and the product is extracted with chloroform. The title compound is obtained after column chromatography.

In a similar manner the acid fluorides listed below were reacted with the appropriately substituted benzoylacetonitrile thallium salts to provide the compounds tabulated below.

| Example No. | Product | Acid Fluoride | Thallium Salt from Example No. |
|---|---|---|---|
| 21 | Cis-2-(m-chlorobenzoyl)-3-hydroxy-2-hexenonitrile | Butyryl Fluoride | 7 |
| 22 | Cis-2-(p-bromobenzoyl)-3-hydroxy-2-pentenonitrile | Propionyl Fluoride | 8 |
| 23 | Cis-2-(o-bromobenzoyl)-3-hydroxy-4-methyl-2-pentenonitrile | Isobutyryl Fluoride | 9 |
| 24 | Cis-2-(2,4-dichlorobenzoyl)-3-hydroxy-2-heptenonitrile | Valeryl Fluoride | 10 |
| 25 | Cis-2-(3,4-difluorobenzoyl)-3-hydroxy-5-methyl-2-hexenonitrile | Isovaleryl Fluoride | 11 |
| 26 | Cis-2-(o-ethylbenzoyl)-3-hydroxy-4-methyl-2-hexenonitrile | 2-Methylbutyryl Fluoride | 12 |

| Example No. | Product | Acid Fluoride | Thallium Salt from Example No. |
|---|---|---|---|
| 27 | Cis-2-(p-*iso*propylbenzoyl)-3-hydroxy-4,4-dimethyl-2-pentenonitrile | Pivalyl Fluoride | 13 |
| 28 | Cis-2-(m-*iso*butylbenzoyl)-3-hydroxy-2-pentenonitrile | Propionyl Fluoride | 14 |
| 29 | Cis-2-(3,4-dimethylylbenzoyl)-3-hydroxy-hexenonitrile | Butyryl Fluoride | 15 |
| 30 | Cis-2-(m-ethoxybenzoyl)-3-hydroxy-4-methyl-2-pentenonitrile | Isobutyryl Fluoride | 16 |
| 31 | Cis-2-(p-*iso*propoxybenzoyl)-3-hydroxy-2-heptenonitrile | Valeryl Fluoride | 17 |
| 32 | Cis-2-(3,4-diethoxybenzoyl)-3-hydroxy-5-methyl-2-hexenonitrile | Isovaleryl Fluoride | 18 |

## Example 33
### Cis-2-(*p*-chlorobenzoyl)-3-hydroxycrotonitrile

To a mixture of 68.5 g. of *p*-chlorobenzonitrile and 24.0 g. of sodium hydride (50% in mineral oil) in 250 ml. of ether is added, 26.1 ml. of acetonitrile and 2 ml. of isopropanol. The mixture is stirred and refluxed on a steam bath for 6 hours and then stirred overnight at room temperature. A 5 ml. portion of methanol and 250 ml. of water are added. The ether is boiled away on a steam bath and the mixture is filtered. The solid is dissolved in warm ethyl acetate and filtered. The filtrate is evaporated to a solid giving β-amino-4-chlorocinnamonitrile.

A 94.68 g. portion of β-amino-4-chlorocinnamonitrile is suspended in 250 ml. of water. A 50 ml. portion of concentrated hydrochloric acid is added and the mixture is stirred overnight. The solid is collected and stirred for 4 hours in a mixture of 250 ml. of water, 250 ml. of ethanol and 50 ml. of concentrated hydrochloric acid. The reaction mixture is evaporated and the solid is taken up in methylene chloride and passed through Magnesol®. The filtrate is evaporated on a steam bath with the addition of hexanes giving crystals of *p*-chlorobenzoylacetonitrile.

A 9.0 g. portion of *p*-chlorobenzoylacetonitrile in 100 ml. of chloroform is reacted with 10 ml. of N,N-dimethylacetamide dimethylacetal as described in Example 2, giving the desired product.

## Example 34
### *Cis*-2-(*o*-chlorobenzoyl)-3-hydroxycrotononitrile

A 150 ml. portion of ammonia is condensed in a reaction flask and a small piece of sodium is added. The blue color is discharged by the addition of ferric chloride and 3.22 g. of sodium are added. After the color is discharged 5.75 ml. of acetonitrile in 10 ml. of ether are added. The reaction is cooled in a dry ice-acetone bath and 13.3 g of 2-chlorobenzonitrile in 25 ml. of tetrahydrofuran are added dropwise. The ammonia is allowed to evaporate, the solvent is blown off with nitrogen and water is added. The mixture is extracted with methylene chloride. The organic extracts are dried over sodium sulfate and filtered through Magnesol®. The filtrate is evaporated with the addition of hexanes giving β-amino-2-chlorocinnamonitrile as a white crystalline solid.

A 45 ml. portion of 1N hydrochloric acid is added to the β-amino-2-chlorocinnamonitrile and the reaction is stirred overnight. The solid is collected, washed with water, dried, taken up in methylene chloride and passed through Magnesol®. The filtrate is evaporated on a steam bath with the addition of hexanes giving *o*-chlorobenzoylacetonitrile as a white crystalline solid.

A 4.4 g. portion of *o*-chlorobenzoylacetonitrile in 50 ml. of chloroform is reacted with 5 ml. of N,N-dimethylacetamide dimethylacetal as described in Example 2, giving the desired product.

## Example 35
### *Cis*-2-(*m*-fluorobenzoyl)-3-hydroxycrotononitrile

To a solution of 4.7 g. of *m*-fluorobenzoylacetonitrile [Pihl, *et al.,* Reakts. Sposabnost. Org. Soedin. Tartu. Gos. Univ., *5* (1), 27 (1968)] in 50 ml. of chloroform, cooled in an ice bath, is added 5 ml. of N,N-dimethylacetamide dimethylacetal. The reaction mixture is stirred at 0°C. for 2 hours then at room temperature for 16 hours. The solution is evaporated *in vacuo* to an oil. The oil is dissolved in 50 ml. of methanol and 15 ml. of 1N hydrochloric acid is added. The reaction mixture is heated on a steam bath for 30 minutes and then evaporated *in vacuo* to an oil. This oil is added to 75 ml. of benzene and extracted three times with a saturated solution of sodium bicarbonate. The combined aqueous phase is

washed with benzene, acidified with concentrated hydrochloric acid and the precipitate is collected and recrystallized from isopropanol with charcoal treatment giving the desired product.

Example 36

*Cis*-2-(*o*-fluorobenzoyl)-3-hydroxycrotononitrile

A 9.8 g. portion of *o*-fluorobenzoylacetonitrile [Dorsch *et al.,* J.A.C.S. *54,* 2960 (1932)] in 100 ml. of chloroform is cooled in an ice-water bath. A 10 ml. portion of N,N-dimethylacetamide dimethylacetal is added and the mixture is stirred in an ice bath for 2 hours and then at room temperature overnight. The mixture is evaporated to an oil, dissolved in 50 ml. of methanol, acidified with 20 ml. of 1N hydrochloric acid, heated on a steam bath for 1/2 hour and evaporated to an oil. A 100 ml. portion of chloroform and 100 ml. of saturated aqueous sodium bicarbonate solution are added. The mixture is shaken and the organic phase is again extracted with saturated aqueous sodium bicarbonate solution. The aqueous phases are washed with chloroform, acidified with concentrated hydrochloric acid and the solid is collected, washed with water and recrystallized from hot isopropanol with charcoal treatment giving the desired product.

Example 37

*Cis*-2-(3-chloro-*o*-toluoyl)-3-hydroxycrotononitrile

A 45.0 g. portion of potassium tertiary butoxide is added to 700 ml. of ether and then cooled in an ice-salt bath. To this is added, over 15 minutes, a mixture of 60.0 g. of 3-chloro-2-methylbenzonitrile and 22.0 ml. of acetonitrile in 300 ml. of ether. The mixture is stirred in the ice bath for 1/2 hour, allowed to warm to room temperature and then stirred for one hour. This mixture is poured into one liter of water and the layers are separated. The aqeuous phase is extracted with ether and the ether layers are combined, washed four times with water and dried over magnesium sulfate. The crystals which form on evaporation are collected and recrystallized from 200 ml. of hot benzene giving 16.0 g. of $\beta$-amino-3-chloro-2-methylcinnamonitrile.

An 11.0 g. portion of the above product is added to 150 ml. of methanol and heated to solution on a steam bath. A 50 ml. portion of 1N hydrochloric acid is added and the mixture is heated on the steam bath for 3 hours. The mixture is cooled, 30 ml. of water are added, the precipitate is collected and air dried. This solid is recrystallized from 80 ml. of hot methanol with charcoal treatment giving 7.8 g. of 3-chloro-2-methylbenzoylacetonitrile.

A 5.4 g. portion of the above ketone in 75 ml. of chloroform is reacted with 60 ml. of N,N-dimethylacetamide dimethylacetal as described in Example 2, to give the desired product.

Example 38

*Cis*-3-hydroxy-2-(*p*-toluoyl)crotonitrile

To a solution of 10 g. of $\alpha$-bromo-*p*-methyl acetophenone in 50 ml. of ethanol is an ice bath is added dropwise a solution of 6.4 g. of sodium cyanide in 30 ml. of water, at a rate so that the temperature is maintained at 25°—30°C. The mixture is then stirred at room temperature for 2 hours, drowned in 600 ml. of water and filtered through Celite®. The filtrate is acidified to pH 5 with acetic acid, allowed to stand 10 minutes and then filtered. The solid is dissolved in 100 ml. of boiling benzene, magnesium sulfate is added together with charcoal and the mixture is filtered. (1) A 400 ml. portion of hexane is added to the filtrate and the mixture is cooled giving a 3.0 g. of *p*-methylbenzoylacetonitrile.

A 5.4 g. portion of the above ketone in 75 ml. of chloroform is reacted with 60 ml. of N,N-3 ml. portion of N,N-dimethylacetamide dimethylacetal is added and the mixture is allowed to stand in an ice bath for 2 hours and then at room temperature for 48 hours. The mixture is evaporated to an oil which is dissolved in 20 ml. of methanol. A 3 ml. portion of 1N hydrochloric acid is added and the mixture is heated on a steam bath for 1/2 hour and evaporated to a dark oil. This oil is dissolved in 75 ml. of benzene and extracted with three 50 ml. portions of saturated aqueous sodium bicarbonate. The combined aqueous phases are washed with benzene and acidified with concentrated hydrochloric acid. The precipitate is collected and recrystallized from 20 ml. of hot isopropanol with charcoal treatment, giving the desired product.

Example 39

*Cis*-3-hydroxy-2-(*o*-anisoyl) crotononitrile

A 10.0 g. portion of $\alpha$-bromo-*o*-methoxyacetophenone is suspended in 50 ml. of ethanol. A solution of 6.4 g. of sodium cyanide in 30 ml. of water is added as described in Example 38. Following the procedure of Example 38, there is obtained *o*-methoxybenzoylacetonitrile.

A 3.1 g. portion of *o*-methoxybenzoylacetonitrile is added to 50 ml. of chloroform cooled in an ice bath. A 3.5 ml. portion of dimethylacetamide dimethylacetal is added and the mixture is stirred in an ice bath for 2 hours and then at room temperature overnight. The mixture is evaporated to an oil which is dissolved in 25 ml. of methanol. A 5 ml. portion of 1N hydrochloric acid is added and the mixture is heated on a steam bath for 1/2 hour and then evaporated to an oil. This oil is dissolved in 50 ml. of benzene. A 50 ml. portion of saturated aqueous sodium bicarbonate solution is added forming two layers. The benzene layer is extracted with two 50 ml. portions of saturated aqueous sodium

becarbonate solution. The aqueous phases are combined, washed twice with 50 ml. of benzene and acidified with concentrated hydrochloric acid. The solid is collected, washed with water and recrystallized from hot isopropanol, with charcoal treatment, giving the desired product.

Example 40

*Cis*-3-hydroxy-2-(*p*-methoxybenzoyl)crotononitrile

A 25.0 g. portion of α-bromo-*p*-methoxyacetophenone is suspended in 125 ml. of ethanol. A solution of 16.0 g. of sodium cyanide in 75 ml. of water is added, using an ice-water bath to keep the temperature at 25°—30°C. The mixture is stirred for 45 minutes at room temperature. A total of 1.2 liters of water is added in 200 ml. increments with filtration through Celite® until no more precipitate forms. The filtrate is acidified to pH 5 with acetic acid. The solid is collected, washed with water, dried and recrystallized from benzene giving *p*-methoxybenzoylacetonitrile.

A 4.7 g. portion of *p*-methoxybenzoylacetonitrile is added to 50 ml. of chloroform, cooled in an ice bath. A 5 ml. portion of N,N-dimethylacetamide dimethylacetal is added and the mixture is stirred in an ice bath for 2 hours and then at room temperature overnight. The mixture is evaporated to an oil which is dissolved in 25 ml. of methanol. A 5 ml. portion of 1N hydrochloric acid is added, the mixture is heated on a steam bath for 1/2 hour and then evaporated to an oil. A 70 ml. portion of saturated aqueous sodium bicarbonate solution and 70 ml. of benzene are added and the mixture separates into two layers. The benzene layer is extracted twice with saturated aqueous sodium bicarbonate solution. The aqueous phases are combined, washed twice with benzene, acidified with 12N hydrochloric acid and the solid is collected. This solid is recrystallized from 50 ml. of hot isopropanol with charcoal treatment giving the desired product.

Example 41

*Cis*-3-hydroxy-2-(α,α,α-trifluoro-*p*-toluoyl)crotononitrile

An 8.0 g. portion of diisopropylamine in 100 ml. of ether is cooled to 0°C. under argon. A solution of 31.5 ml. of 2.5M *n*-butyllithium in hexane is added dropwise and the reaction is further cooled to −10°. A 5.94 g. portion of 5-methylisoxazole is then added dropwise and the mixture is stirred for 1/2 hour. A 15.0 g. portion of *p*-trifluoromethylbenzoylchloride is added while the reaction is held at 0°C. The mixture is allowed to warm to room temperature and stand for 18 hours. The reaction is quenched with water, acidified with dilute hydrochloric acid and extracted with ether. The ether phase is treated with aqueous sodium carbonate producing three phases. The dark middle phase is isolated, acidified and extracted with chloroform. The extract is dried, filtered and evaporated. The residue is recrystallized from ethanol-hexane giving the desired compound.

Example 42

Cyanoacetone, sodium salt

A solution of 0.174 mole of sodium ethoxide is prepared by dissolving 4.0 g. of sodium in 200 ml. of absolute ethanol. A neat sample of 15 ml. (0.184 mole) of 5-methylisoxazole is then added dropwise as a colorless precipitate forms. When the addition is complete, the mixture is cooled in an ice bath and then filtered. The precipitate is collected, and washed with hexane, yielding 14.0 g. of colorless product.

Example 43

*Cis*-3-hydroxy-2-(α,α,α-trifluoro-*m*-toluoyl)-crotononitrile

A mixture of 5.0 g. (48 mmole) of cyanoacetone, sodium salt in 20 ml. of tetrahydrofuran is stirred at room temperature as a solution of 3.3 g. (16 mmole) of m-trifluoromethylbenzoyl chloride in 5 ml. of tetrahydrofuran is added. The reaction is heated to reflux for 2 hours and the solvent is then evaporated. The residue is acidified and extracted with chloroform. Evaporation of the organic phase provides 4.5 g. of an orange solid which is recrystallized from chloroform to provide 2.4 g. of the title compound.

Example 44

1-Cyano-2-butanone, sodium salt

A solution of 0.174 moles of sodium ethoxide is prepared by dissolving 4.0 g. of sodium in 200 ml. of absolute ethanol. A neat sample of 5-ethylisoxazole (17.9 g., 0.185 mole) is then added. The reaction is stirred for one hour as a colorless precipitate forms. The mixture is cooled in an ice bath and filtered. The precipitate is washed with hexane and air dried, yielding the title compound.

Example 45

*Cis*-2-(p-bromobenzoyl)-3-hydroxy-2-pentenonitrile

A mixture of 3.0 g. (25 mmole) of 1-cyano-2-butanone, sodium salt in 20 ml. of tetrahydrofuran is stirred as a solution of (8.6 mmole) of p-bromobenzoyl chloride in 6 ml. of tetrahydrofuran is added. The reaction is heated to reflux for 3 hours, then cooled and the solvent evaporated. The residue is acidified and extracted with chloroform. The organic phase is extracted twice with aqueous sodium bicarbonate which in turn is acidified and extracted again with chloroform. The organic phase is dried and

evaporated to yield the title compound.

Example 46
*Cis*-2-($\alpha$,$\alpha$,$\alpha$-trichloro-*p*-toluoyl)-3-hydroxy-crotononitrile

In the manner described in Example 41, reaction of 5-methylisoxazole with *p*-trichloromethylbenzoyl chloride provides the corresponding *cis*-2-(*p*-trichloromethylbenzoyl)-3-hydroxy-crotononitrile.

Example 47
Preparation of 50 mg. Tablets

| Per Tablet | | Per 10,000 Tablets |
|---|---|---|
| 0.050 gm. | One of the active compounds listed below | 500 gm. |
| 0.080 gm. | Lactose | 800 gm. |
| 0.010 gm. | Corn Starch (for Mix) | 100 gm. |
| 0.008 gm. | Corn Starch (for paste) | 75 gm. |
| 0.148 gm. | | 1475 gm. |
| 0.002 gm. | Magnesium Stearate (1%) | 15 gm. |
| 0.150 gm. | | 1490 gm. |

Active compounds for above preparation:

*Cis*-2-(p-fluorobenzoyl)-3-hydroxycrotononitrile

*Cis*-2-benzoyl-3-hydroxycrotononitrile

*Cis*-2-(5-fluoro-2-thenoyl-3-hydroxycrotononitrile

The active compound, lactose and corn starch (for mix) are blended together. The corn starch (for paste) is suspended in 600 ml. of water and heated with stirring to form a paste. This paste is then used to granulate the mixed powders. Additional water is used if necessary. The wet granules are passed through a No. 8 hand screen and dried at 120°F. The dry granules are then passed through a No. 16 screen. The mixture is lubricated with 1% magnesium stearate and compressed into tablets in a suitable tableting machine.

Example 48
Preparation of Oral Suspension

| Ingredient | Amount |
|---|---|
| One of the active compounds listed below | 500 mg. |
| Sorbitol solution (70% N.F.) | 40 ml. |
| Sodium benzoate | 150 mg. |
| Saccharin | 10 mg. |
| Red dye | 10 mg. |
| Cherry flavor | 50 mg. |
| Distilled water qs ad | 100 ml |

Active compounds for above preparation:

*Cis*-2-(p-bromobenzoyl)-3-hydroxy-2-pentenonitrile

*Cis*-2-benzoyl-3-hydroxy-2-pentenonitrile

*Cis*-2-(5-fluoro-3-thenoyl)-3-hydroxy-2-pentenonitrile

The sorbitol solution is added to 40 ml. of distilled water and the active compound is suspended therein. The saccharin, sodium benzoate, flavor and dye are added and dissolved. The volume is adjusted to 100 ml. with distilled water. Each ml. of syrup contains 5 mg. of the active compound.

Example 49
Preparation of Parenteral Solution

In a solution of 700 ml. of propylene glycol and 200 ml. of water for injection is suspended 20.0 grams of one of the below-listed active compounds with stirring. After suspension is complete, the pH is adjusted to 5.5 with hydrochloric acid and the volume is made up to 1000 ml. with water for injection. The formulation is sterilized, filled into 5.0 ml. ampoules each containing 2.0 ml. (representing 40 mg. of drug) and sealed under nitrogen. Active compounds for the above preparation:

*Cis*-3-hydroxy-2-($\alpha$,$\alpha$,$\alpha$-trifluoro-p-toluoyl)crotononitrile
*Cis*-2-benzoyl-3-hydroxy-2-hexenonitrile
*Cis*-3-hydroxy-2-(5-bromo-2-thenoyl)crotononitrile.

Example 50
Preparation of Topical Cream

| Ingredient | Amount |
|---|---|
| One of the active compounds listed below | 1.0% |
| Ethoxylated stearyl alcohol | 10.0% |
| Benzyl alcohol | 0.9% |
| Isopropyl palmitate | 5.0% |
| Sorbitol solution (USP) | 5.0% |
| Glycerin | 5.0% |
| Lactic acid qs to pH 4.0—5.0 | |
| Water qs ad | 100.0% |

Active compounds for the above preparation:

*Cis*-2-(2-methyl-4-*iso*propoxybenzoyl)-3-hydroxy-4-methyl-2-pentenonitrile
*Cis*-2-benzoyl-3-hydroxy-4-methyl-2-pentenonitrile
*Cis*-2-(5-bromo-3-thenoyl)-3-hydroxy-4-methyl-2-pentenonitrile

The ethoxylated stearyl alcohol and isopropyl palmitate are heated to liquefying temperature. About 95% of the total volume of water is placed in a separate container followed by the glycerin and sorbitol solution. This aqueous mixture is brought to a boil and then cooled to 60°—75°C. The active compound is added to the wax phase and the mixture is stirred until a clear solution is obtained. The benzyl alcohol is added and dissolved in the wax phase. The water phase is passed through a screen into the wax phase while maintaining agitation. Both phases are kept at about the same temperature during transfer. The mixture is cooled while agitation is continued. At a temperature of 50°—55°C. the balance of the water is added. The pH is adjusted to 4.0—5.0 with lactic acid. The batch is cooled with minimum agitation until the cream sets in its final form.

Example 51
Preparation of Intra-articular Product

| Ingredient | Amount |
|---|---|
| One of the active compounds listed below | 2—20 mg. |
| NaCl (physiological saline) | 0.9% |
| Benzyl alcohol N.F. | 0.9% |
| Sodium carboxymethylcellulose | 1—5% |
| pH adjusted to 5.0—7.5 | |
| Water for injection qs ad | 100% |

Active compounds for the above preparation:

*Cis*-2-(2-methoxy-4-trifluoromethylbenzoyl)-3-hydroxy-2-heptenonitrile
*Cis*-2-benzoyl-3-hydroxy-2-heptenonitrile
*Cis*-2-(5-chloro-3-thenoyl)-3-hydroxy-2-heptenonitrile

Example 52
Preparation of Injectable Depo Suspension

| Ingredient | % W/V |
|---|---|
| One of the active compounds listed below | 0.05—5 |
| Polysorbate 80 USP | 0.2 |
| Polyethylene glycol 4000 USP | 3.0 |
| Sodium chloride USP | 0.8 |
| Benzyl alcohol N.F. | 0.9 |
| HCl to pH 6—8 | qs |
| Water for injection qs ad | 100.0 |

Active compounds for the above preparation:
*Cis*-2-(3,4-difluorobenzoyl)-3-hydroxy-4,4-dimethyl-2-pentenonitrile
*Cis*-2-benzoyl-3-hydroxy-4,4-dimethyl-2-pentenonitrile

Example 53
2,5-Dichloro-β-aminocinnamonitrile

To a dry 250 ml. three-neck flask is added 30 ml. of hexane under argon, followed by 2.2 g. (46 mmole) of 50% sodium hydride in oil. The mixture is stirred for 5 minutes, the hexane is siphoned off and replaced by 30 ml. of ether. A solution of 7.0 g. (41 mmole) of 2,5-dichlorobenzonitrile, 2,4 ml. (46 mmole) of acetonitrile and 0.4 ml. (4 mmole) of t-butanol diluted to 45 ml. with ether is then added dropwise to the stirred sodium hydride mixture. When the addition is complete, the mixture is heated to reflux overnight. After 24 hours, 40 ml. of water is cautiously added and the ether phase is separated. This solution is dried and evaporated to yield 6.4 g. of off-white solid. This residue is dissolved in chloroform, filtered through a Magnesol® pad and the filtrate is concentrated on the steam bath and diluted with hexane. Upon cooling, 5.0 g. of colorless product precipitated.

Similarly prepared is 3,5-dimethoxy-β-aminocinnamonitrile; 3,4,5-trimethoxy-β-aminocinnamonitrile; and 2,4,6-trimethyl-β-aminocinnamonitrile.

Example 54
2,5-Dichlorobenzoylacetonitrile

A two-phase mixture of 5.0 g. of 2,5-dichloro-β-aminocinnamonitrile, 50 ml. of chloroform, and 30 ml. of 3N aqueous hydrochloric acid is stirred overnight at 25°C. After 15 hours the layers are separated and the chloroform phase is dried over sodium sulfate, and filtered through a pad of Magnesol®. The product is crystallized directly from the filtrate to provide 4.3 g. of colorless solid.

Similarly prepared is 3,5-dimethoxybenzoylacetonitrile, 3,4,5-trimethoxybenzoylacetonitrile and 2,4,6-trimethylbenzoylacetonitrile.

Example 55
*Cis*-2-(3,4-difluorobenzoyl)-3-hydroxycrotononitrile

A solution of 10.2 g. of 3,4-difluorobenzoylacetonitrile, 9.2 g. of triethyl orthoacetate and 20 ml. of acetic anhydride is heated on a steam bath for 0.5 hour and then poured into 200 ml. of water which is then heated on the steam bath for 2 hours. On cooling, the solid is filtered and then dissolved in methylene chloride. This solution is extracted with two portions of aqueous sodium bicarbonate and the aqueous extracts are combined and acidified with concentrated hydrochloric acid. The precipitate is extracted into methylene chloride, passed through a pad of Magnesol, heated and diluted with hexane. Upon cooling, colorless crystals result, yielding the title compound, m.p. 51°—54°C.

Similarly prepared are *cis*-2-(2,5-difluorobenzoyl)-3-hydroxycrotononitrile, *cis*-2-(3,4,5-trimethoxybenzoyl)-3-hydroxycrotononitrile, *cis*-2-(2,4,6-trimethylbenzoyl)-3-hydroxycrotononitrile, and *cis*-2-(2,5-dichlorobenzoyl)-3-hydroxycrotononitrile.

Example 56
2-(Pentafluorobenzoyl)-3-hydroxycrotononitrile

A mixture of 6.8 g. of cyanoacetone, sodium salt in 70 ml. of dry tetrahydrofuran is cooled in ice and 5.0 g. of pentafluorobenzoyl chloride diluted to 10 ml. with tetrahydrofuran is added dropwise. The reaction is stirred overnight and then the solvent is evaporated. The residue is acidified and extracted into chloroform. This chloroform phase is then extracted with aqueous sodium bicarbonate which in

turn is acidified and the colorless precipitate is again extracted with chloroform. Upon evaporation of the solvent, a yellow semi-solid results which is recrystallized from ethyl ether (—78°C.) to afford off-white crystals, m.p. 62°—63°C.

Similarly prepared is 2-(2-chloro-6-fluorobenzoyl)-3-hydroxycrotononitrile.

Example 57

*Cis*-2-(*p*-fluorobenzoyl)-3-hydroxycrotononitrile

A mixture of 1.0 g (9.5 mmole) of cyanoacetone, sodium salt in 20 ml. of tetrahydrofuran is stirred in room temperature as a solution of 0.37 ml. (3.2 mmole) of *p*-fluorobenzoyl chloride in 5 ml. of tetrahydrofuran is added. The reaction is heated to reflux for 2 hours and the solvent is then evaporated. The residue is acidified and extracted with diethyl ether. Evaporation of the organic phase provides 0.7 g. of a yellow oil which crystallized on standing to provide the title compound.

Example 58

2-(*p*-Fluorobenzoyl)-3-methoxycrotononitrile

A solution containing 55.8 g. (0.34 mole) of *p*-fluorobenzoylacetonitrile, 41 g. (0.34 mole) of trimethyl orthoacetate, and 90 g. (0.88 mole) of acetic anhydride is heated to reflux for 5 hours. The excess solvent and volatile by-products are then removed by vacuum distillation and the residue is recrystallized from diethyl ether. Alternatively, the distillation residue may be distilled over on a Kugelrohr apparatus at 160°C./0.25 mm. to provide a viscous oil which may be crystallized from diethyl ether to provide the pure title compound.

Example 59

*Cis*-2-(*p*-fluorobenzoyl)-3-hydroxycrotononitrile

A solution of 5 g. of *cis*-2-(*p*-fluorobenzoyl)-3-methoxycrotononitrile in 50 ml. of ethanol is treated with 25 ml. of 3N aqueous hydrochloric acid at room temperature for one hour. The solvent is evaporated, the residue diluted with water, and the product is extracted with chloroform. The organic phase is separated, dried, filtered and evaporated to provide the title compound.

Example 60

2-(Dimethylaminomethylene)-2-(*p*-fluorophenyl)-butane-1,3-dione

A solution of 15 g. of *p*-fluorobenzoylacetone in 35 ml. of N,N-dimethylformamide dimethylacetal is heated on a steam bath and the volatile by-product (methanol) is distilled out. When the theoretical amount of methanol has been collected, the reaction mixture is pumped dry, then the product is collected by distillation from the reaction pot by 150°C./0.025 mm. Crystalline product (19.5 g., 97% yield) is thus obtained upon cooling of the distillate.

Example 61

4-Acetyl-5-(p-fluorophenyl)isoxazole and 4-(p-fluorobenzoyl)-5-methylisoxazole

To a solution of 12.8 g. of 2-(dimethylaminomethylene)-1-(*p*-fluorophenyl)butane-1,3-dione in 130 ml. of THF is added 4.10 g. of hydroxylamine hydrochloride, followed by 40 ml. of water. The reaction is heated to reflux for 2 hours, then cooled and the solvent is evaporated. The residue is partitioned between ether and water and the organic layer is separated and evaporated. The residue is distilled at 100—110°C./0.25 mm. to provide 9.0 g. of an oily mixture of approximated equal amounts of 4-(*p*-fluorobenzoyl)-5-methylisoxazole and 4-acetyl-5-(*p*-fluorophenyl)isoxazole.

Example 62

*Cis*-2-(*p*-fluorobenzoyl)-3-hydroxycrotononitrile

A solution of 1.65 g. of a mixture of 4-acetyl-5-(*p*-fluorophenyl)isoxazole and 4-(*p*-fluorobenzoyl)-5-methylisoxazole in equal amounts is dissolved in 7 ml. of methanol and treated with 5 ml. of 10% aqueous sodium hydroxide. The solution is heated on a steam bath for 15 minutes then cooled, acidified with 1N aqueous HCl and extracted with ether. Evaporation of the ether provides 1.55 g. of product. Recrystallization from *iso*-propanol provides 1.0 g. (66%) of *cis*-2-(*p*-fluorobenzoyl)-3-hydroxycrotononitrile.

Example 63

3-Amino-2-(p-fluorobenzoyl)crotononitrile

Approximately 50 ml. of liquid ammonia is condensed in a three-neck flask cooled to —78° in a dry ice-acetone bath. To this is added 200 mg. of sodium metal, followed by 100 mg. of ferric chloride hexahydrate. When the blue color had vanished, 6.2 g. of 2-(*p*-fluorobenzoyl)-3-methoxycrotononitrile is added. After stirring for one hour, the reaction is allowed to warm and the solvent evaporate. The residue is dissolved in hot ethyl acetate and filtered through Celite®. The filtrate is concentrated on the steam bath and diluted with hexane. Upon cooling, the crystalline product precipitates, is filtered and dried.

Example 64

2-(p-Fluorobenzoyl)-3-methylaminocrotonitrile

In the glass liner of a stainless steel bomb cooled to —78° is condensed 10 ml. of methylamine. To this is added 0.5 ml. of 2M *n*-butyllithium (in hexane), followed by 5.0 g. of 2-(*p*-fluorobenzoyl)-3-methoxycrotononitrile. The bomb is sealed and heated on a steam bath overnight. The bomb is then cooled, opened and the contents evaporated. The residue is dissolved in chloroform, passed through a Magnesol® pad and the filtrate is evaporated. The residue is recrystallized from chloroformhexane yielding the title compound.

Example 65

2-(p-Fluorobenzoyl)-3-butylaminocrotononitrile

A sample of 2.5 g. of 2-(*p*-fluorobenzoyl)-3-methoxy crotononitrile is dissolved in 10 ml. of *n*-butylamine. The solution is sealed in a stainless steel bomb and heated on a steam bath overnight. The bomb is then opened and the solvent evaporated under reduced pressure. A greenish oil is obtained which is distilled on a Kugelrohr apparatus at 200°C./0.1 mm. to obtain the yellow oily product.

Example 66

2-(p-Fluorobenzoyl)-3-dimethylaminocrotononitrile

To a solution of 14.5 g. (0.10 m) of (*p*-fluorobenzoyl)acetonitrile in 100 ml. of chloroform, cooled in an ice-salt bath, was added 13.3 g. (0.1 m) of N,N-dimethylacetamide dimethylacetal in 20 ml. chloroform. The reaction mixture was stirred in the ice-salt bath for 2 hours, then evaporated *in vacuo* to an orange oil. The oil was dissolved in 150 ml. benzene and passed through Magnesol®. The filtrate was evaporated and the oil thus obtained was dissolved in 50 ml. benzene to which petroleum ether was added until the solution became cloudy. The precipitate which formed upon cooling was collected. The solid was recrystallized twice from benzene-petroleum ether, with charcoal treatment to yield 5.8 g. (27%) of light yellow crystals, m.p. 90°—92°C.

Example 67

2-(p-Fluorobenzoyl)-3-hydroxycrotononitrile

A solution of 1.35 g. (0.006 m) of 2-(*p*-fluorobenzoyl)-3-dimethylaminocrotononitrile in a mixture of 15 ml. methanol and 8 ml. of 1N HCl was heated on the steam bath in an open beaker for 45 minutes. Upon cooling in an ice bath, a solid formed, was collected, and then recrystallized from cyclohexanepetroleum ether with charcoal treatment, giving 0.7 g. (64%) of light pinkish crystals, m.p. 65°—67°C.

Example 68

2-(p-Fluorobenzoyl)-3-methoxy-2-pentenonitrile

A solution containing 30 g. of (*p*-fluorobenzoyl)-acetonitrile, 36.4 g. of trimethylorthopropionate and 57 g. of acetic anhydride is heated in an oil bath at 130° for 3 hours. The ethanol and excess acetic anhydride is distilled off and the residue is diluted with chloroform. The chloroform solution is passed quickly through Magnesol® and concentrated, then cooled to produce the title product.

Example 69

2-(p-Fluorobenzoyl)-3-hydroxy-2-pentenonitrile

A two-phase mixture of 5.0 g. 2-(*p*-fluorobenzoyl)-3-methoxy-2-pentenonitrile in 50 ml. of chloroform is stirred with a 50 ml. solution of aqueous 1N hydrochloric acid. After 24 hours, the organic phase is separated and evaporated. The residue is recrystallized from chloroform to yield the title compound.

Example 70

2-(p-Fluorobenzoyl)-3-N-piperidylcrotononitrile

To a solution of 5.0 g. of 2-(*p*-fluorobenzoyl)-3-methoxycrotononitrile in 50 ml. of dry tetrahydrofuran is added 5 ml. of piperidine. The reaction is heated to reflux for 2 hours, cooled and evaporated *in vacuo*. The residue is recrystallized from chloroform/hexane to provide the crystalline title compound.

Example 71

2-(p-Fluorobenzoyl)-3-diethylaminocrotononitrile

A volume of 10 ml. of diethylamine is condensed in a glass tube at —78°C. A sample of 5 g. of 2-(*p*-fluorobenzoyl)-5-methoxycrotononitrile is added, the tube is sealed in stainless steel bomb and heated on a steam bath overnight. The bomb is then cooled, opened, and the contents evaporated. The residue was recrystallized from chloroform to provide the title compound.

Example 72

2-(p-Fluorobenzoyl)-3-hydroxycrotononitrile, sodium salt

A solution of 5.0 g. of 2-(*p*-fluorobenzoyl)-3-hydroxycrotononitrile in 30 ml. of dry tetrahydrofuran is added dropwise to a stirred suspension of sodium hydride (1.0 g.) in 30 ml. of dry tetrahydrofuran. After the addition is complete, the reaction mixture is stirred for 2 hours and the resulting orange solution is poured into diethyl ether. A colorless solid precipitates which is filtered and dried to provide the title compound.

Example 73

2-(p-Fluorobenzoyl)-3-hydroxycrotononitrile, triethyl ammonium salt

A solution of 1.3 ml. of triethyl amine in 10 ml. of diethyl ether is added dropwise to a solution of 1.5 g. of 2-(*p*-fluorobenzoyl)-3-hydroxycrotononitrile in 20 ml. of diethyl ether. After stirring for 1 hour, a yellow oil separated which crystallizes upon evaporation of the solvent. The product was filtered, washed with ether and dried, providing the title compound.

Example 74

Benzoylacetonitrile, thallium (I) salt

A suspension of 10 g. of benzoylacetonitrile in 200 ml. of dry diethyl ether is stirred at room temperature while 17.2 g. of neat thallium (I) ethoxide is slowly added. The resulting reaction mixture is stirred for one hour at room temperature and then filtered. The collected precipitate is washed with diethyl ether and air dried whereby there is obtained 23.7 g. (99% yield) of the thallium (I) salt of benzoylacetonitrile which may be stored indefinitely at room temperature.

Example 75

*Cis*-2-benzoyl-3-hydroxycrotononitrile

To a suspension of 2.2 g. of benzoylacetonitrile thallium (I) salt in 20 ml. of tetrahydrofuran is added 2 ml. of acetyl fluoride with stirring at room temperature. After one hour, another 2 ml. of acetyl fluoride is added, followed by 2 ml. more after twelve hours. Twelve hours later, the precipitated thallium (I) fluoride is removed by filtration and the filtrate is evaporated to dryness. The off-white residue is recrystallized from ethanol to provide the *cis*-2-benzoyl-3-hydroxycrotononitrile which has been described by Musante, *Gazz. Chim. Ital. 69,* 523 (1939).

Example 76

*Cis*-2-benzoyl-3-hydroxy-2-pentenonitrile

To a suspension of 5 g. of benzoylacetonitrile, thallium (I) salt in 50 ml. of diethyl ether is added a solution of 5 g. of propionyl fluoride in 10 ml. of diethyl ether with stirring at room temperature. After 12 hours, the reaction mixture is filtered and the filtrate evaporated and then pumped dry on a vacuum pump for one hour. The residue is then treated with chloroform and filtered and the solid collected provides the title compound.

Example 77

*Cis*-2-benzoyl-3-hydroxy-2-hexenonitrile

The procedure of Example 75 is repeated substituting an equimolecular amount of butyryl fluoride for the acetyl fluoride employed in that example. There is thus obtained the *cis*-2-benzoyl-3-hydroxy-2-hexenonitrile after purification by column chromatography.

Example 78

*Cis*-2-benzoyl-3-hydroxy-4-methyl-2-pentenonitrile

By replacing the acetyl fluoride employed in Example 75 with an equimolar amount of *iso*butyryl fluoride, there is obtained the corresponding *cis*-2-benzoyl-3-hydroxy-4-methyl-2-pentenonitrile after purification by column chromatography.

Example 79

*Cis*-2-benzoyl-3-hydroxy-2-heptenonitrile

The general procedure of Example 75 is repeated but replacing the acetyl fluoride employed in that Example with an equivalent amount of valeryl fluoride whereby there is obtained the *cis*-2-benzoyl-3-hydroxy-2-heptenonitrile after purification by column chromatography.

Example 80

2-Methylbutyryl fluoride

A sample of 2-methylbutyryl chloride in dimethylformamide is treated with a threefold excess of sodium fluoride. The temperature is raised and the pure acid fluoride is collected by distillation from the reaction mixture.

Example 81

*Cis*-2-benzoyl-3-hydroxy-4-methyl-2-hexenonitrile

Following the general procedure of Example 76, benzoylacetonitrile, thallium (I) salt is treated with 2-methylbutyryl fluoride to give the *cis*-2-benzoyl-3-hydroxy-4-methyl-2-hexenonitrile after

purification by column chromatography.

Example 82

*Cis*-2-benzoyl-3-hydroxy-5-methyl-2-hexenonitrile

Treatment of benzoylacetonitrile, thallium (I) salt with *iso*valeryl fluoride by the procedure described in Example 76 is productive of the *cis*-2-benzoyl-3-hydroxy-5-methyl-2-hexenonitrile after purification by column chromatography.

Example 83

*Cis*-2-benzoyl-3-hydroxy-4,4-dimethyl-2-pentenonitrile

In the manner described in Example 76, reaction of benzoylacetonitrile, thallium (I) salt with pivalyl fluoride provides the corresponding *cis*-2-benzoyl-3-hydroxy-4,4-dimethyl-2-pentenonitrile after purification by column chromatography.

Example 84

*Cis*-2-benzoyl-3-hydroxycrotononitrile, sodium salt

A calculated concentration of sodium hydroxide in water is prepared and an excess of *cis*-2-benzoyl-3-hydroxy-crotononitrile is added and stirred for half an hour. The mixture is filtered and the filtrate is evaporated. The gummy residue is dissolved in dry acetone and again evaporated, providing the title compound.

Example 85

*Cis*-2-benzoyl-3-hydroxy-2-pentenonitrile, triethylammonium salt

A sample of *cis*-2-benzoyl-3-hydroxy-2-pentenonitrile is dissolved in dry diethyl ether and an equivalent amount of triethylamine is added dropwise. The mixture is cooled in ice and the precipitate is removed by filtration to provide the title compound.

Example 86

*Cis*-2-benzoyl-3-hydroxycrotononitrile, copper (II) salt

A weighed sample of *cis*-2-benzoyl-3-hydroxycrotononitrile is dissolved in a dilute solution of ammonium hydroxide and an equivalent quantity of cupric sulfate in water is added. The blue solution is heated on a steam bath for 3 hours and cooled. The precipitate is removed by filtration and washed with water to yield the title compound.

Example 87

*Cis*-2-benzoyl-3-hydroxycrotononitrile

A mixture of 1.0 g. (9.5 mmole) of cyanoacetone, sodium salt in 20 ml. of tetrahydrofuran is stirred at room temperature as a solution of 0.37 ml. (3.2 mmole) of benzoyl chloride in 5 ml. of tetrahydrofuran is added. The reaction is heated to reflux for 2 hours and the solvent is then evaporated. The residue is acidified and extracted with diethyl ether. Evaporation of the organic phase provides 0.7 g. of yellow oil which crystallizes on standing to provide the title compound.

Example 88

*Cis*-2-benzoyl-3-hydroxy-2-pentenonitrile

The procedure of Example 45 is repeated but the *p*-bromobenzoyl chloride is replaced by 0.9 g. (8.6 mmole) of benzoyl chloride, and there is obtained the title compound.

Example 89

2-(*p*-Fluorobenzoyl)-3-ethoxycrotononitrile

A solution of 16.3 g of *p*-fluorobenzoylacetonitrile and 18.3 ml. of triethyl orthoacetate in 35 ml. of acetic anhydride is heated on a steam bath for 2.5 hours. The solvents are removed *in vacuo* and the residue is distilled at 165°—167°C./300 $\mu$. Upon cooling, this distillate solidifies and is recrystallized from methylene chloride/hexane to give the desired 2-(*p*-fluorobenzoyl)-3-ethoxy-crotononitrile, m.p. 77°/89°C.

Example 90

2-Benzoyl-3-methylaminocrotononitrile

In the glass liner of a stainless steel bomb cooled to —78° is condensed 10 ml. of methylamine. To this is added 0.5 ml. of 2M *n*-butyllithium (in hexane), followed by 5.0 g. of 2-benzoyl-3-methoxycrotononitrile. The bomb is sealed and heated on a steam bath overnight. The bomb is then cooled, opened and the contents evaporated. The residue is dissolved in chloroform, passed through a Magnesol® pad and the filtrate is evaporated. The residue is recrystallized from chloroformhexane yielding the title compound.

Example 91

2-Benzoyl-3-butylaminocrotononitrile

A sample of 2.5 g. of 2-benzoyl-3-methoxycrotononitrile is dissolved in 10 ml. of *n*-butylamine. The solution is sealed in a stainless steel bomb and heated on a steam bath overnight. The bomb is then opened and the solvent evaporated under reduced pressure. A greenish oil is obtained which is distilled on a Kugelrohr apparatus at 200°C./0.1 mm. to obtain the yellow oily product.

Example 92

2-Benzoyl-3-dimethylaminocrotononitrile

To a solution of 14.5 g. (0.10 m) of benzoylacetonitrile in 100 ml. of chloroform, cooled in an ice-salt bath, was added 13.3 g. (0.1 m) of N,N-dimethylacetamide dimethylacetal in 20 ml. chloroform. The reaction mixture was stirred in the ice-salt bath for 2 hours, then evaporated *in vacuo* to an orange oil. The oil was dissolved in 150 ml. benzene and passed through Magnesol®. The filtrate was evaporated and the oil thus obtained was dissolved in 50 ml. benzene to which petroleum ether was added until the solution became cloudy. The precipitate which formed upon cooling was collected. The solid was recrystallized twice from benzene petroleum ether, with charcoal treatment to yield 5.8 g. (27%) of light yellow crystals, m.p. 105°—106°C.

Example 93

*Cis*-2-benzoyl-3-hydroxycrotononitrile

A solution of 1.35 g. (.006 m) of 2-benzoyl-3-dimethylaminocrotononitrile in a mixture of 15 ml. methanol and 8 ml. of 1N HCl was heated on the steam bath in an open beaker for 45 minutes. Upon cooling in an ice bath, a solid formed, was collected and then recrystallized from cyclohexane-petroleum ether with charcoal treatment, giving 0.7 g. (64%) light pinkish crystals, m.p. 71°—73°C.

Example 94

2-Benzoyl-3-methoxy-2-pentenonitrile

A solution containing 30 g. of benzoylacetonitrile, 36.4 g. of triethylorthopropionate and 57 g. of acetic anhydride is heated in an oil bath at 130° for 3 hours. The ethanol and excess acetic anhydride is distilled off and the residue is diluted with chloroform. The chloroform solution is passed quickly through Magnesol® and concentrated, then cooled to produce the title product. Similarly prepared is 2-benzoyl-3-methoxy-2-hexenonitrile and 2-benzoyl-3-methoxy-2-heptenonitrile.

Example 95

*Cis*-2-benzoyl-3-hydroxy-2-pentenonitrile

A two-phase mixture of 5.0 g. 2-benzoyl-3-methoxy-2-pentenonitrile in 50 ml. of chloroform is stirred with a 50 ml. solution of aqueous 1N hydrochloric acid. After 24 hours, the organic phase is separated and evaporated. The residue is recrystallized from chloroform to yield the title compound. Similarly, *cis*-2-benzoyl-3-hydroxy-2-hexenonitrile is prepared from 2-benzoyl-3-methoxy-2-hexenonitrile and *cis*-2-benzoyl-3-hydroxy-2-heptenonitrile is prepared from 2-benzoyl-3-methoxy-2-heptenonitrile.

Example 96

2-Benzoyl-3-N-piperidylcrotononitrile

To a solution of 5.0 g. of 2-benzoyl-3-methoxy-crotononitrile in 50 ml. of dry tetrahydrofuran is added 5 ml. of piperidine. The reaction is heated to reflux for 2 hours, cooled and evaporated *in vacuo*. The residue is recrystallized from chloroform/hexane to provide the crystalline title compound.

Similarly prepared are 2-benzoyl-3-N-piperidylcrotononitrile, 2-benzoyl-3-N-pyrazoylcrotononitrile and 2-benzoyl-3-N-piperazylcrotononitrile.

Example 97

2-Benzoyl-3-dimethylaminocrotononitrile

A volume of 10 ml. of diethylamine is condensed in a glass tube at —78°C. A sample of 5 g. of 2-benzoyl-5-methoxycrotononitrile is added, the tube is sealed in a stainless steel bomb and heated on a steam bath overnight. The bomb is then cooled, opened, and the contents evaporated. The residue was recrystallized from chloroform to provide the title compound.

Similarly prepared were 2-benzoyl-3-diethylaminocrotononitrile and 2-benzoyl-3-di-isopropylaminocrotononitrile.

Example 98

2-(*p*-Fluorobenzoyl)-3-dimethylaminocrotononitrile

Dimethylamine is bubbled into a solution of 2-(*p*-fluorobenzoyl)-3-ethoxycrotononitrile in 50 ml. of diethyl ether for 15 minutes. After stirring at room temperature for one hour, the reaction mixture is allowed to evaporate to dryness and the resulting solid is recrystallized from methylene chloride/hexane to give 2-(*p*-fluorobenzoyl)-3-dimethylaminocrotononitrile, m.p. 65°—67°C.

Example 99

*Cis*-2-(5-chloro-o-toluoyl)-3-hydroxycrotononitrile

The procedure of Example 37 is repeated but the 3-chloro-2-methylbenzonitrile is replaced by the same amount of 5-chloro-2-methylbenzonitrile, and there is obtained the title compound.

Example 100

*Cis*-2-(2,4-dichlorobenzoyl)-3-hydroxycrotononitrile

The procedure of Example 34 is repeated, but the 2-chlorobenzonitrile is replaced by 16.6 g. of 2,4-dichlorobenzonitrile, and there is obtained the title compound.

Example 101

*Cis*-2-(2-thenoyl)-3-hydroxycrotononitrile

A 67 ml. portion of ethyl 2-thiophenecarboxylate is added to 56 g. of potassium *t*-butoxide (exothermic). After cooling, 33 ml. of acetonitrile is added and the mixture is stirred and heated on a steam bath for 1.5 hours. A 250 ml. portion of water is added followed by 200 ml. of methylene chloride. The mixture is stirred overnight. The aqueous layer is separated and combined with 42 ml. of concentrated hydrochloric acid. This mixture is extracted with 300 ml. of methylene chloride followed by two 100 ml. portions of methylene chloride. The combined extracts are then washed with 200 ml. followed by 100 ml. of 10% sodium bicarbonate. The extracts are dried over sodium sulfate and passed through Magnesol®. The filtrate is combined with hexanes and evaporated giving crystals of $\beta$-oxo-2-thiophenepropionitrile.

A 10.0 g. portion of $\beta$-oxo-2-thiophenepropionitrile in 100 ml. of chloroform is reacted with 10 ml. of N,N-dimethylacetamide dimethylacetal in an ice bath. The mixture is stirred in an ice bath for 2 hours and then at room temperature overnight. The mixture is evaporated to an oil which is dissolved in 50 ml. of methanol. A 15 ml. portion of 1N hydrochloric acid is added and the mixture is heated on a steam bath for 1/2 hour. Evaporation produces an oil which is combined with 75 ml. of benzene and 75 ml. of saturated aqueous sodium bicarbonate and mixed. The aqueous phase is saved. The organic phase is washed three times with saturated aqueous sodium bicarbonate. The aqueous phases are combined, washed twice with 100 ml. of benzene, and acidified with concentrated hydrochloric acid and the solid is collected. This solid is dried, dissolved in 50 ml. of hot *iso*propanol, treated with charcoal and cooled giving the desired product as a solid, m.p. 106°—108°C.

Example 102

*Cis*-2-(3-thenoyl)-3-hydroxycrotononitrile

A 50 ml. portion of quinoline is added to a mixture of 35 g. of cuprous cyanide and 50 g. of 3-bromothiophene. The mixture is warmed slowly with stirring until a solution forms and then heated at 190°C. for one hour. The mixture is poured into water, 200 ml. of concentrated ammonium hydroxide is added and the solid residue is extracted five times with diethyl ether. The combined ether extracts are dried, treated with charcoal, filtered and evaporated to a brown liquid. This liquid is distilled, collecting four fractions which are combined, dissolved in diethyl ether, washed twice with 4N hydrochloric acid, water and saturated sodium chloride solution, dried and evaporated giving 27.7 g. of 3-cyanothiophene.

A 113 g. portion of potassium *t*-butoxide is added to 800 ml. of diethyl ether. To this is added a mixture of 110.6 g. of 3-cyanothiophene and 54 ml. of acetonitrile in 350 ml. of ether. The reaction mixture is stirred in an ice-salt bath, more ether is added and the mixture is shaken vigorously. After standing for one hour at room temperature the mixture is poured into one liter of water. The layers are separated. The aqueous phase is extracted with diethyl ether. The ether phases are combined, washed with water and evaporated. The residue is dissolved in benzene, dried over magnesium sulfate and evaporated. To the residue is added 200 ml. of benzene and some petroleum ether. The mixture is cooled and the precipitate is collected and saved. The mother liquor is filtered through Magnesol®, evaporated and petroleum ether is added. Cooling produces a precipitate which is collected, dissolved in hot benzene, treated with charcoal and cooled giving a precipitate which is collected and combined with the first precipitate. The combined precipitates are recrystallized twice from hot benzene and charcoal giving 29.6 g. of $\beta$-amino-3-thiopheneacrylonitrile.

A 2.5 g. portion of the $\beta$-amino-3-thiopheneacrylonitrile is combined with 20 ml. of 1N hydrochloric acid and sufficient methanol to produce solution. The mixture is stirred at room temperature for 1/2 hour, evaporated to 1/2 volume, cooled and the precipitate is collected. This solid is recrystallized with charcoal treatment from 30 ml. of hot *iso*propanol giving 2.4 g. of $\beta$-oxo-3-thiophenepropionitrile.

A 1.5 g. portion of $\beta$-oxo-3-thiophenepropionitrile in 25 ml. of chloroform is reacted wth 1.5 ml. of N,N-dimethylacetamide dimethylacetal as described in Example 101, giving the desired product as a solid, m.p. 74°—76°C.

Example 103

*Cis*-2-(5-chloro-2-thenoyl)-3-hydroxycrotononitrile

A 50 g. portion of 5-chloro-2-thiophenecarboxaldehyde, 27.6 g. of hydroxylamine hydrochloride, 43.1 g. of sodium formate and 500 ml. of formic acid are reacted to produce 5-chloro-2-cyanothiophene.

A mixture of 32.4 g. of potassium *t*-butoxide in 600 ml. of toluene is cooled in an ice bath. A solution of 37.9 g. of 5-chloro-2-cyanothiophene and 21.0 ml. of acetonitrile diluted to 150 ml. with toluene is added dropwise, with rapid stirring over a one hour period. The mixture is stirred at room temperature overnight. One liter of water is added. The mixture is stirred for 1/2 hour. The aqueous layer is extracted with three 250 ml. portions of diethyl ether which are combined, dried over magnesium sulfate and concentrated almost to dryness. The residue is dissolved in a minimum of hot toluene, treated with charcoal and cooled. Petroleum ether is added, the mixture is chilled and the precipitate is collected as crystals of $\beta$-amino-5-chloro-2-thiopheneacrylonitrile.

A 9.2 g. portion of $\beta$-amino-5-chloro-2-thiopheneacrylonitrile is dissolved in 100 ml. of methanol. A 50 ml. portion of 1N hydrochloric acid is added and the mixture is stirred at room temperature for 3 hours. The precipitate is collected giving 5-chloro-$\beta$-oxo-2-thiophenepropionitrile.

A 9.8 g. portion of 5-chloro-$\beta$-oxo-2-thiophenepropionitrile in 75 ml. of chloroform is reacted with 9.0 ml. of N,N-dimethylacetamide dimethylacetal as described in Example 101 giving the desired product, m.p. 103°—105°C.

Example 104

$\beta$-Oxo-2-thiophenepropionitrile, thallium (I) salt

A solution of 10 g. of $\beta$-oxo-2-thiophene-propionitrile in 150 ml. of diethyl ether is stirred at 25°C. while 17.2 g. of neat thallium (I) ethoxide is added dropwise. The mixture is stirred as a colorless precipitate forms. After one hour, the mixture is filtered and the precipitate is washed with diethyl ether. Upon air-drying, 21.3 g. of colorless thallium (I) salt is obtained.

Similarly prepared are the thallium (I) salts of $\beta$-oxo-3-thiophenepropionitrile and 5-chloro-$\beta$-oxo-2-thiophenepropionitrile.

Example 105

*Cis*-2-(2-thenoyl)-3-hydroxycrotononitrile

A suspension of 5.5 g of $\beta$-oxo-2-thiophenepropionitrile, thallium (I) salt in 50 ml. of tetrahydrofuran is stirred at room temperature as acetyl fluoride is bubbled through. After 3 hours, the gas flow is stopped, the reaction vessel is stoppered and the mixture is allowed to stand overnight. After 24 hours, the mixture is filtered and the filtrate is evaporated to dryness. Recrystallization of the residue of chloroform/hexane provides the title compound.

Similarly prepared are *cis*-2-(3-thenoyl)-3-hydroxycrotononitrile and *cis*-2-(5-chloro-2-thenoyl)-3-hydroxycrotononitrile.

Example 106

*Cis*-2-(2-thenoyl)-3-hydroxycrotononitrile

A solution of 10 g. of $\beta$-oxo-2-thiophenepropionitrile, 10.7 g. of triethyl orthoacetate and 20 ml. of acetic anhydride is heated on a steam bath for 12 hours, then cooled and the excess acetic anhydride is evaporated under reduced pressure. The residue is treated with 50 ml. of ethanol and 50 ml. of 1N aqueous hydrochloric acid. After one hour, the solution is diluted with water and the product extracted with methylene chloride. The organic phase is washed with three portions of aqueous sodium bicarbonate which are combined, acidified, and extracted with methylene chloride. This organic phase is dried and evaporated. The crude residue is recrystallized from chloroform/hexane to provide the title compound.

Similarly, *cis*-2-(2-thenoyl)-3-hydroxy-2-pentenonitrile is prepared from $\beta$-oxo-2-thiophenepropionitrile and triethylorthopropionate.

Example 107

*Cis*-2-(3-thenoyl)-3-hydroxycrotononitrile

A neat sample of 20.0 g. of 3-thiophenecarboxylic acid was added dropwise to 50 ml. of thionyl chloride. The solution was stirred at room temperature overnight and the excess thionyl chloride was then evaporated under reduced pressure. The residue was vacuum distilled to provide 21.2 g of 3-thiophenecarboxylic acid chloride.

A mixture of 19.0 g. of cyanoacetone, sodium salt in 200 ml. of dry tetrahydrofuran is stirred while 10.0 g. of 3-thiophenecarboxylic acid chloride in 75 ml. of tetrahydrofuran is added dropwise. The reaction mixture is stirred overnight and then most of the solvent is evaporated under reduced pressure. The residue is acidified and extracted with chloroform. The organic phase is washed three times with aqueous sodium bicarbonate and the combined basic washes are acidified. The resulting mixture is extracted with methylene chloride. Evaporation of the organic layer and recrystallization of the residue provides the title compound.

Example 108

*Cis*-2-(3-thenoyl)-3-hydroxycrotononitrile

An 8.0 g. portion of di*iso*propylamine in 100 ml. of diethyl ether is cooled to 0°C. under argon. A solution of 31.5 ml. of 2.5M *n*-butyllithium in hexane is added dropwise and the reaction is further cooled to —10°C. A 5.94 g. portion of 5-methylisoxazole is then added dropwise and the mixture is stirred for 1/2 hour. A 10.5 g. portion of 3-thiophenecarboxylic acid chloride is added while the reaction is held at 0°C. The mixture is allowed to warm to room temperature and stand for 18 hours. The reaction is quenched with water, acidified with dilute hydrochloric acid and extracted with chloroform. The chloroform phase is extracted three times with aqueous sodium bicarbonate and the combined aqueous extracts are acidified and washed three times with chloroform. The organic extract is dried. filtered and evaporated. The residue is recrystallized from ethanol-hexane giving the desired compound.

Example 109

*Cis*-2-(5-methyl-3-thenoyl)-3-hydroxy-2-hexenonitrile

The general procedure of Example 106 is repeated but replacing the β-oxo-2-thiophenepropionitrile and triethyl orthoacetate employed in that Example with equivalent amounts of 5-methyl-β-oxo-3-thiophenepropionitrile and triethyl orthobutyrate whereby there is obtained the title compound in equally good yield.

Example 110

5-Methyl-2-thiophene carbonyl chloride

Heating 100 g. of 5-methyl-2-thiophenecarboxylic acid in 130 ml. of thionyl chloride at 100°C. for 1—1/2 hours followed by distillation gave 112.26 g. (99.3%) of 5-methyl-2-thiophene carbonyl chloride, which boiled at 108°—110°C/13 mm.

Example 111

*Cis*-2-(5-methyl-2-thenoyl)-3-hydroxycrotononitrile

A mixture of 0.05 mole (8.03 g.) of 5-methyl-2-thiophenecarbonyl chloride and 0.15 mole (15.76 g.) of cyanoacetone sodium enolate in 100 ml. of tetrahydrofuran was stirred for 3 hours. After removal of the tetrahydrofuran *in vacuo*, the residue was slurried with water, acidified with hydrochloric acid, and extracted into methylene chloride. The methylene chloride extracts were, in turn, extracted with two 50 ml. portions of saturated sodium bicarbonate. The combined bicarbonate extracts were treated with Darco® and acidified to give, after recrystallization from *iso*propanol, 5.19 g. (50%) of the desired product as a light yellow crystalline solid melting at 71°—73°C.

Example 112

4-(*p*-Chlorophenyl)-2-(1-hydroxyethylidene)acetoacetonitrile

A 54 g. portion of sodium methoxide in 250 ml. of methanol is cooled and stirred in an ice bath while 82 ml. (83.5 g.) of 5-methyl isoxazole is added dropwise. After addition is complete the reaction is cooled in ice and the solid is filtered, washed with ether and dried giving 57.18 g. of cyanoacetone sodium enolate.

A 100 ml. portion of thionyl chloride is added to 51 g. of *p*-chlorophenylacetic acid and the mixture is refluxed on a steam bath for 3 hours. The thionyl chloride is removed on a rotary evaporator and the resulting oil is distilled under a water aspirator at 12 mm. giving 51.41 g. of *p*-chlorophenylacetyl chloride as a pink liquid.

A 9.45 g. portion of *p*-chlorophenylacetyl chloride followed by 50 ml. of tetrahydrofuran is added to a mixture of 15.76 g. of cyanoacetone sodium enolate in 50 ml. of tetrahydrofuran. The mixture is stirred overnight, then the solvent is removed on a rotary evaporator. The solid is taken up in 100 ml. of water, acidified with concentrated hydrochloric acid and extracted with three 100 ml. portions of methylene chloride. The extracts are combined and extracted with three 100 ml. portions of saturated aqueous sodium bicarbonate. These extracts are combined and acidified with 12N hydrochloric acid, giving an oil. This mixture is extracted with methylene chloride. The extract is dried over magnesium sulfate, filtered through diatomaceous earth and reduced in volume on a steam bath giving the desired product as an oil which crystallizes on standing and after recrystallization from hexanes, then aqueous ethanol has a m.p. 50—53°C.

Following the general procedure of Example 112, other representative compounds of this invention, such as those found in Table VII, may be prepared.

TABLE VII

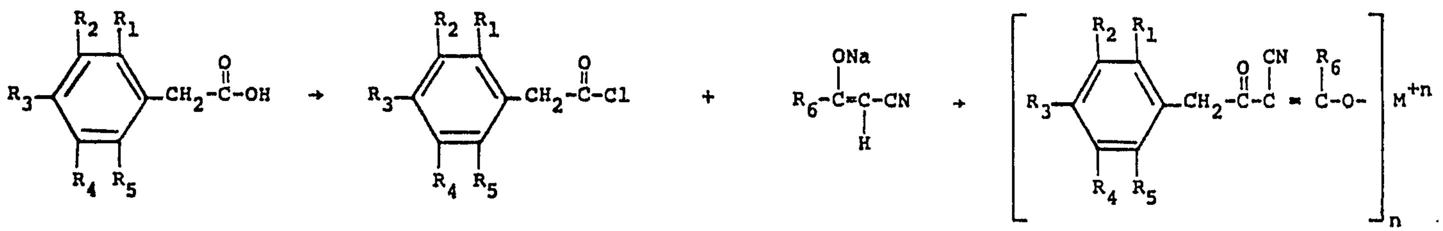

(1) (2) (3) (4)

| Example | Phenyl Acetic Acid | Phenyl Acetyl Chloride | Cyanoacetone Sodium Enolate | Phenyl Hydroxyethylidene Acetoacetonitrile | m.p. 0°C. |
|---|---|---|---|---|---|
| 113 | $R_1$, $R_3$, $R_4$, $R_5$ = H<br>$R_2$ = Cl | $R_1$, $R_3$, $R_4$, $R_5$ = H<br>$R_2$ = Cl | $R_6$ = $CH_3$ | $R_1$, $R_3$, $R_4$, $R_5$, M = H<br>$R_6$ = $CH_3$, $R_2$ = Cl | 60–62 |
| 114 | $R_1$, $R_2$, $R_4$, $R_5$ = H<br>$R_3$ = F | $R_1$, $R_2$, $R_4$, $R_5$ = H<br>$R_3$ = F | $R_6$ = $CH_3$ | $R_1$, $R_2$, $R_4$, $R_5$, M = H<br>$R_6$ = $CH_3$<br>$R_3$ = F | 57–59 |
| 115 | $R_2$, $R_4$, $R_5$ = H<br>$R_1$, $R_3$ = Cl | $R_2$, $R_4$, $R_5$ = H<br>$R_1$, $R_3$ = Cl | $R_6$ = $CH_3$ | $R_2$, $R_4$, $R_5$, M = H<br>$R_6$ = $CH_3$<br>$R_1$, $R_3$ = Cl | 113–117* |

* Final product recrystallized from isopropanol.

TABLE VII (Continued)

| Example | Phenyl Acetic Acid | Phenyl Acetyl Chloride | Cyanoacetone Sodium Enolate | Phenyl Hydroxyethylidene Acetoacetonitrile | m.p. 0°C. |
|---|---|---|---|---|---|
| 116 | $R_1$, $R_4$, $R_5$ = H | $R_1$, $R_4$, $R_5$ = H | $R_6$ = $CH_3$ | $R_1$, $R_4$, $R_5$, M = H | 85–87* |
| | $R_2$, $R_3$ = Cl | $R_2$, $R_3$ = Cl | | $R_6$ = $CH_3$ | |
| | | | | $R_2$, $R_3$ = Cl | |
| 117 | $R_2$, $R_3$, $R_4$, $R_5$ = H | $R_2$, $R_3$, $R_4$, $R_5$ = H | $R_6$ = $CH_3$ | $R_2$, $R_3$, $R_4$, $R_5$, M = H | 61–64* |
| | $R_1$ = F | $R_1$ = F | | $R_6$ = $CH_3$ | |
| | | | | $R_1$ = F | |
| 118 | $R_1$, $R_3$, $R_4$, $R_5$ = H | $R_1$, $R_3$, $R_4$, $R_5$ = H | $R_6$ = $CH_3$ | $R_1$, $R_3$, $R_4$, $R_5$, M = H | 43–45 |
| | $R_2$ = F | $R_2$ = F | | $R_6$ = $CH_3$, $R_2$ = F | |
| 119 | $R_2$, $R_3$, $R_4$ = H | $R_2$, $R_3$, $R_4$ = H | $R_6$ = $CH_3$ | $R_2$, $R_3$, $R_4$, M = H | 123–124* |
| | $R_1$, $R_5$ = Cl | $R_1$, $R_5$ = Cl | | $R_6$ = $CH_3$ | |
| | | | | $R_1$, $R_5$ = Cl | |

* Final product recrystallized from isopropanol.

Example 120

2-Cyano-3-hydroxythiocrotonic acid-S-phenyl ester

A 72 ml. portion of thionyl chloride is added dropwise to a mixture of 70.24 g. of 5-methylisoxazole-4-carboxylic acid [H. Yasuda, Yakugaku Zasshi, *79*, 836—838 (1959); C.A. *53*, 12885d] and 64.44 g. of sodium carbonate in 250 ml. of chloroform. The mixture is heated gently on a steam bath for 4 hours, then the solid is filtered and the filtrate is evaporated to an oil. This oil is distilled at 4.5 mm. and the material boiling at 68—70°C. is collected, giving 65.23 g. of 5-methylisoxazole-4-carbonyl chloride.

A mixture of 10.27 ml. (11.02 g.) of thiophenol and 11.15 ml. (14.6 g.) of 5-methylisoxazole-4-carbonyl chloride in 50 ml. of ether is cooled in an ice bath. A 14 ml. portion of triethylamine is diluted to 50 ml. with ether and added dropwise. The mixture is stirred for 2 hours and the solid is filtered and washed with ether. The filtrate is evaporated to an oil which crystallizes, is taken up in methylene chloride and filtered through diatomaceous earth. The filtrate is concentrated on a steam bath with the addition of hexanes, giving an oil. Cooling crystallizes this oil giving 16.65 g. of 5 methylthio-4-isoxazolecarboxylic acid-S-phenyl ester as a white crystalline solid.

An 8 ml. portion of triethylamine in 50 ml. of ether is cooled in an ice bath and a mixture of 11.91 g. of 5-methylthio-4-isoxazolecarboxylic acid-S-phenyl ester in 60 ml. of ether is added dropwise. The reaction is stirred overnight and the solid is filtered, giving 15.15 g. of 2-cyano-3-hydroxythiocrotonic acid-S-phenyl ester with triethylamine as a white crystalline solid. A 10.17 g. portion of this triethylamine salt is dissolved in 100 ml. of water and 2.64 ml. of concentrated hydrochloric acid is added, giving an oil which solidifies. The solid is filtered and dried, giving the desired product, m.p. 106—108°C.

Example 121

2-Cyano-3-hydroxythiocrotonic acid-S-(*p*-chlorophenyl)ester

A 14.46 g. portion of *p*-chlorothiophenol and 11.15 ml. (14.6 g.) of 5-methylisoxazole-4-carbonyl chloride in 50 ml. of ether is cooled in an ice bath and a solution of 14 ml. of triethylamine in 50 ml. of ether is added dropwise. The mixture is stirred for 3 hours and then diluted with ether. The solid is filtered and washed with ether. The combined filtrate and washing is evaporated to a solid which is taken up in methylene chloride, filtered and evaporated on a steam bath with the addition of hexanes giving an oil which crystallizes on cooling, giving 5-methylthio-4-isoxazolecarboxylic acid-S-(*p*-chlorophenyl)ester as a white crystalline solid.

A 17.60 g. portion of the above isoxazole is slurried with 100 ml. of ether and cooled in an ice bath. A solution of 15 ml. of triethylamine in 50 ml. of ether is added dropwise and the reaction is stirred overnight. The solid is filtered and dried, giving 23.61 g. of 2-cyano-3-hydroxythiocrotonic acid-S-(*p*-chlorophenyl)ester triethylamine salt as a white crystalline solid.

A 9.17 g. portion of the above salt is taken up in 50 ml. of water and filtered. A 2.4 ml. portion of concentrated hydrochloric acid is added to the filtrate and the reaction is stirred for 2 hours. The solid is filtered and dried giving 6.10 g. of the desired product as a white crystalline solid, m.p. 91—94°C.

Following the general procedure of Examples 120 and 121, other representative compounds of this invention, such as those found in Table VIII, may be made.

TABLE VIII

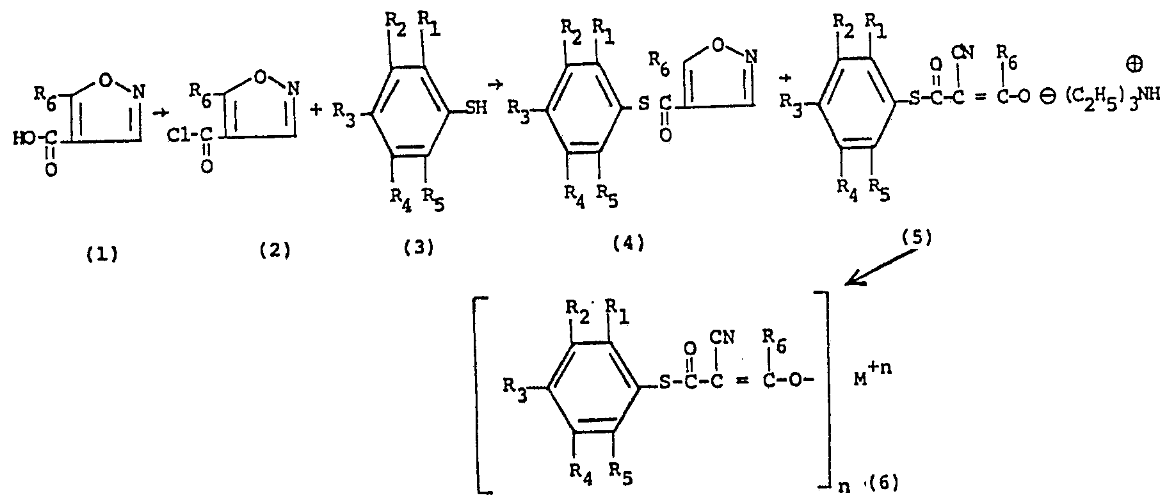

(1) (2) (3) (4) (5) (6)

| Example | Alkyl Isoxazole Acid (1) | Alkyl Isoxazole Chloride (2) | Thiophenol (3) | Alkylthio Isoxazole Phenyl Ester (4) | 2-Cyano-3-hydroxy-crotonic Acid Phenyl ester Triethylamine Salt (5) | 2-Cyano-3-hydroxy-crotonic Acid Phenyl Ester (6) | m.p. °C. | Method of |
|---|---|---|---|---|---|---|---|---|
| 122 | $R_6 = CH_3$ | $R_6 = CH_3$ | $R_1$, $R_2$,<br>$R_4$, $R_5 = H$<br>$R_3 = CH_3$ | $R_1$, $R_2$,<br>$R_4$, $R_5 = H$<br>$R_3 = CH_3$<br>$R_6 = CH_3$ | $R_1$, $R_2$,<br>$R_4$, $r_5 = H$<br>$R_3 = CH_3$<br>$R_6 = CH_3$ | $R_1$, $R_2$,<br>$R_4$, $R_5$, M = H<br>$R_3 = CH_3$<br>$R_6 = CH_3$ | 74–80 | Example 120 |
| 123 | $R_6 = CH_3$ | $R_6 = CH_3$ | $R_1$, $R_2$<br>$R_4$, $R_5 = H$<br>$R_3 = F$ | $R_1$, $R_2$<br>$R_4$, $R_5 = H$<br>$R_3 = F$<br>$R_6 = CH_3$ | $R_1$, $R_2$<br>$R_4$, $R_5 = H$<br>$R_3 = F$<br>$R_6 = CH_3$ | $R_1$, $R_2$<br>$R_4$, $R_5$, M = H<br>$R_3 = F$<br>$R_6 = CH_3$ | 105–110 | Example 121 |

TABLE VIII (Continued)

| Example | Alkyl Isoxazole Acid (1) | Alkyl Isoxazole Chloride (2) | Thiophenol (3) | Alkylthio Isoxazole Phenyl Ester (4) | 2-Cyano-3-hydroxy-crotonic Acid Phenyl Ester Triethylamine Salt (5) | 2-Cyano-3-hydroxy-crotonic Acid Phenyl Ester (6) | m.p. °C. | Method of |
|---|---|---|---|---|---|---|---|---|
| 124 | $R_6 = CH_3$ | $R_6 = CH_3$ | $R_1 - R_3$,<br>$R_5 = H$<br>$R_4 = CH_3$ | $R_1$–$R_3$,<br>$R_5 = H$<br>$R_4$, $R_6 = CH_3$ | $R_1$–$R_3$,<br>$R_5 = H$<br>$R_4$, $R_6 = CH_3$ | $R_1$–$R_3$,<br>$R_5$, $M = H$<br>$R_4$, $R_6 = CH_3$ | 57–61 | Example 120 |
| 125 | $R_6 = CH_3$ | $R_6 = CH_3$ | $R_1 - R_3$,<br>$R_5 = H$<br>$R_4 = Cl$ | $R_1$–$R_3$,<br>$R_5 = H$<br>$R_4 = Cl$<br>$R_6 = CH_3$ | $R_1 - R_3$,<br>$R_5 = H$<br>$R_4 = Cl$<br>$R_6 = CH_3$ | $R_1 - R_3$,<br>$R_5$, $M = H$<br>$R_4 = Cl$<br>$R_6 = CH_3$ | 93–94 | Example 121 |
| 126 | $R_6 = CH_3$ | $R_6 = CH_3$ | $R_2 - R_5 = H$<br>$R_1 = Cl$ | $R_2 - R_5 = H$<br>$R_1 = Cl$<br>$R_6 = CH_3$ | $R_2 - R_5 = H$<br>$R_1 = Cl$<br>$R_6 = CH_3$ | $R_2 - R_5$, $M = H$<br>$R_1 = Cl$<br>$R_6 = CH_3$ | 73–74 | Example 121 |
| 127 | $R_6 = CH_3$ | $R_6 = CH_3$ | $R_2 - R_5 = H$<br>$R_1 = CH_3O$ | $R_2 - R_5 = H$<br>$R_1 = CH_3O$<br>$R_6 = CH_3$ | $R_2 - R_5 = H$<br>$R_1 = CH_3O$<br>$R_6 = CH_3$ | $R_2 - R_5$, $M = H$<br>$R_3 = CH_3O$<br>$R_6 = CH_3$ | 131–134 | Example 121 |
| 128 | $R_6 = CH_3$ | $R_6 = CH_3$ | $R_1 - R_3$,<br>$R_5 = H$ | $R_1 - R_3$<br>$R_5 = H$<br>$R_4 = CH_3O$<br>$R_6 = CH_3$ | $R_1 - R_3$<br>$R_5 = H$<br>$R_4 = CH_3O$<br>$R_6 = CH_3$ | $R_1 - R_3$<br>$R_5$, $M = H$<br>$R_4 = CH_3O$<br>$R_6 = CH_3$ | 61–67 | Example 121 |

TABLE VIII (Continued)

| Example | Alkyl Isoxazole Acid (1) | Alkyl Isoxazole Chloride (2) | Thiophenol (3) | Alkylthio Isoxazole Phenyl Ester (4) | 2-Cyano-3-hydroxy-crotonic Acid Phenyl Ester Triethylamine Salt (5) | 2-Cyano-3-hydroxy-crotonic Acid Phenyl Ester (6) | m.p. °C. | Method of |
|---|---|---|---|---|---|---|---|---|
| 129 | $R_6 = CH_3$ | $R_6 = CH_3$ | $R_2 - R_5 = H$<br>$R_1$,<br>$R_6 = CH_3$ | $R_2 - R_5 = H$<br>$R_1$, $R_6 = CH_3$ | $R_2 - R_5 = H$<br>$R_1$, $R_6 = CH_3$ | $R_2 - R_5$, $M = H$<br>$R_1$, $R_6 = CH_3$ | 51–54 | Example<br>120 |
| 130 | $R_6 = CH_3$ | $R_6 = CH_3$ | $R_1$, $R_2$,<br>$R_4$, $R_5 = H$<br>$R_3 = CF_3$<br>$R_6 = CH_3$ | $R_1$, $R_2$,<br>$R_4$, $R_5 = H$<br>$R_3 = CF_3$<br>$R_6 = CH_3$ | $R_1$, $R_2$,<br>$R_4$, $R_5 = H$<br>$R_3 = CF_3$<br>$R_3 = CH_3$ | $R_1$, $R_2$,<br>$R_4$, $R_5$, $M = H$<br>$R_3 = CF_3$<br>$R_3 = CH_3$ | | Example<br>120 |
| 131 | $R_6 = CH_3$ | $R_6 = CH_3$ | $R_1 - R_3$,<br>$R_5 = H$<br>$R_4 = CF_3$<br>$R_6 = CH_3$ | $R_1 - R_3$,<br>$R_5 = H$<br>$R_4 = CF_3$<br>$R_6 = CH_3$ | $R_1 - R_3$,<br>$R_5 = H$<br>$R_4 = CF_3$<br>$R_6 = CH_3$ | $R_1 - R_3$,<br>$R_5$, $M = H$<br>$R_4 = CF_3$<br>$R_6 = CH_3$ | | Example<br>120 |
| 132 | $R_6 = CH_3$ | $R_6 = CH_3$ | $R_1 - R_3$,<br>$R_5 = H$<br>$R_4Br$<br>$R_4 = CH_3$ | $R_1 - R_3$,<br>$R_5 = H$<br>$R_4Br$<br>$R_6 = CH_3$ | $R_1 - R_3$,<br>$R_5 = H$<br>$R_4Br$<br>$R_6 = CH_3$ | $R_1 - R_3$,<br>$R_5$, $M = H$<br>$R_4Br$<br>$R_6 = CH_3$ | | Example<br>120 |

## Example 133

### 2-Cyano-3-hydroxycrotonic acid, *p*-chlorophenyl ester

A 130 ml. portion of thionyl chloride is added slowly to a mixture of 63.67 g. of 5-methylisoxazole-4-carboxylic acid [H. Yasuda, Yakugaku Zasshi, *79*, 836—838 (1959); C.A., *53*, 21885d] and 58.4 g. of sodium carbonate in 250 ml. of chloroform. The mixture is heated on a steam bath for 2 hours, filtered and the solvents are removed from the filtrate by evaporation. Two portions of chloroform are successively added to the residue and removed by evaporation. The resulting oil is distilled at 1—2 mm and the material boiling at 66—68°C. is collected, giving 64.48 g. of 5-methylisoxazole carbonyl chloride.

A mixture of 12.86 g. of *p*-chlorophenol and 11.5 ml. (14.6 g.) of 5-methylisoxazole carbonyl chloride in 100 ml. of ether is cooled in an ice bath. A 14 ml. portion of triethylamine in 50 ml. of ether is added dropwise. The mixture is stirred for one hour and then filtered. The filtrate is evaporated to an oil which is distilled. The fraction boiling at 132—137°C. is collected, giving 14.85 g. of 5-methyl-4-isoxazolecarboxylic acid, *p*-chlorophenyl ester.

An 8 ml. portion of triethylamine in 50 ml. of ether is cooled in an ice bath. A 10.15 g. portion of 5-methyl-4-isoxazolecarboxylic acid, *p*-chlorophenyl ester in 50 ml. of ether is added dropwise and then stirred overnight. The resulting solid is filtered and washed with ether, giving 12.41 g. of 2-cyano-3-hydroxycrotonic acid, *p*-chlorophenyl ester triethylamine salt as a white crystalline solid.

A 9.03 g. portion of the above salt is taken up in water, filtered and cooled. A 2.3 ml. portion of concentrated hydrochloric acid is added giving 6.33 g. of the desired product as crystals, mp. 97—100°C.

## Example 134

### 2-Cyano-3-hydroxycrotonic acid, 2,3-dichlorophenyl ester

A 16.3 g. portion of 2,3-dichlorophenol and 11.15 ml. (14.6 g.) of 5-methylisoxazole carbonyl chloride in 50 ml. of ether is cooled in an ice bath and a solution of 14 ml. of triethylamine is added dropwise. The mixture is stirred for 3 hours and the solid is filtered and washed with ether. The combined filtrate and wash is evaporated to an oil on a steam bath. This oil is taken up in methylene chloride, filtered through diatomaceous earth and the filtrate evaporated on a steam bath with the addition of hexanes to about 50 ml. Cooling gives 24.02 g. of 5-methyl-4-isoxazolecarboxylic acid, 2,3-dichlorophenyl ester as a tan crystalline solid.

A 17.95 g. portion of the above isoxazole is slurried in 100 ml. of ether and cooled in an ice bath. A solution of 12 ml. of triethylamine in 50 ml. of ether is added dropwise and the reaction is stirred overnight. The solid is recovered by filtration and dried, giving 22.92 g. of 2-cyano-3-hydroxycrotonic acid, 2,3-dichlorophenyl ester, triethylamine salt as a light tan crystalline solid.

An 11.88 g. portion of the above salt is slurried in 100 ml. of water and acidified with 2.65 ml. of concentrated hydrochloric acid. The mixture is stirred for several hours and the solid is filtered and dried, giving 8.52 g. of the desired product, mp. 142—146°C.

Following the general procedure of Examples 133 and 134 other representative compounds of this invention such as those found in Table IX, may be made.

TABLE IX

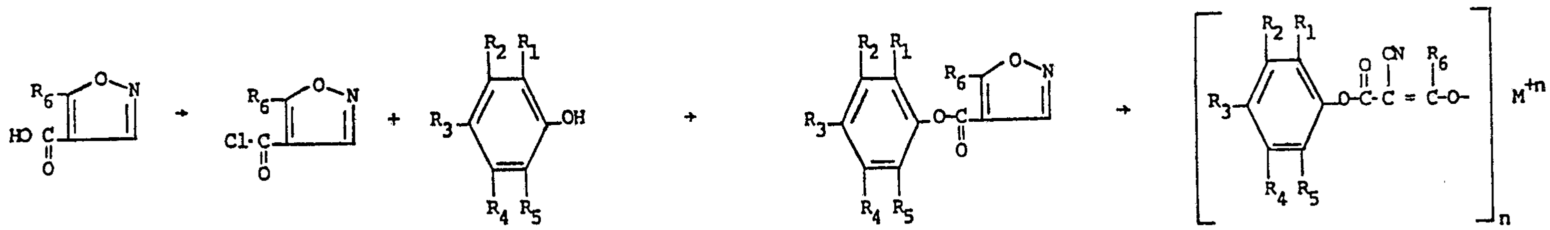

(1) (2) (3) (4) (5 & 6)

| Example | Alkylisoxazole Acid (1) $R_6$ = | Alkylisoxazole Chloride (2) $R_6$ = | Phenol (2) | Alkylisoxazole carboxylic Phenyl Ester (4) | 2-Cyano Alkenoic Acid Phenyl Ester Triethylamine Salt (5) | 2-Cyano Alkenoic Acid Phenyl Ester (6) | m.p. °C. | Method of |
|---|---|---|---|---|---|---|---|---|
| 135 | $CH_3$ | $CH_3$ | $R_1 - R_4 = H$<br>$R_5 = Cl$ | $R_1 - R_4 = H$<br>$R_5 = Cl$ | M = H<br>$R_1 - R_4 = H$<br>$R_5 = Cl$ | M = H<br>$R_1 - R_4 = H$<br>$R_5 = Cl$ | 88–92 | Example 133 |
| 136 | $CH_3$ | $CH_3$ | $R_1$, $R_2$,<br>$R_4$, $R_5$ =<br>H<br>$R_3 = CH_3O$ | $R_1$, $R_2$,<br>$R_4$, $R_5$ =<br>H<br>$R_3 = CH_3O$ | M = H<br>$R_1$, $R_2$, $R_4$,<br>$R_5 = H$<br>$R_3 = CH_3O$ | — | 112–115 | Example 134 |
| 137 | $CH_3$ | $CH_3$ | $R_1$, $R_2$,<br>$R_4$, $R_5 = H$<br>$R_3 = CH_3$ | $R_1$, $R_2$,<br>$R_4$, $R_5 = H$<br>$R_3 = CH_3$ | M = H<br>$R_1$, $R_2$, $R_4$,<br>$R_5 = H$, $R_3 = CH_3$ | — | 127–130 | Example 133 |

TABLE IX (Continued)

| Example | Alkylisoxazole Acid (1) $R_6$ = | Alkylisoxazole Chloride (2) $R_6$ = | Phenol (3) | Alkylisoxazole carboxylic Phenyl Ester (4) | 2-Cyano Alkenoic Acid Phenyl Ester Triethylamine Salt (5) | 2-Cyano Alkenoic Acid Phenyl Ester (6) | m.p. °C. | Method of |
|---|---|---|---|---|---|---|---|---|
| 138 | $CH_3$ | $CH_3$ | $R_1 = CH_3O$<br>$R_2 - R_5 = H$ | $R_1 = CH_3O$<br>$R_2 - R_5 = H$ | M = H<br>$R_1 = CH_3O$<br>$R_2 - R_5 = H$ | — | 86–88 | Example 134 |
| 139 | $CH_3$ | $CH_3$ | $R_1 - R_4 = H$<br>$R_5 = F$ | $R_1 - R_4 = H$<br>$R_5 = F$ | M = H<br>$R_1 - R_4 = H$<br>$R_5 = F$ | M = H<br>$R_1 - R_4 = H$<br>$R_5 = F$ | 94–97 | Example 133 |
| 140 | $CH_3$ | $CH_3$ | $R_2, R_3,$<br>$R_4, R_5 = H$<br>$R_1 = CH_3O$ | $R_2, R_3,$<br>$R_4, R_5 = H$<br>$R_1 = CH_3O$ | $R_2, R_3, R_4,$<br>$R_5$, M = H<br>$R_1 = CH_3O$ | $R_2, R_3, R_4,$<br>$R_5$, M = H<br>$R_1 = CH_3O$ | 101–105 | Example 134 |
| 141 | $CH_3$ | $CH_3$ | $R_1, R_2,$<br>$R_5 = H$<br>$R_3, R_4 = Cl$ | $R_1, R_2,$<br>$R_5 = H$<br>$R_3, R_4 = Cl$ | $R_1, R_2, R_5,$<br>M = H<br>$R_3, R_4 = Cl$ | $R_1, R_2, R_5,$<br>M = H<br>$R_3, R_4 = Cl$ | 115–121 | Example 134 |
| 142 | $CH_3$ | $CH_3$ | $R_1, R_2,$<br>$R_4, R_5 = H$<br>$R_3 = F$ | $R_1, R_2,$<br>$R_4, R_5 = H$<br>$R_3 = F$ | $R_1, R_2, R_4,$<br>$R_5$, M = H<br>$R_3 = F$ | $R_1, R_2, R_4,$<br>$R_5$, M = H<br>$R_3 = F$ | 93–95 | Example 133 |

TABLE IX (Continued)

| Example | Alkylisoxazole Acid (1) $R_6$ = | Alkylisoxazole Chloride (2) $R_6$ = | Phenol (3) | Alkylisoxazole carboxylic Phenyl Ester (4) | 2-Cyano Alkenoic Acid Phenyl Ester Triethylamine Salt (5) | 2-Cyano Alkenoic Acid Phenyl Ester (6) | m.p. °C. | Method of |
|---|---|---|---|---|---|---|---|---|
| 143 | $CH_3$ | $CH_3$ | $R_1$, $R_4$, $R_5$ = H<br>$R_2$, $R_3$ = $CH_3O$ | $R_1$, $R_4$, $R_5$ = H<br>$R_1$, $R_3$ = $CH_3O$ | $R_1$, $R_4$, $R_5$, M = H<br>$R_2$, $R_3$ = $CH_3O$ | $R_1$, $R_4$, $R_5$, M = H<br>$R_2$, $R_3$ = $CH_3O$ | 112–115 | Example 134 |
| 144 | $CH_3$ | $CH_3$ | $R_1$, $R_2$, $R_4$, $R_5$ = H<br>$R_3$ = Br | $R_1$, $R_2$, $R_4$, $R_5$ = H<br>$R_3$ = Br | $R_1$, $R_2$, $R_4$, $R_5$, M = H<br>$R_3$ = Br | $R_1$, $R_2$, $R_4$, $R_5$, M = H<br>$R_3$ = Br | 115–120 | Example 133 |
| 145 | $CH_3$ | $CH_3$ | $R_2$, $R_3$, $R_4$, $R_5$ = H<br>$R_1$ = Br | $R_2$, $R_3$, $R_4$, $R_5$ = H<br>$R_1$ = Br | $R_2$, $R_3$, $R_4$, $R_5$, M = H<br>$R_1$ = Br | $R_2$, $R_3$, $R_4$, $R_5$, M = H<br>$R_1$ = Br | 93–97 | Example 133 |
| 146 | $CH_3$ | $CH_3$ | $R_1$, $R_3$, $R_4$, $R_5$ = H<br>$R_2$ = Br | $R_1$, $R_3$, $R_4$, $R_5$ = H<br>$R_2$ = Br | $R_1$, $R_3$, $R_4$, $R_5$, M = H<br>$R_2$ = Br | $R_1$, $R_3$, $R_4$, $R_5$, M = H<br>$R_2$ = Br | 104–108 | Example 133 |
| 147 | $CH_3$ | $CH_3$ | $R_1$, $R_2$, $R_3$, $R_4$, $R_5$ = H | $R_1$, $R_2$, $R_3$, $R_4$, $R_5$ = H | $R_1$, $R_2$, $R_3$, $R_4$, $R_5$, M = H | $R_1$, $R_2$, $R_3$, $R_4$, $R_5$, M = H | 74–78 | Example 133 |

**Claims for Contracting States: BE, CH, DE, FR, GB, IT, LU, NL, SE**

1. 2-Carbonyl-3-hydroxy-2-alkenonitriles of the formula:

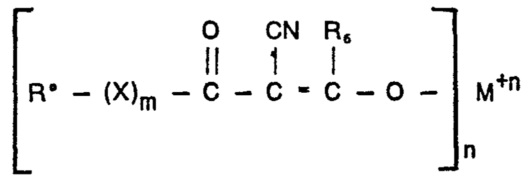

...I

wherein
n is an integer from 1 to 3 inclusive;
M is hydrogen or a pharmaceutically acceptable cation;
$R_6$ is an alkyl group of 1 to 4 carbons;
R° is one of the following aromatic groups:
(1) the group

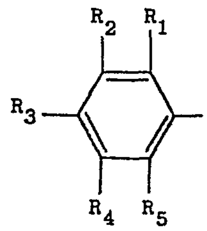

in which $R_1$, $R_2$, $R_3$, $R_4$ and $R_5$ are the same or different and are hydrogen, halogen, alkyl having up to 4 carbons, alkoxy having up to 4 carbons, trifluoromethyl or trichloromethyl, with the proviso that when m is the number 0 at least two of $R_1$, $R_2$, $R_3$, $R_4$ and $R_5$ must be hydrogen but when $R_6$ is methyl and m is the number 0 not all of $R_1$, $R_2$, $R_3$, $R_4$ and $R_5$ may be hydrogen; or
(2) the thienyl moiety

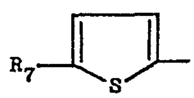 or 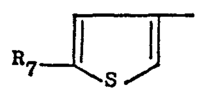

wherein $R_7$ is hydrogen, an alkyl group of up to 3 carbons, fluorine, chlorine or bromine;
X is $CH_2$, O or S and m is a number 0 or 1, provided that when R° is a thienyl moiety then m is 0; and the tautometers of the above compounds.

2. A compound according to claim 1, wherein R° is the group:

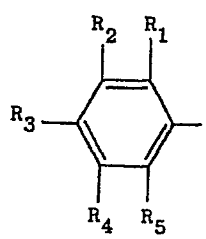

wherein $R_1$, $R_2$, $R_3$, $R_4$ and $R_5$ are as defined; and m is the number 0.

3. A compound according to claim 2, wherein one of the radicals $R_1$, $R_2$, $R_3$, $R_4$ and $R_5$ is bromo, chloro, fluoro, trifluoromethyl, methyl or methoxy and the remaining of said radicals are hydrogen; or $R_1$ is methyl, $R_3$ is chloro and $R_2$, $R_4$ and $R_5$ are each hydrogen.

4. A compound according to claim 1, wherein R° is a thienyl moiety and m is the number 0.

5. A compound according to claim 4, wherein $R_7$ is hydrogen, chloro, fluoro, methyl or isopropyl.

6. A compound according to claim 1, wherein R° is the group

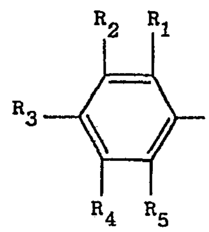

wherein $R_1$, $R_2$, $R_3$, $R_4$ and $R_5$ are as defined; X is $CH_2$ and m is the number 1.

7. A compound according to claim 6, wherein one or two of the radicals $R_1$, $R_2$, $R_3$, $R_4$ and $R_5$ is chloro and the remaining of said radicals are hydrogen; or one of said radicals is fluoro and the remaining of said radicals are hydrogen.

8. A compound according to claim 1, wherein R° is the group:

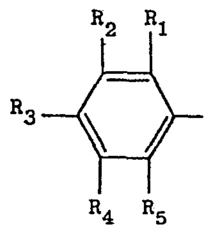

wherein $R_1$, $R_2$, $R_3$, $R_4$ and $R_5$ are as defined; X is S and m is the number 1.

9. A compound according to claim 8, wherein one of the radicals $R_1$, $R_2$, $R_3$; $R_4$ and $R_5$ is bromo, chloro, fluoro, trifluoromethyl, methyl or methoxy and the remaining of said radicals are hydrogen; or all of said radicals are hydrogen.

10. A compound according to claim 1, wherein R° is the group:

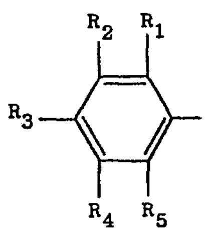

wherein $R_1$, $R_2$, $R_3$, $R_4$ and $R_5$ are as defined; X is O and m is the number 1.

11. A compound according to claim 10, wherein one of the radicals $R_1$, $R_2$, $R_3$, $R_4$ and $R_5$ is bromo, chloro, fluoro, methyl or methoxy and the remaining of said radicals are hydrogen; or two of said radicals are chloro, the remaining being hydrogen; or two of said radicals are methoxy, the remaining being hydrogen; or all of said radicals are hydrogen.

12. A compound according to any one of the preceding claims, wherein $R_6$ is methyl.

13. A compound according to any one of the preceding claims, wherein M is hydrogen, sodium or triethylammonium and n is 1, or M is cupric and n is 2..

14. An anti-arthritic composition for use in inhibiting the progression of arthritis, meliorating inflammation and/or inhibiting progressive joint deterioration in mammals, comprising a pharmaceutically acceptable carrier or diluent and a 2-carbonyl-3-hydroxy-2-alkenonitrile of the formula:

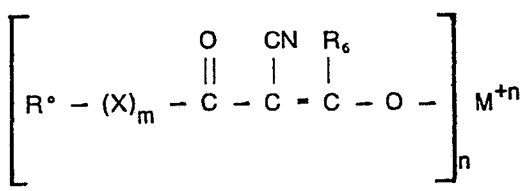

...II

wherein

n is an integer from 1 to 3 inclusive;

M is hydrogen or a pharmaceutically acceptable cation;

$R_6$ is an alkyl group of 1 to 4 carbons;

R° is one of the following aromatic groups:

(1) the group

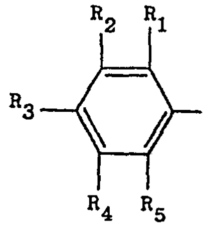

in which $R_1$, $R_2$, $R_3$, $R_4$ and $R_5$ are the same or different and are hydrogen, halogen, alkyl having up to 4 carbons, alkoxy having up to 4 carbons, trifluoromethyl or trichloromethyl, with the proviso that when m is the number 0 at least two of $R_1$, $R_2$, $R_3$, $R_4$ and $R_5$ must be hydrogen; or

(2) the thienyl moiety

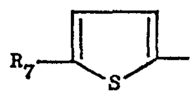

or

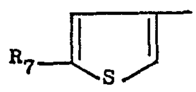

wherein $R_7$ is hydrogen, an alkyl group of up to 3 carbons, fluorine, chlorine or bromine;

X is $CH_2$, O or S and m is the number 0 or 1, provided that when R° is a thienyl moiety then m is 0;

and the tautomers of the above compounds.

15. A composition according to claim 14, wherein said 2-carbonyl-3-hydroxy-2-alkenonitrile is as defined in any one of claims 2 to 13.

16. A composition according to claim 14, wherein R° is the group:

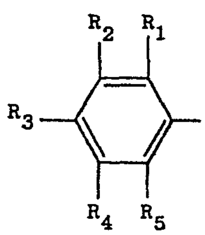

in which $R_1$, $R_2$, $R_3$, $R_4$ and $R_5$ are hydrogen; m is the number 0; and $R_6$ is methyl.

17. A composition according to any of Claims 14—16, in dosage unit form comprising from 1—250 mg. per kg. of body weight per dosage unit of active compound.

18. A method for preparing an anti-arthritic composition for use in inhibiting the progression of arthritis, meliorating inflammation and/or inhibiting progressive joint deterioration in mammals, comprising incorporating into a pharmaceutical carrier or diluent a 2-carbonyl-3-hydroxy-2-alkenonitrile of the Formula II as defined in any of claims 14 to 16.

19. A method of producing compounds of Formula I, as defined in Claim 1, which comprises:

(a) reacting an acetonitrile of the formula:

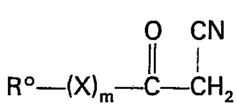

with a compound of the formula:

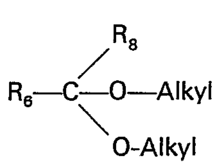

wherein R°, X, m and $R_6$ are as defined in claim 1 and $R_8$ is O-Alkyl or $N(Alkyl)_2$ and then hydrolyzing the product produced, and wherein, when $R_8$ is O-Alkyl the resultant product may be treated with ammonia or a primary or secondary amine prior to said hydrolysis; or

(b) reacting the metal (Me) enolate salt of an acetonitrile of the formula:

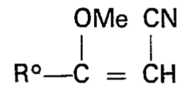

with an acyl halide of the formula:

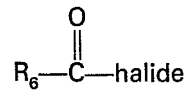

wherein R° and $R_6$ are as defined above; or

(c) reacting an acyl halide of the formula:

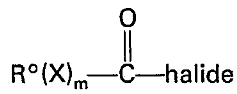

with a metal (Me) enolate salt of the formula:

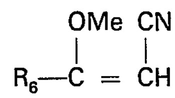

wherein X is $CH_2$, m is the number 0 or 1, R° and $R_6$ are as defined above; or

(d) reacting a compound of the formula:

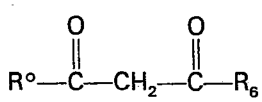

with N,N-dimethylformamide dimethylacetal to produce an intermediate compound of the formula:

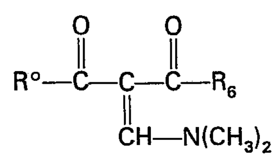

reacting said intermediate with hydroxylamine hydrochloride and treating the resulting reaction mixture with a strong base, wherein R° and $R_6$ are as defined above; or

(e) reacting a compound of the formula:

R°—X—H

with a compound of the formula:

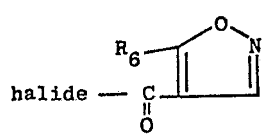

in the presence of a proton scavenger such as a tertiary amine, to form an intermediate of the formula:

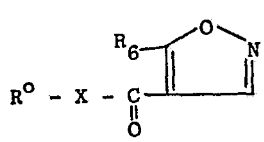

wherein X is O or S and R° and $R_6$ are as defined above, and further treating said intermediate with a proton scavenger such as a tertiary amine or alkoxide, thereby producing the corresponding final product; and

(f) if desired, forming pharmaceutically acceptable cation salts.

20. A method according to claim 19, wherein compounds are produced in accordance with the definitions in any one of claims 2 to 13.

21. A 2-carbonyl-3-hydroxy-2-alkenonitrile of the Formula II as defined in any one of Claims 14—16 for use in inhibiting the progression of arthritis, meliorating inflammation and/or inhibiting progressive joint deterioration in mammals.

**Revendications pour les Etats contractants: BE, CH, DE, FR, GB, IT LU, NL, SE**

1. Carbonyl-2 hydroxy-3 alcène-2 nitriles de formule:

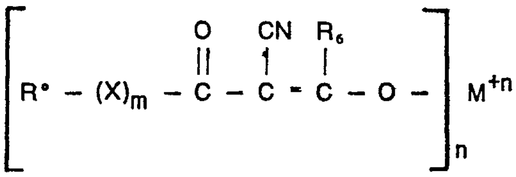

où

n est un nombre entier de 1 à 3 inclusivement:
M représente un atome d'hydrogène ou un cation convenant en pharmacie;
$R_6$ représente un radical alkyle comportant 1 à 4 atomes de carbone;
$R^o$ représente un des radicaux aromatiques suivants:
(1) le radical:

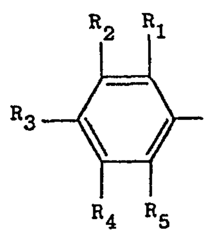

où $R_1$, $R_2$, $R_3$, $R_4$ et $R_5$ sont identiques ou différents et représentent un atome d'hydrogène ou un radical halogèno, alkyle comportant jusqu'à 4 atomes de carbone, alcoxy comportant jusqu'à 4 atomes de carbone, trifluorométhyle ou trichlorométhyle, sous réserve que, lorsque m est zéro, au moins deux des symboles $R_1$, $R_2$, $R_3$, $R_4$ et $R_5$ doivent représenter un atome d'hydrogène mais, lorsque $R_6$ représente un radical méthyle et m est zéro, tous les radicaux $R_1$, $R_2$, $R_3$, $R_4$ et $R_5$ ne peuvent pas représenter chacun un atome d'hydrogène; ou
(2) le fragment thiényle:

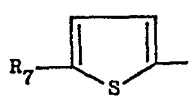 ou 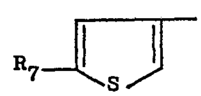

où $R_7$ représente un atome d'hydrogène ou un radical alkyle comportant jusqu'à 3 atomes de carbone, fluoro, chloro ou bromo;
représente $CH_2$, O ou S et m est égal à zero ou 1, sous réserve que, lorsque $R^o$ représente un fragment thiényle, m est égal à zéro;
et les tautomères des composés ci-dessus.

2. Composé selon la revendication 1, où $R^o$ représente le radical:

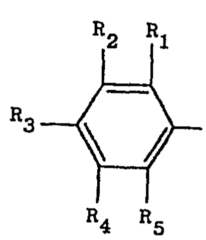

où $R_1$, $R_2$, $R_3$, $R_4$ et $R_5$ sont comme définis; et m est égal à zéro.

3. Composé selon la revendication 2, où un des radicaux $R_1$, $R_2$, $R_3$, $R_4$ et $R_5$ représente un radical bromo, chloro, fluoro, trifluorométhyle, méthyle ou méthoxy et les autres représentent chacun un atome d'hydrogène; ou $R_1$ représente un radical méthyle, $R_3$ représente un radical chloro et $R_2$, $R_4$ et $R_5$ représentent chacun un atome d'hydrogène.

4. Composé selon la revendication 1, où $R^o$ représente un fragment thiényle et m est égal à zéro.

5. Composé selon la revendication 4, où $R_7$ représente un atome d'hydrogène ou un radical chloro, fluoro, méthyle ou isopropyle.

6. Composé selon la revendication 1, où R° représente le radical:

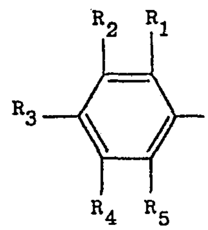

où $R_1$, $R_2$, $R_3$, $R_4$ et $R_5$ sont comme définis, X représente $CH_2$ et m est égal à 1.

7. Composé selon la revendication 6, où un ou deux des symboles $R_1$, $R_2$, $R_3$, $R_4$ et $R_5$ représentent un radical chloro et les autres représentent un atome d'hydrogène; ou un des ces symboles représente le radical fluoro et les autres représentent un atome d'hydrogène.

8. Composé selon la revendication 1, où R° représent le radical:

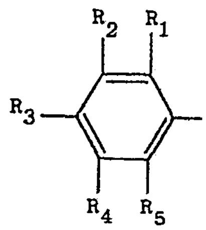

où $R_1$, $R_2$, $R_3$, $R_4$ et $R_5$ sont comme définis; X représente S et m est égal à 1.

9. Composé selon la revendication 8, où un des symboles $R_1$, $R_2$, $R_3$, $R_4$ et $R_5$ représente un radical bromo, chloro, fluoro, trifluorométhyle, méthyle ou méthoxy et les autres représentent un atome d'hydrogène; ou tous ces symboles représentent un atome d'hydrogène.

10. Composé selon la revendication 1, où R° représente le radical:

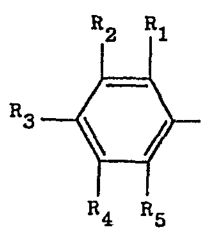

où $R_1$, $R_2$, $R_3$, $R_4$ et $R_5$ sont comme définis; X représente O et m est égal à 1.

11. Composé selon la revendication 10, où des symboles $R_1$, $R_2$, $R_3$, $R_4$ et $R_5$ représente un radical bromo, chloro, fluoro, méthyle ou méthoxy et les autres représentent un atome d'hydrogène; ou deux de ces symboles représentent un radical chloro et les autres représentent un atome d'hydrogène; ou deux de ces symboles représentent un radical méthoxy et les autres représentent un atome d'hydrogène; ou tous ces symboles représentent un atome d'hydrogène.

12. Composé selon l'une quelconque des revendications précédentes, où $R_6$ représente le radical méthyle.

13. Composé selon l'une quelconque des revendications précédentes, où M représente un atome d'hydrogène ou de sodium ou le radical triéthylammonium et n est égal à 1, ou M représente un atome de cuivre cuivrique et ne est égal à 2.

14. Composition antiarthritique pour l'inhibition de l'évolution de l'arthrite, la résolution de l'inflammation et/ou l'inhibition de la détérioration progressive des articulations chez les mammifères, comprenant un véhicule ou diluant convenant en pharmacie et un carbonyl-2 hydroxy-3 alcène-2 nitrile de formule:

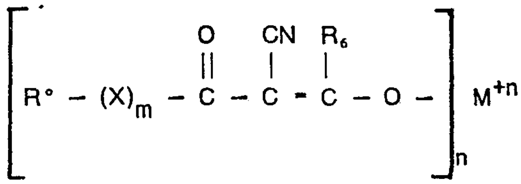

II

où

n est un nombre entier de 1 à 3 inclusivement;

M représente un atome d'hydrogène ou un cation convenant en pharmacie;

$R_6$ représente un radical alkyle comportant 1 à 4 atomes de carbone;

R° représente un des radicaux aromatiques suivants:

(1) le radical:

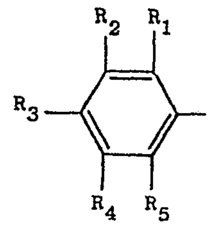

où $R_1$, $R_2$, $R_3$, $R_4$ et $R_5$ sont semblables ou différents et représentent un atome d'hydrogène ou un radical halogèno, alkyle comportant jusqu'à 4 atomes de carbone, alcoxy comportant jusqu'à 4 atomes de carbone, trifluorométhyle ou trichlorométhyle, sous réserve que, lorsque m est égal à zéro, au moins deux des symboles $R_1$, $R_2$, $R_3$, $R_4$ et $R_5$ doivent représenter un atome d'hydrogène; ou

(2) le fragment thiényle:

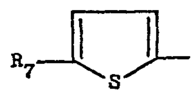 ou 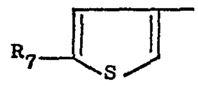

où $R_7$ représente un atome d'hydrogène ou un radical alkyle comportant jusqu'à 3 atomes de carbone, fluoro, chloro ou bromo;

X représente $CH_2$, O ou S et m est égal à zéro ou un, sous réserve que, lorsque R° est un fragment thiényle, m est égal à zéro;

et les tautomères des composés ci-dessus.

15. Composition selon la revendication 14, où ledit carbonyl-2 hydroxy-3 alcène-2 nitrile est comme défini dans l'une quelconque des revendications 2 à 13.

16. Composition selon la revendication 14, où R° représente le radical:

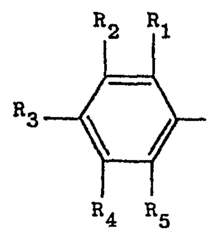

où $R_1$, $R_2$, $R_3$, $R_4$ et $R_5$ représentent un atome d'hydrogène; m est égal à zéro et $R_6$ représente le radical méthyle.

17. Composition selon l'une quelconque des revendications 14 à 16 sous forme d'une dose unitaire contenant 1 à 250 mg de composé actif par kg de poids corporel par dose unitaire.

18. Procédé pour préparer une composition antiarthritique pour empêcher l'évolution de l'arthrite, faire disparaître l'inflammation et/ou inhiber la détérioration progressive des articulations chez les mammifères qui consiste à incorporer à un véhicule ou diluant pharmaceutique un carbonyl-2 hydroxy-3 alcène-2 nitrile de formule II comme défini dans l'une quelconque des revendications 14 à 16.

19. Procédé pour préparer les composés de formule I comme défini dans la revendication 1 qui consiste à:

(a) faire réagir un acétonitrile de formule:

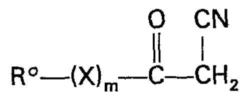

avec un composé de formule:

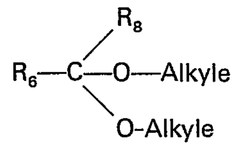

où R°, X, m et $R_6$ ont la même définition que dans la revendication 1 et $R_8$ représente O-Alkyle ou N(Alkyle)$_2$ puis à hydrolyser le produit obtenu et où, lorsque $R_8$ représente O-alkyle, le produit obtenu peut être traité par l'ammoniac ou une amine primaire ou secondaire avant ladite hydrolyse; ou

(b) faire réagir l'énolate de métal (Me) d'un acétonitrile de formule:

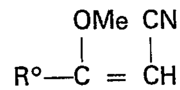

avec un halogénure d'acyle de formule:

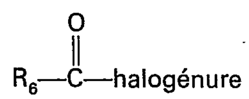

où R° et $R_6$ ont la même définition que dans la revendication 1; ou

(c) faire réagir un halogénure d'acyle de formule:

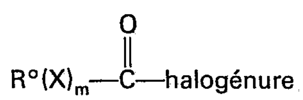

avec un énolate de métal (Me) de formule:

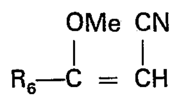

où X représente $CH_2$, m est égal à zéro ou 1 et R° et $R_6$ ont la même définition que ci-dessus; ou

(d) faire réagir un composé de formule:

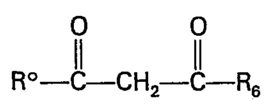

avec le N,N-diméthylformamide-diméthylacétal pour produire un composé intermédiaire de formule:

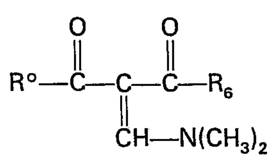

faire réagir cet intermédiaire avec le chlorhydrate d'hydroxylamine et traiter le mélange réactionnel obtenu avec une base forte, où R° et $R_6$ ont la même définition que ci-dessus; ou

(e) faire réagir un composé de formule:

R°—X—H

avec un composé de formule:

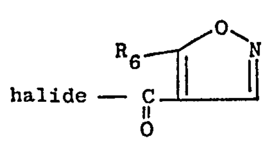

en présence d'un fixateur de protons tels qu'une amine tertiaire pour former un intermédiaire de formule:

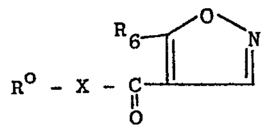

où X représente O ou S et R° et $R_6$ ont la même définition que ci-dessus, puis traiter cet intermédiaire avec un fixateur de protons tel qu'une amine tertiaire ou un alcoolate pour obtenir le produit final corespondant; et

(f) si on le désire former les sels de cations convenant en pharmacie.

20. Procédé selon la revendication 19, où on produit les composés selon les définitions de l'une quelconque des revendications 2 à 13.

21. Carbonyl-2-hydroxy-3 alcène-2 nitrile de formule II comme défini dans l'une quelconque des revendications 14 à 16 pour l'emploi dans l'inhibition de l'évolution de l'arthrite, la résolution de l'inflammation et/ou l'inhibition de la détérioration progressive des articulations chez les mammifères.

**Patentansprüche für die Vertragsstaaten: BE, CH, DE, FR, GB, IT, LU, NL, SE**

1. 2-Carbonyl-3-hydroxy-2-alkenonitrile der Formel;

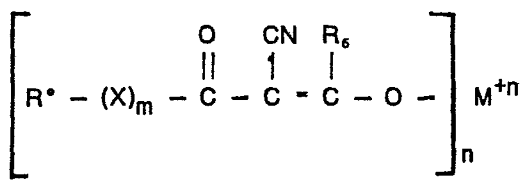

I

worin

n eine ganze Zahl von 1 bis einschließlich 3 ist;

M Wasserstoff oder ein pharmazeutisch akzeptables Kation ist;

$R_6$ eine Alkylgruppe von 1 bis 4 Kohlenstoffen ist;

R° eine der folgenden aromatischen Gruppen ist:

(1) die Gruppe

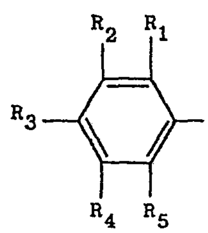

in der $R_1$, $R_2$, $R_3$, $R_4$ und $R_5$ gleich oder verschieden sind und Wasserstoff, Halogen, Alkyl mit bis zu 4 Kohlenstoffen, Alkoxy mit bis zu 4 Kohlenstoffen, Trifluormethyl oder Trichlormethyl sind, mit der Maßgabe, daß, wenn m die Zahl 0 ist, wenigstens zwei von $R_1$, $R_2$, $R_3$, $R_4$ und $R_5$ Wasserstoff sein müssen jedoch wenn $R_6$ Methyl ist und m die Zahl 0 ist, nicht alle $R_1$, $R_2$, $R_3$, $R_4$ und $R_5$ Wasserstoff sein dürfen; oder

(2) die Thienyleinheit 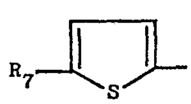 oder 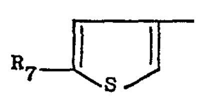

worin $R_7$ Wasserstoff, eine Alkylgruppe von bis zu 3 Kohlenstoffen, Fluor, Chlor oder Brom ist;

X $CH_2$, O oder S ist und m die Zahl 0 oder 1 ist, vorausgesetzt, daß dann, wenn R° eine Thienyleinheit ist, m 0 ist; und die Tautomeren der obigen Verbindungen.

2. Eine Verbindung gemäß Anspruch 1, worin R° die Gruppe:

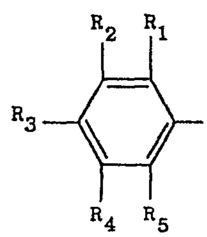

ist, worin $R_1$, $R_2$, $R_3$, $R_4$ und $R_5$ die angegebenen Bedeutungen haben; und m die Zahl 0 ist.

3. Eine Verbindung gemäß Anspruch 2, worin eines der Radikale $R_1$, $R_2$, $R_3$, $R_4$ und $R_5$ Brom, Chlor, Fluor, Trifluormethyl, Methyl oder Methoxy ist und die restlichen der genannten Radikale Wasserstoff sind; oder $R_1$ Methyl ist, $R_3$ Chlor ist und $R_2$, $R_4$ und $R_5$ jedes Wasserstoff sind.

4. Eine Verbindung gemäß Anspruch 1, worin R° eine Thienyleinheit ist und m die Zahl 0 ist.

5. Eine Verbindung gemäß Anspruch 4, worin $R_7$ Wasserstoff, Chlor, Fluor, Methyl oder Isopropyl ist.

6. Eine Verbindung gemäß Anspruch 1, worin R° die Gruppe

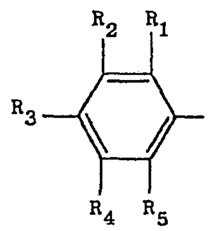

ist, worin $R_1$, $R_2$, $R_3$, $R_4$ und $R_5$ die angegebenen Bedeutungen haben; X $CH_2$ ist und m die Zahl 1 ist.

7. Eine Verbindung gemäß Anspruch 6, worin eines oder zwei de Radikale $R_1$, $R_2$, $R_3$, $R_4$ und $R_5$ Chlor ist und die restlichen der genannten Radikale Wasserstoff sind; oder eines der genannten Radikale Fluor ist und die restlichen der genannten Radikale Wasserstoff sind.

8. Eine Verbindung gemäß Anspruch 1, worin R° die Gruppe

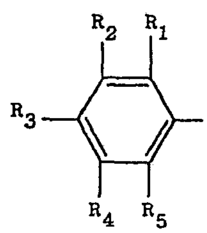

ist, worin $R_1$, $R_2$, $R_3$, $R_4$ und $R_5$ die angegebenen Bedeutungen haben; X S ist und m die Zahl 1 ist.

9. Eine Verbindung gemäß Anspruch 8, worin eines der Radikale $R_1$, $R_2$, $R_3$, $R_4$ und $R_5$ Brom, Chlor, Fluor, Trifluormethyl, Methyl oder Methoxy ist und die restlichen der genannten Radikale Wasserstoff sind; oder alle der genannten Radikale Wasserstoff sind.

10. Eine Verbindung gemäß Anspruch 1, worin R° die Gruppe

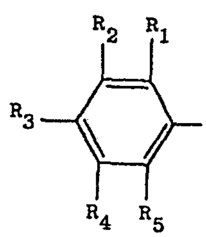

ist, worin $R_1$, $R_2$, $R_3$, $R_4$ und $R_5$ die angegebenen Bedeutungen haben; X O ist und m die Zahl 1 ist.

11. Eine Verbindung gemäß Anspruch 10, worin eines der Radikale $R_1$, $R_2$, $R_3$, $R_4$ und $R_5$ Brom, Chlor, Fluor, Methyl oder Methoxy ist und die restlichen der genannten Radikale Wasserstoff sind; oder zwei der genannten Radikale Chlor sind, die restlichen Wasserstoff sind; oder zwei der genannten Radikale Methoxy sind, die restlichen Wasserstoff sind; oder alle der genannten Radikale Wasserstoff sind.

12. Eine Verbindung gemäß irgendeinem der vorhergehenden Ansprüche, worin $R_6$ Methyl ist.

13. Eine Verbindung gemäß irgendeinem der vorhergehenden Ansprüche, worin M Wasserstoff, Natrium oder Triäthylammonium ist und n 1 ist oder M Kupfer(II) ist und n 2 ist.

14. Eine anti-arthritisches Mittel zur Verwendung bei der Hemmung des Fortschreitens von Arthritis, der Besserung von Entzündungen und/oder der Hemmung fortschreitender Gelenkentartung bei Säugetieren, umfassend einen pharmazeutisch akzeptablen Träger oder Verdünnungsstoff und ein 2-Carbonyl-3-hydroxy-1-alkenonitril der Formel:

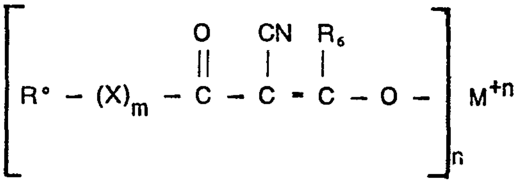 II

worin

n eine ganze Zahl von 1 bis einschließlich 3 ist;

M Wasserstoff oder ein pharmazeutisch akzeptables Kation ist;

$R_6$ eine Alkylgruppe von 1 bis 4 Kohlenstoffen ist;

R° eine der folgenden aromatischen Gruppen ist:

(1) die Gruppe

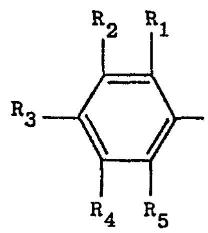

in der $R_1$, $R_2$, $R_3$, $R_4$ und $R_5$ gleich oder verschieden sind und Wasserstoff, Halogen, Alkyl mit bis zu 4 Kohlenstoffen, Alkoxy mit bis zu 4 Kohlenstoffen, Trifluormethyl oder Trichlormethyl sind, mit der Maßgabe, daß, wenn m die Zahl 0 ist, wenigstens zwei von $R_1$, $R_2$, $R_3$, $R_4$ und $R_5$ Wasserstoff sein müssen, oder

(2) die Thienyleinheit

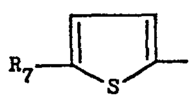 oder 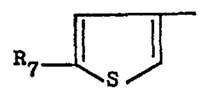

worin $R_7$ Wasserstoff, ein Alkylgruppe von bis zu 3 Kohlenstoffen, Fluor, Chlor oder Brom ist;

X $CH_2$, O oder S ist und m die Zahl 0 oder 1 ist, vorausgesetzt, daß dann, wenn R° eine Thienyleinheit ist, m 0 ist; und die Tautomeren der obigen Verbindungen.

15. Ein Mittel gemäß Anspruch 14, worin das genannte 2-Carbonyl-3-hydroxy-2-alkenonitril so ist, wie es in irgendeinem der Ansprüche 2 bis 13 definiert wurde.

16. Ein Mittel gemäß Anspruch 14, worin R° die Gruppe

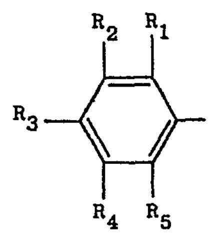

in der $R_1$, $R_2$, $R_3$, $R_4$ und $R_5$ Wasserstoff sind; m die Zahl 0 ist; und $R_6$ Methyl ist.

17. Ein Mittel gemäß irgendeinem der Ansprüche 14 bis 16 in einer Dosiseinheitsform, die von 1 bis 250 mg pro kg Körpergewicht pro Dosiseinheit der aktiven Verbindung umfaßt.

18. Ein Verfahren zur Hersetllung eines anti-arthritischen Mittels zur Verwendung bei der Hemmung des Fortschreitens von Arthritis, der Besserung von Entzündungen und/oder der Hemmung fortschreitender Gelenkentartung bei Säugetieren, umfassend das Einverleiben eines 2-Carbonyl-3-hydroxy-2-alkenonitrtils der Formel II gemäß der Definition in irgendeinem der Ansprüche 14 bis 16 in einen pharmazeutischen Träger oder Verdünnungsstoff.

19. Ein Verfahren zur Herstellung von Verbindungen der Formel I gemäß der Definition in Anspruch 1, umfassend

(a) die Umsetzung eines Acetonitrils der Formel:

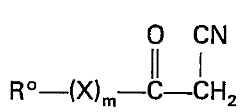

mit einer Verbindung der Formel:

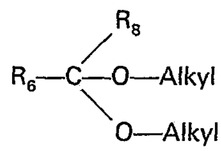

worin R°, X, m und $R_6$ die in Anspruch 1 angegebenen Bedeutungen haben und $R_8$ O-Alkyl oder $N(Alkyl)_2$ ist, und anschließend die Hydrolyse des gebildeten Produktes, und wobei, falls $R_8$ O-Alkyl ist, das resultierende Produkt mit Ammoniak oder einem primären oder sekundären Amin vor der genannten Hydrolyse behandelt werden kann; oder

(b) die Umsetzung des Metall(Me)-enolatsalzes eines Acetonitrils der Formel:

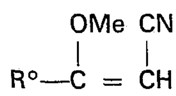

mit einem Acylhalogenid der Formel:

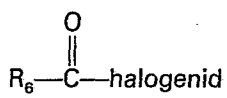

worin R° und $R_6$ die oben angegebenen Bedeutungen haben; oder

(c) die Unsetzung eines Acylhalogenids der Formel:

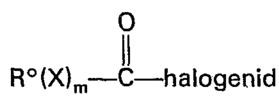

mit einem Metall(Me)-enolatsalz der Formel:

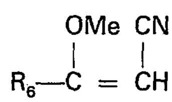

worin X $CH_2$ ist, m die Zahl 0 oder 1 ist, R° und $R_6$ die oben angegebenen Bedeutungen haben; oder

(d) die Umsetzung einer Verbindung der Formel:

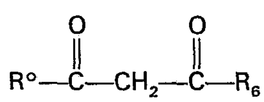

mit N,N-Dimethylformamid-dimethylacetal zur Bildung einer Zwischenproduktverbindung der Formel

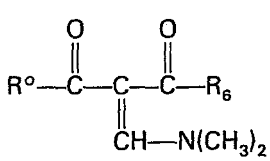

die Umsetzung des genannten Zwischenproduktes mit Hydroxylamin-hydrochlorid und die Behandlung des resultierenden Reaktionsgemisches mit einer starken Base, worin R° und $R_6$ die oben angegebenen Bedeutungen haben; oder

(e) die Umsetzung einer Verbindung der Formel:

R°—X—H

mit einer Verbindung der Formel:

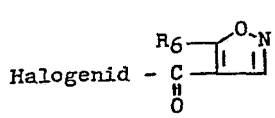

in Gegenwart eines Protonenfängers, wie eines tertiären Amins, zur Bildung eines Zwischenproduktes der Formel

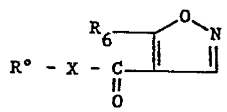

worin X O oder S ist und R° und $R_6$ die oben angegebenen Bedeutungen haben, und weiter die Behandlung des genannten Zwischenproduktes mit einem Protonenfänger, wie einem tertiären Amin oder Alkoxid, wobei das korrespondierende Endprodukt gebildet wird; und

(f) falls erwünscht, die Bildung pharmazeutisch akzeptabler Kationensalze.

20. Ein Verfahren gemäß Anspruch 19, worin Verbindungen gebildet werden, gemäß der Definitionen in irgendeinem der Ansprüche 2 bis 13.

21. Ein 2-Carbonyl-3-hydroxy-2-alkenonitril der Formel II gemäß der Definition in irgendeinem der Ansprüche 14 bis 16 zur Verwendung bei der Hemmung des Fortschreitens von Arthritis, der Besserung von Entzündung und/oder der Hemmung fortschreitender Gelenkentartung bei Säugetieren.

**Claims for Contracting State: AT**

1. A method for preparing an anti-arthritic composition for use in inhibiting the progression of arthritis, meliorating inflammation and/or inhibiting progressive joint deterioration in mammals, comprising incorporating into a pharmaceutical carrier or diluent a 2-carbonyl-3-hydroxy-2-alkenonitrile of the formula:

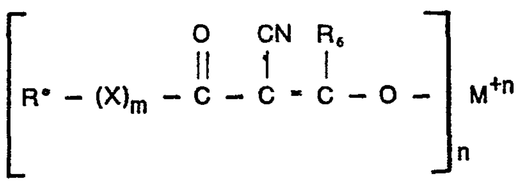

I

wherein

n is an integer from 1 to 3 inclusive;

M is hydrogen or a pharmaceutically acceptable cation;

$R_6$ is an alkyl group of 1 to 4 carbons;

R° is one of the following aromatic groups:

(1) the group

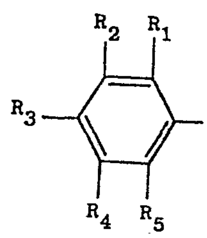

in which $R_1$, $R_2$, $R_3$, $R_4$ and $R_5$ are the same or different and are hydrogen, halogen, alkyl having up to 4 carbons, alkoxy having up to 4 carbons, trifluoromethyl or trichloromethyl, with the proviso that when m is the number 0 at least two of $R_1$, $R_2$, $R_3$, $R_4$ and $R_5$ must be hydrogen; or

(2) the thienyl moiety

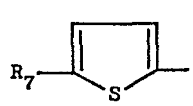

or

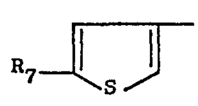

wherein $R_7$ is hydrogen, an alkyl group of up to 3 carbons, fluorine, chlorine or bromine;

X is $CH_2$, O or S and m is the number 0 or 1, provided that when R° is a thienyl moiety then m is 0; and the tautomers of the above compounds.

2. A method according to Claim 1, wherein R° is the group:

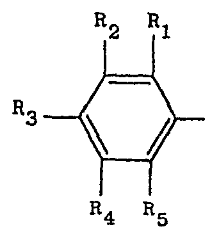

wherein $R_1$, $R_2$, $R_3$, $R_4$ and $R_5$ are as defined; and m is the number 0.

3. A method according to Claim 2, wherein one of the radicals $R_1$, $R_2$, $R_3$, $R_4$ and $R_5$ is bromo, chloro, fluoro, trifluoromethyl, methyl or methoxy and the remaining of said radicals are hydrogen; or $R_1$ is methyl, $R_3$ is chloro and $R_2$, $R_4$ and $R_5$ are each hydrogen.

4. A method according to Claim 1, wherein R° is a thienyl moiety and m is the number 0.

5. A method according to Claim 4, wherein $R_7$ is hydrogen, chloro, fluoro, methyl or isopropyl.

6. A method according to Claim 1, wherein R° is the group

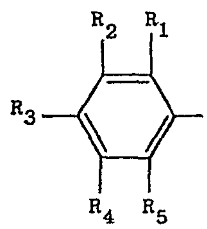

wherein $R_1$, $R_2$, $R_3$, $R_4$ and $R_5$ are as defined; X if $CH_2$ and m is the number 1.

7. A method according to Claim 6, wherein one or two of the radicals $R_1$, $R_2$, $R_3$, $R_4$ and $R_5$ is chloro and the remaining of said radicals are hydrogen; or one of said radicals is fluoro and the remaining of said radicals are hydrogen.

8. A method according to Claim 1, wherein R° is the group:

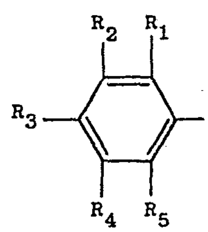

wherein $R_1$, $R_2$, $R_3$, $R_4$ and $R_5$ are as defined; X is S and m is the number 1.

9. A method according to Claim 8, wherein one of the radicals $R_1$, $R_2$, $R_3$, $R_4$ and $R_5$ is bromo, chloro, fluoro, trifluoromethyl, methyl or methoxy and the remaining of said radicals are hydrogen; or all of said radicals are hydrogen.

10. A method according to Claim 1, wherein R° is the group:

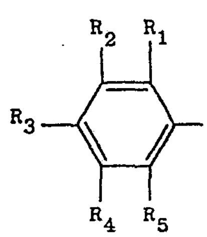

wherein $R_1$, $R_2$, $R_3$, $R_4$ and $R_5$ are as defined; X is O and m is the number 1.

11. A method according to Claim 10, wherein one of the radicals $R_1$, $R_2$, $R_3$, $R_4$ and $R_5$ is bromo, chloro, fluoro, methyl or methoxy and the remaining of said radicals are hydrogen; or two of said radicals are chloro, the remaining being hydrogen; or two of said radicals are methoxy, the remaining being hydrogen; or all of said radicals are hydrogen.

12. A method according to any one of the preceding claims, wherein $R_6$ is methyl.

13. A method according to any one of the preceding claims, wherein M is hydrogen, sodium or triethylammonium and n is 1, or M is cupric and n is 2.

14. A method for preparing a 2-carbonyl-3-hydroxy-2-alkenonitrile of the formula:

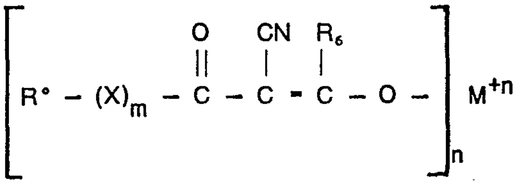

II

wherein

n is an integer from 1 to 3 inclusive;

M is hydrogen or a pharmaceutically acceptable cation;

$R_6$ is an alkyl group of 1 to 4 carbons;

R° is one of the following aromatic groups:

(1) the group

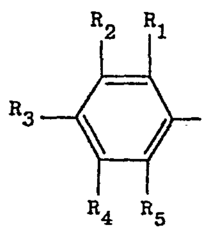

in which $R_1$, $R_2$, $R_3$, $R_4$ and $R_5$ are the same or different and are hydrogen, halogen, alkyl having up to 4 carbons, alkoxy having up to 4 carbons, trifluoromethyl or trichloromethyl, with the proviso that when m is the number 0 at least two of $R_1$, $R_2$, $R_3$, $R_4$ and $R_5$ must be hydrogen but when $R_6$ is methyl and m is the number 0 not all of $R_1$, $R_2$, $R_3$, $R_4$ and $R_5$ may be hydrogen; or

(2) the thienyl moiety

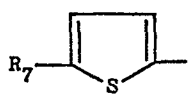 or 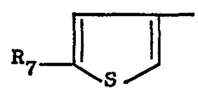

wherein $R_7$ is hydrogen, an alkyl group of up to 3 carbons, fluorine, chlorine or bromine;

X is $CH_2$, O or S and m is the number 0 or 1, provided that when R° is a thienyl moiety then m is 0; which comprises:

(a) reacting an acetonitrile of the formula:

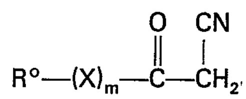

with a compound of the formula:

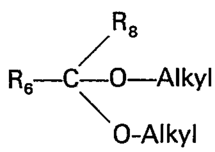

wherein R°, X, m and $R_6$ are as defined above and $R_8$ is O-Alkyl or N(Alkyl)$_2$ and then hydrolyzing the product produced, and wherein, when $R_8$ is O-Alkyl the resultant product may be treated with ammonia or a primary or secondary amine prior to said hydrolysis; or

(b) reacting the metal (Me) encolate salt of an acetonitrile of the formula:

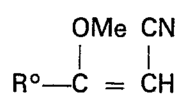

with an acyl halide of the formula:

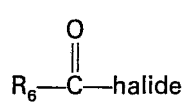

wherein R° and $R_6$ are as defined above; or

(c) reacting an acyl halide of the formula:

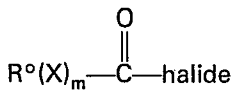

with a metal (Me) enolate salt of the formula:

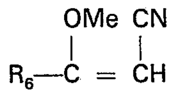

wherein X is $CH_2$, m is the number 0 or 1, R° and $R_6$ are as defined above; or

(d) reacting a compound of the formula:

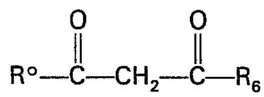

with N,N-dimethylformamide dimethylacetal to produce an intermediate compound of the formula:

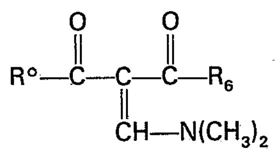

reacting said intermediate with hydroxylamine hydrochloride and treating the resulting reaction mixture with a strong base, wherein R° and $R_6$ are as defined above; or

(e) reacting a compound of the formula:

R°—X—H

with a compound of the formula:

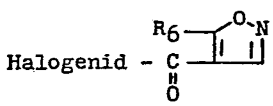

in the presence of a proton scavenger such as a tertiary amine, to form an intermediate of the formula:

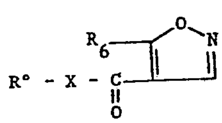

wherein X is O or S and R° and $R_6$ are as defined above, and further treating said intermediate with a proton scavenger such as a tertiary amine or alkoxide, thereby producing the corresponding final product; and

(f) if desired, forming pharmaceutically acceptable cation salts.

15. A method according to Claim 14, wherein compounds are produced in accordance with the definitions in any one of Claims 2—13.

**Revendications pour L'Etat contractant: AT**

1. Méthode pour préparer une composition antiarthritique pour l'inhibition de l'évolution de l'arthrite, la résolution de l'inflammation et/ou l'inhibition de la détérioration progressive des articulations chez les mammifères, comprenant un véhicule ou diluant convenant en pharmacie et un carbonyl-2 hydroxy-3 alcène-2 nitrile de formule:

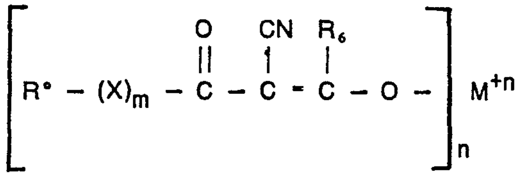

. . . I

où

n est un nombre entier de 1 à 3 inclusivement;

M représente un atome d'hydrogène ou un cation convenant en pharmacie;

$R_6$ représente un radical alkyle comportant 1 à 4 atomes de carbone;

R° représente un des radicaux aromatiques suivants:

(1) le radical:

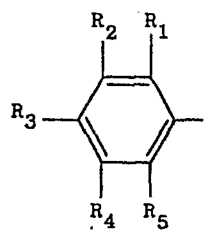

où $R_1$, $R_2$, $R_3$, $R_4$ et $R_5$ sont semblables ou différents et représentent un atome d'hydrogène ou un radical halogèno, alkyle comportant jusqu'à 4 atomes de carbone, alcoxy comportant jusqu'à 4 atomes de carbone, trifluorométhyle ou trichlorométhyle, sous réserve que, lorsque m est égal à zero, au moins deux des symboles $R_1$, $R_2$, $R_3$, $R_4$ et $R_5$ doivent représenter un atome d'hydrogène; ou

(2) le fragment thienyle:

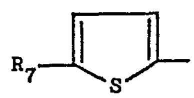

ou

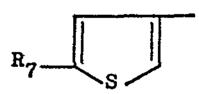

où $R_7$ représente un atome d'hydrogène ou un radical alkyle comportant jusqu'à 3 atomes de carbone, fluoro, chloro ou bromo;

X représente $CH_2$, O ou S et m est égal à zero ou un, sous réserve que, lorsque R° est un fragment thiényle, m est égal a zéro;

et les tautomères des composés ci-dessus.

2. Méthode selon la revendication 1, où R° represente le radical:

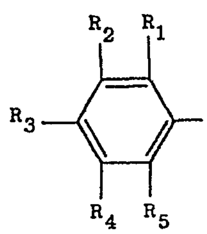

où $R_1$, $R_2$, $R_3$, $R_4$ et $R_5$ sont comme définis et m est égal à zéro.

3. Méthode selon la revendication 2, où un des radicaux $R_1$, $R_2$, $R_3$, $R_4$ et $R_5$ représente un radical bromo, chloro, fluoro, trifluoromethyle, méthyle ou méthoxy et les autres représentent chacun un atome d'hydrogène; ou $R_1$ représente un radical méthyle, $R_3$ représente un radical chloro et $R_2$, $R_4$ et $R_5$ représentent chacun un atome d'hydrogène.

4. Méthode selon la revendication 1, où R° représente un fragment thiényle et m est égal à zéro.

5. Méthode selon la revendication 4, où $R_7$ représente un atome d'hydrogène ou un radical chloro, fluoro, méthyle ou isopropyle.

6. Méthode selon la revendication 1, où R° représente le radical:

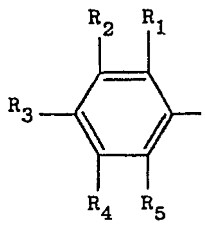

où $R_1$, $R_2$, $R_3$, $R_4$ et $R_5$ sont comme définis; X répresente $CH_2$ et m est égal à 1.

7. Méthode selon la revendication 6, où un ou deux des symboles $R_1$, $R_2$, $R_3$, $R_4$ et $R_5$ représentent un radical chloro et les autres représentent un atome d'hydrogène; ou un de ces symboles représente le radical fluoro et les autres représentent un atome d'hydrogène.

8. Méthode selon la revendication 1, où R° représente le radical:

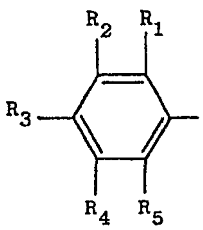

où $R_1$, $R_2$, $R_3$, $R_4$ et $R_5$ sont comme définis; X représente S et m est égal à 1.

9. Méthode selon la revendication 8, où un des symboles $R_1$, $R_2$, $R_3$, $R_4$ et $R_5$ représente un radical bromo, chloro, fluoro, trifluorométhyle, méthyle ou méthoxy et les autres représentent un atome d'hydrogène; ou tous ces symboles représentent un atome d'hydrogène.

10. Méthode selon la revendication 1, où R° représente le radical:

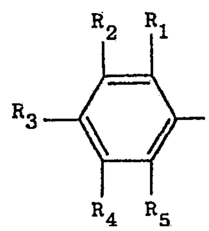

où $R_1$, $R_2$, $R_3$, $R_4$ et $R_5$ sont comme définis; X représente O et m est égal à 1.

11. Méthode selon la revendication 10, où des symboles $R_1$, $R_2$, $R_3$, $R_4$ et $R_5$ représente un radical bromo, chloro, fluoro, méthyle ou méthoxy et les autres représentent un atome d'hydrogène; ou deux des ces symboles représentent un radical chloro et les autres représentent un atome d'hydrogène; ou deux de ces symboles représentent un radical méthoxy et les autres représentent un atome d'hydrogène; ou tous ces symboles représentent un atome d'hydrogène.

12. Méthode selon l'une quelconque des revendications précédentes, où $R_6$ représente le radical méthyle.

13. Méthode selon l'une quelconques des revendications précédentes, où M représente un atome d'hydrogène ou de sodium ou le radical triéthylammonium et n est égal a 1 ou M représente un atome de cuivre cuivrique et n est égal à 2.

14. Méthode pour préparer carbonyl-2 hydroxy-3 alcène-2 nitriles de formule:

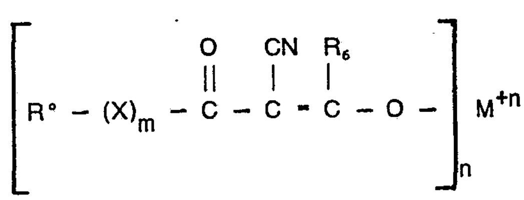

...II

où

n est un nombre entier de 1 à 3 inclusivement;

M représente un atome d'hydrogène ou un cation convenant en pharmacie;

$R_6$ représente un radical alkyle comportant 1 à 4 atomes de carbone;

R° représente un des radicaux aromatiques suivants:

(1) le radical:

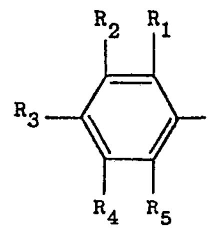

où $R_1$, $R_2$, $R_3$, $R_4$ et $R_5$ sont identiques ou différents et représentent un atome d'hydrogène ou un radical halogèno, alkyle comportant jusqu'à 4 atomes de carbone, alcoxy comportant jusqu'à 4 atomes de carbone, trifluorométhyle ou trichlorométhyle, sous réserve que, lorsque m est zéro, au moins deux des symboles $R_1$, $R_2$, $R_3$, $R_4$ et $R_5$ doivent représenter un atome d'hydrogène mais, lorsque $R_6$ représente un radical méthyle et m est zéro, tous les radicaux $R_1$, $R_2$, $R_3$, $R_4$ et $R_5$ ne peuvent pas représenter chacun un atome d'hydrogène; ou

(2) le fragment thiényle:

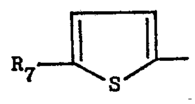 ou 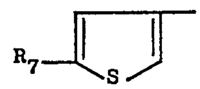

où $R_7$ représente un atome d'hydrogène ou un radical alkyle comportant jusqu'à 3 atomes de carbone, fluoro, chloro ou bromo;

X représente $CH_2$, O ou S et m est égal à zéro ou 1, sous réserve que, lorsque R° représente un fragment thiényle, m est égal à zero;

qui consiste à:

(a) faire réagir un acétonitrile de formule:

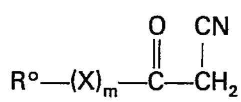

avec un composé de formule:

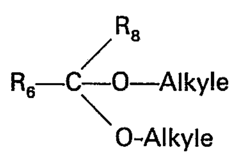

où R°, X, m et $R_6$ ont la même définition que ci-dessus et $R_8$ représente O-Alkyle ou N(Alkyle)$_2$ puis à hydrolyser le produit obtenu et où, lorsque $R_8$ représente O-alkyle, le produit obtenu peut être traité par l'ammoniac ou une amine primaire ou secondaire avant ladite hydrolyse; ou

(b) faire réagir l'énolate de métal (Me) d'un acétonitrile de formule:

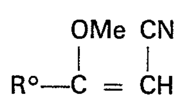

avec un halogénure d'acyle de formule:

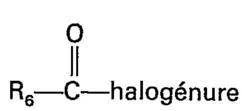

où R° et $R_6$ ont la même définition que ci-dessus; ou

(c) faire réagir un halogénure d'acyle de formule:

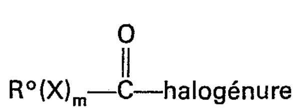

avec un énolate de métal (Me) de formule:

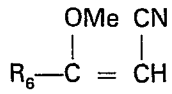

où X représente $CH_2$, m est égal à zéro ou 1 et R° et $R_6$ ont la même définition que ci-dessus; ou

(d) faire réagir un composé de formule:

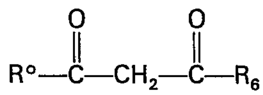

avec le N,N-diméthylformamide-diméthylacétal pour produire un composé intermédiaire de formule:

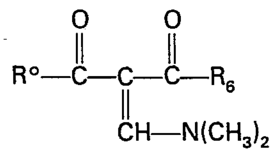

faire réagir cet intermédiaire avec le chlorhydrate d'hydroxylamine et traiter le mélange réactionnel obtenu avec une base forte, où R° et $R_6$ ont la même définition que ci-dessus; ou

(e) faire réagir un composé de formule:

$$R°—X—H$$

avec un composé de formule:

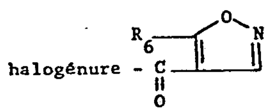

en présence d'un fixateur protons tels qu'une amine tertiaire pour former un intermédiaire de formule:

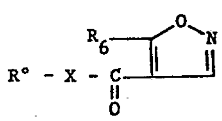

où X représente O ou S et R° et $R_6$ ont la même définition que ci-dessus, puis traiter cet intermédiaire avec un fixateur de protons tel qu'une amine tertiaire ou un alcoolate pour obtenir le produit final correspondant; et

(f) si on le désire former les sels de cations convenant en pharmacie.

15. Méthode selon la revendication 14, où les composés sont produits comme définit dans l'une quelconque des revendications 2 à 13.

**Patentansprüche für den Vertragsstaat: AT**

1. Verfahren zur Herstellung eines anti-arthritischen Mittles zur Verwendung bei der Hemmung des Fortschreitens von Arthritis, der Besserung von Entzündung und/oder der Hemmung fortschreitender Gelenkentartung bei Säugetieren, umfassend die Einverleibung eines 2-Carbonyl-3-hydroxy-2-alkenonitrils der Formel

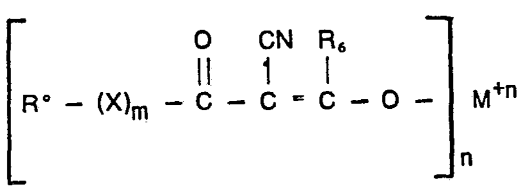

in einen pharmazeutischen Träger oder ein Verdünnungsmittel, wobei

n eine ganze Zahl von 1 bis einschließlich 3 ist;

M Wasserstoff oder ein pharmazeutisch akzeptables Kation ist;

$R_6$ eine Alkylgruppe von 1 bis 4 Kohlenstoffen ist;

R° eine der folgenden aromatishen Gruppen ist:

(1) die Gruppe

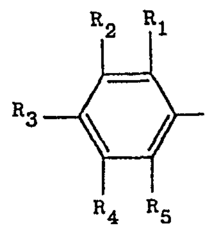

in der $R_1$, $R_2$, $R_3$, $R_4$ und $R_5$ gleich oder verschieden sind und Wasserstoff, Halogen, Alkyl mit bis zu 4 Kohlenstoffen, Alkoxy mit bis zu 4 Kohlenstoffen, Trifluormethyl oder Trichlormethyl sind, mit der Maßgabe, daß, wenn m die Zahl 0 ist, wenigstens zwei von $R_1$, $R_2$, $R_3$, $R_4$ und $R_5$ Wasserstoff sein müssen; oder

(2) die Thienyleinheit

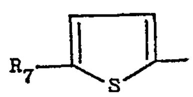 oder 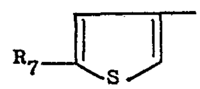

worin $R_7$ Wasserstoff, eine Alkylgruppe von bis zu 3 Kohlenstoffen, Fluor, Chlor oder Brom ist;

X $CH_2$, O oder S ist und

m die Zahl 0 oder 1 ist, vorausgesetzt, daß dann, wenn R° eine Thienyleinheit ist, m 0 ist; und die Tautomeren der obigen Verbindungen.

2. Ein Verfahren gemäß Anspruch 1, worin R° die Gruppe

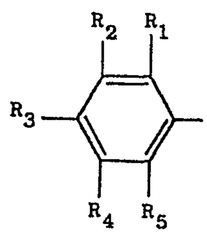

worin $R_1$, $R_2$, $R_3$, $R_4$ und $R_5$ die angegebenen Bedeutungen haben; und m die Zahl 0 ist.

3. Ein Verfahren gemäß Anspruch 2, worin eines der Radikale $R_1$, $R_2$, $R_3$, $R_4$ und $R_5$ Brom, Chlor, Fluor, Trifluormethyl, Methyl oder Methoxy ist und die restlichen der genannten Radikale Wasserstoff sind; oder $R_1$ Methyl ist, $R_3$ Chlor ist und $R_2$, $R_4$ und $R_5$ jedes Wasserstoff sind.

4. Ein Verfahren gemäß Anspruch 1, worin R° eine Thienyleinheit ist und m die Zahl 0 ist.

5. Ein Verfahren gemäß Anspruch 4, worin $R_7$ Wasserstoff, Chlor, Fluor, Methyl oder Isopropyl ist.

6. Ein Verfahren gemäß Anspruch 1, worin R° die Gruppe

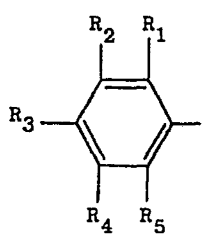

worin $R_1$, $R_2$, $R_3$, $R_4$ und $R_5$ die angegebenen Bedeutungen haben; X $CH_2$ ist und m die Zahl 1 ist.

7. Ein Verfahren gemäß Anspruch 6, worin eines oder zwei der Radikale $R_1$, $R_2$, $R_3$, $R_4$ und $R_5$ Chlor ist und die restlichen der genannten Radikale Wasserstoff sind; oder eines der genannten Radikale Fluor ist und die restlichen der genannten Radikale Wasserstoff sind.

8. Ein Verfahren gemäß Anspruch 1, worin R° die Gruppe

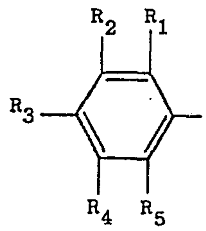

worin $R_1$, $R_2$, $R_3$, $R_4$ und $R_5$ die angegebenen Bedeutungen haben; X S ist und m die Zahl 1 ist.

9. Ein Verfahren gemäß Anspruch 8, worin eines der Radikale $R_1$, $R_2$, $R_3$, $R_4$ und $R_5$ Brom, Chlor, Fluor, Trifluormethyl, Methyl oder Methoxy ist und die restlichen der genannten Radikale Wasserstoff sind; oder alle der genannten Radikale Wasserstoff sind.

10. Ein Verfahren gemäß Anspruch 1, worin R° die Gruppe

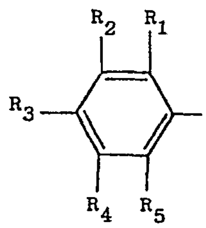

worin $R_1$, $R_2$, $R_3$, $R_4$ und $R_5$ die angegebenen Bedeutungen haben; X O ist und m die Zahl 1 ist.

11. Ein Verfahren gemäß Anspruch 10, worin eines der Radikale $R_1$, $R_2$, $R_3$, $R_4$ und $R_5$ Brom, Chlor, Fluor, Methyl oder Methoxy ist und die restlichen der genannten Radikale Wasserstoff sind; oder zwei der genannten Radikale Chlor sind, die restlichen Wasserstoff sind; oder zwei der genannten Radikale Methoxy sind, die restlichen Wasserstoff sind; oder alle der genannten Radikale Wasserstoff sind.

12. Ein Verfahren gemäß irgendeinem der vorhergehenden Ansprüche, worin $R_6$ Methyl ist.

13. Ein Verfahren gemäß irgendeinem der vorhergehenden Ansprüche, worin M Wasserstoff, Natrium oder Triäthylammonium ist und n 1 ist oder M Kupfer(II) ist und n 2 ist.

14. Ein Verfahren der Herstellung eines 2-Carbonyl-3-hydroxy-2-alkenonitrils der Formel

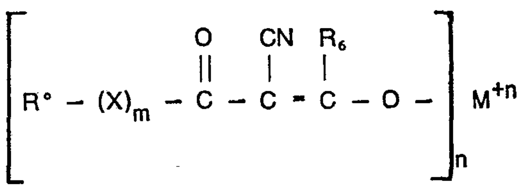

worin

n eine ganze Zahl von 1 bis einschließlich 3 ist;

M Wasserstoff oder ein pharmazeutisch akzeptables Kation ist;

$R_6$ eine Alkylgruppe von 1 bis 4 Kohlenstoffen ist;

R° eine der folgenden aromatischen Gruppen ist:

(1) die Gruppe

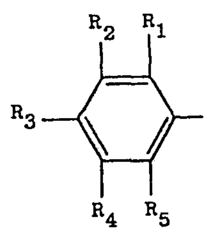

in der $R_1$, $R_2$, $R_3$, $R_4$ und $R_5$ gleich oder verschieden sind und Wasserstoff, Halogen, Alkyl mit bis zu 4 Kohlenstoffen, Alkoxy mit bis zu 4 Kohlenstoffen, Trifluormethyl oder Trichlormethyl sind, mit der Maßgabe, daß, wenn m die Zahl 0 ist, wenigstens zwei von $R_1$, $R_2$, $R_3$, $R_4$ und $R_5$ Wasserstoff sein müssen, jedoch wenn $R_6$ Methyl ist und m die Zahl 0 ist, nicht alle $R_1$, $R_2$, $R_3$, $R_4$ und $R_5$ Wasserstoff sein dürfen; oder

(2) die Thienyleinheit

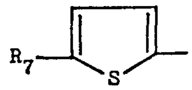 oder 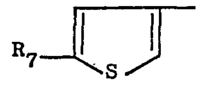

worin $R_7$ Wasserstoff, ein Alkylgruppe von bis zu 3 Kohlenstoffen, Fluor, Chlor oder Brom ist;

X $CH_2$, O oder S ist und m die Zahl 0 oder 1 ist, vorausgesetzt, daß dann, wenn R° eine Thienyleinheit ist, m 0 ist;

umfassend:

(a) die Umsetzung eines Acetonitrils der Formel

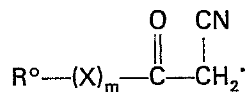

mit einer Verbindung der Formel

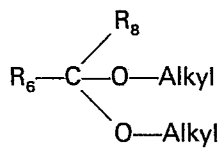

worin R°, X, m und $R_6$ die oben angegebenen Bedeutungen haben und $R_8$ O-Alkyl oder $N(Alkyl)_2$ ist, und anschließend die Hydrolyse des gebildeten Produkts, und wobei, falls $R_8$ O-Alkyl ist, das resultierende Produkt mit Ammoniak oder einem primären oder sekundären Amin vor der genannten Hydrolyse behandelt werden kann; oder

(b) die Umsetzung des Metall(Me)-enolatsalzes eines Acetonitrils der Formel:

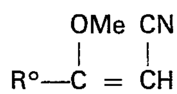

mit einem Acylhalogenid der Formel:

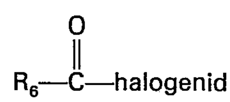

worin R° und $R_6$ die oben angegebenen Bedeutungen haben; oder

(c) die Umsetzung eines Acylhalogenids der Formel:

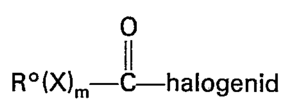

mit einem Metall(Me)-enolatsalz der Formel:

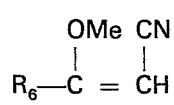

worin X $CH_2$ ist, m die Zahl 0 oder 1 ist, R° und $R_6$ die oben angegebenen Bedeutungen haben; oder

(d) die Umsetzung einer Verbindung der Formel:

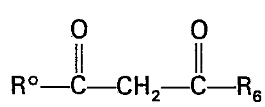

mit N,N-Dimethylformamid-dimethylacetal zur Bildung einer Zwischenproduktverbindung der Formel:

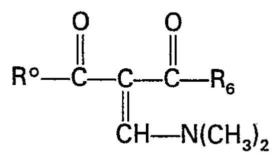

die Umsetzung des genannten Zwischenprodukts mit Hydroxylamin-hydrochlorid und die Behandlung des resultierenden Reaktionsgemisches mit einer starken Base, worin R° und $R_6$ die oben angegebenen Bedeutungen haben; oder

(e) die Umsetzung einer Verbindung der Formel:

$$R°—X—H$$

mit einer Verbindung der Formel:

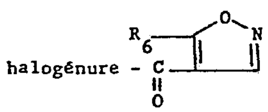

in Gegenwart eines Protonenfängers, wie eines tertiären Amins, zur Bildung eines Zwischenproduktes der Formel

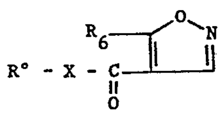

worin X O oder S ist und R° und $R_6$ die oben angegebenen Bedeutungen haben, und weiter die Behandlung des genannten Zwischenproduktes mit einem Protonenfänger, wie einem tertiären Amin oder Alkoxid, unter Bildung des korrespondierenden Endproduktes; und

(f) falls erwünscht, die Bildung pharmazeutisch akzeptabler Kationensalze.

15. Ein Verfahren gemäß Anspruch 14, worin Verbindungen hergestellt werden, in Übereinstimmung mit den Definitionen in irgendeinem der Ansprüche 2 bis 13.